(19) **Europäisches Patentamt / European Patent Office / Office européen des brevets**

(11) **EP 2 030 016 B1**

(12) # EUROPEAN PATENT SPECIFICATION

(45) Date of publication and mention
of the grant of the patent:
**25.03.2015 Bulletin 2015/13**

(51) Int Cl.:
*A61K 38/55* (2006.01)   *C07K 16/40* (2006.01)
*C12Q 1/37* (2006.01)   *G01N 33/50* (2006.01)

(21) Application number: **07798837.6**

(22) Date of filing: **20.06.2007**

(86) International application number:
**PCT/US2007/071691**

(87) International publication number:
**WO 2007/149935 (27.12.2007 Gazette 2007/52)**

(54) **METHODS AND COMPOSITIONS FOR MODULATING HEPSIN ACTIVATION OF UROKINASE-TYPE PLASMINOGEN ACTIVATOR**

VERFAHREN UND ZUSAMMENSETZUNGEN ZUR MODULATION DER HEPSIN-AKTIVIERUNG DES UROKINASE-PLASMINOGEN-AKTIVATORS

PROCÉDÉS ET COMPOSITIONS POUR MODULER L'ACTIVATION DE L'HEPSINE DE L'ACTIVATEUR DU PLASMINOGÈNE DE TYPE UROKINASE

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **22.06.2006 US 805584 P**

(43) Date of publication of application:
**04.03.2009 Bulletin 2009/10**

(73) Proprietor: **Genentech, Inc.**
**South San Francisco CA 94080-4990 (US)**

(72) Inventors:
• **KIRCHHOFER, Daniel, K.**
**Los Altos, California 94024 (US)**
• **MORAN, Paul, M.**
**El Cerrito, California 94530 (US)**

(74) Representative: **Woolley, Lindsey Claire**
**Mewburn Ellis LLP**
**33 Gutter Lane**
**London EC2V 8AS (GB)**

(56) References cited:
**WO-A-01/57194   WO-A-03/064620**
**WO-A-2006/014928   US-A1- 2004 009 911**
**US-A1- 2004 166 117**

• **TAKEUCHI T ET AL: "CELLULAR LOCALIZATION OF MEMBRANE-TYPE SERINE PROTEASE 1 AND IDENTIFICATION OF PROTEASE-ACTIVATED RECEPTOR-2 AND SINGLE-CHAIN UROKINASE-TYPE PLASMINOGEN ACTIVATOR AS SUBSTRATES" JOURNAL OF BIOLOGICAL CHEMISTRY, AMERICAN SOCIETY OF BIOLOCHEMICAL BIOLOGISTS, BIRMINGHAM,, US, vol. 275, no. 34, 25 August 2000 (2000-08-25), pages 26333-26342, XP001000977 ISSN: 0021-9258**
• **KIRCHHOFER D ET AL: "Hepsin activates pro-hepatocyte growth factor and is inhibited by hepatocyte growth factor activator inhibitor-1B (HAI-1B) and HAI-2", FEBS LETTERS, ELSEVIER, AMSTERDAM, NL, vol. 579, no. 9, 28 March 2005 (2005-03-28), pages 1945-1950, XP027696479, ISSN: 0014-5793 [retrieved on 2005-03-28]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

CROSS-REFERENCE TO RELATED APPLICATIONS

This application claims priority under 35 USC §119 to US Provisional Application No. 60/805,584, filed June 22, 2006.

TECHNICAL FIELD

The present invention relates generally to the fields of molecular biology and growth factor regulation. More specifically, the invention concerns modulators of enzymatic activation of urokinase-type plasminogen activator, and uses of said modulators.

BACKGROUND

Hepsin is a type II transmembrane serine protease (TTSP) expressed on the surface of epithelial cells. The 417 amino acid protein is composed of a short N-terminal cytoplasmic domain, a transmembrane domain and a single scavenger receptor cysteine-rich domain that packs tightly against the C-terminal protease domain (1). The physiologic function of hepsin is unclear. Despite its expression in the very early stages of embryogenesis (2), hepsin-deficient mice were viable and developed normally (3,4). Hepsin was found not to be essential for liver regeneration and for coagulation-related physiological functions (3,4). However, hepsin has been implicated in ovarian cancer ((5) & WO2001/62271) and prostate cancer (6-11), where several gene expression studies have identified it as one of the most highly induced genes. Hepsin RNA levels were found to be low in normal prostate and benign hyperplasia, but strongly increased in prostate carcinoma, particularly in advanced stages (7-10). Hepsin protein staining with a monoclonal anti-hepsin antibody showed that hepsin expression was highest at sites of bone metastasis and in late stage primary tumors (12), which is consistent with the finding that increased hepsin RNA levels correlated with higher Gleason grades and tumor progression (9-10,13). In contrast, using a different antibody, Dhanasekaran et al. (6) found strongest hepsin expression in high-grade prostate intra-neoplastic lesions and lower expression in primary carcinoma and in metastatic lesions. These studies raised the question of whether hepsin is involved in prostate cancer. In-vitro studies did not provide clear answers, and depending on the experimental conditions used, hepsin was found to promote, inhibit or not affect tumor cell growth (12,14,15).

Evidence for a role of hepsin in prostate cancer came from a recent study by Klezovitch et al. (16) demonstrating that in a mouse model of non-metastasizing prostate cancer, overexpression of hepsin led to primary tumor progression and metastasis. Intriguingly, hepsin overexpression was associated with basement membrane disruption (16) pointing towards the possibility that hepsin activity is somehow linked to the degradation of basement membrane components. In vitro, hepsin was able to convert the latent growth factor pro-hepatocyte growth factor (pro-HGF) into its active two-chain form (HGF), which induced Met receptor signaling (17, 18, WO2006/014928 Because the HGF/Met pathway has been implicated in invasive tumor growth and metastasis, it is possible that overexpression of hepsin activates the HGF/Met axis in prostate cancer. Hepsin was also shown to cleave other substrates in vitro, mainly coagulation-related proteins (17,19). However, their role in tumorigenesis is not known.

In view of the basement membrane defects that were associated with hepsin-overexpression in the mouse prostate, we hypothesized that hepsin might activate protease zymogens that are directly linked to basement membrane degradation. It was known from previous studies that hepsin does not activate plasminogen (18).

The expression profile of hepsin in cancer tissues as described above, coupled with its potential role in acting as a regulator of cellular factors whose dysregulation might underlie carcinogenesis, suggests that modulation of hepsin's interaction with such cellular factors could prove to be an efficacious therapeutic approach. In this regard, there is a clear need for a comprehensive understanding of hepsin's physiological substrates. The invention fulfills this need and provides other benefits.

WO 01/57194 discloses an MTSP (such as e.g., the membrane-type serine protease MTSP1) inhibitor (antagonist) which can be an antibody, or an inhibitor of MTSP activation, therefore of interaction between the MTSP and the substrate (such as, e.g. sc-uPA, or pro-tPA). Other MTSP such as Prostate-specific antigen (PSA); or Hepsin; or Serine protease-like gene, designated normal epithelial cell-specific 1 (NES1) are also disclosed.

US 2004/009911 is concerned with hepsin-cleavable molecules having a hepsin cleavage site. US 2004/009911 describes such hepsin-cleavable molecules linked to an inhibitory moiety capable of inactivation of hepsin as hepsin inhibitors, and prodrugs with such hepsin-cleavable molecules linked to a cell modulating moiety for release.

DISCLOSURE OF THE INVENTION

It is disclosed herein that hepsin does not cleave pro-tissue type plasminogen activator (pro-tPA) but efficiently converts pro-uPA into high molecular weight uPA by cleavage at the $Lys^{158}$-$Ile^{159}$ ($P_1$ - $P_1$') peptide bond. The recognition of a Lys as the $P_1$ residue was surprising, since other studies showed that hepsin prefers Arg over Lys at the $P_1$ position. Yet, structural modeling supported the experimental findings, indicating that the pro-uPA scissile peptide was easily accommodated into the hepsin active site. Moreover, uPA generated by hepsin displayed enzymatic activity towards small synthetic and macromolecular substrates indistinguishable from uPA produced by plasmin. The catalytic efficiency of pro-uPA activation by hepsin ($k_{cat}/K_m$ 4.8 x $10^5$ $M^{-1}$ $s^{-1}$) was similar to that of plasmin, which is considered the most potent pro-uPA activator, and was about 6-fold higher than that of matriptase. Conversion of pro-uPA was also demonstrated with cell surface-expressed full-length hepsin. A stable hepsin-overexpressing LnCaP cell line converted pro-uPA into high molecular weight uPA at a rate of 6.6 ± 1.9 nM uPA $hr^{-1}$, which was about 3-fold higher than LnCaP cells expressing lower hepsin levels on their surface. It is concluded that hepsin may play a role in activating pro-uPA to initiate plasmin-mediated proteolytic pathways at the tumor/stroma interface, leading to basement membrane disruption and tumor progression.

As described herein, a physiological substrate for hepsin is pro-urokinase-type plasminogen activator, which is a protease zymogen that is directly linked to basement membrane degradation and other physiological functions associated with various aspects of cancer development. Hepsin is shown herein to cleave pro-uPA with potent activity, resulting in activated uPA that exhibits normal enzymatic activities. The invention provides methods and compositions based at least in part on these findings, which are described in detail herein. Hepsin and its interaction with pro-uPA is a unique and advantageous target for greater fine-tuning in designing prophylatic and/or therapeutic approaches against pathological conditions associated with abnormal or unwanted hepsin and/or uPA/plasmin-mediated proteolytic activity. Thus, the invention provides methods, compositions, kits and articles of manufacture for identifying and for using substances that are capable of modulating the hepsin and/or uPA/plasmin-mediated proteolytic pathway through modulation of molecular interactions involved in the regulation of uPA activation.

Accordingly, the application provides a method of screening for (or identifying) a candidate inhibitor (i.e., antagonist) substance that inhibits hepsin activation of pro-uPA, said method comprising: (a) contacting a candidate substance with a first sample comprising hepsin and a pro-uPA substrate, and (b) comparing amount of pro-uPA activation in the sample with amount of pro-uPA activation in a reference sample comprising similar amounts of hepsin and pro-uPA substrate as the first sample but that has not been contacted with said candidate substance, whereby a decrease in amount of pro-uPA activation in the first sample compared to the reference sample indicates that the candidate substance is capable of inhibiting hepsin activation of pro-uPA. In one embodiment, hepsin in a sample is in an effective amount for activating said pro-uPA substrate. A pro-uPA substrate suitable for use in these methods can be in a number of forms, so long as it mimics the characteristic of the hepsin cleavage site on pro-uPA. Examples of pro-uPA substrate include, but are not limited to, full length single chain uPA comprising a wild type form of the $Lys_{158}$-$Ile_{159}$ ($P_1$ - $P_1$') peptide bond, and any fragment of uPA that comprises this peptide linkage. Such fragment can be any length, for example at least (about) 5, 7, 10, 15, 20, 25 amino acids in length, or between (about) 4 and 25, 5 and 20, 7 and 15 amino acids in length. Generally and preferably, a pro-uPA substrate comprises a $Lys_{158}$-$Ile_{159}$ ($P_1$ - $P_1$') peptide bond capable of being cleaved by wild type hepsin.

The application provides a method of screening for a substance that blocks pro-uPA activation by hepsin, said method comprising screening for a substance that binds (preferably, but not necessarily, specifically) hepsin or pro-uPA and blocks specific interaction (e.g., binding) between hepsin and pro-uPA. In the disclosure, the substance competes with hepsin for binding to uPA. In the disclosure, the substance competes with pro-uPA for binding to hepsin. In the disclosure, the substance comprises, consists or consists essentially of an amino acid sequence having at least about 60%, 70%, 80%, 90%, 95%, 99% sequence similarity or identity with respect to pro-uPA (e.g., human), e.g., a fragment of human uPA comprising amino acid residues $Lys_{158}$ peptide linked to $Ile_{159}$. In the disclosure wherein the substance comprises, consists or consists essentially of such an amino acid sequence, the fragment is mutated or devoid of at least a portion of the uPA sequence associated with enzymatic activity, e.g. activation of plasminogen.

As would be evident to one skilled in the art, screening assays consistent with those described above can also comprise a first step of screening for formation of hepsin-uPA complex to obtain a first set of candidate modulatory substance, followed by a second step of screening based on ability of the first set of candidate modulatory substance to modulate activation of pro-uPA and/or conversion of pro-uPA into a form that is enzymatically active. Suitable readouts can be any that would be evident to one skilled in the art, based on knowledge of enzyme-substrate complex formation and/or biological activities associated with the hepsin/uPA/plasmin pathway. Enzyme-substrate complex formation can be measured using, for example, routine biochemical assays (e.g., gel electrophoresis, chromatography, NMR, etc.). uPA/plasmin biological activities include but are not limited to degradation/disruption of basement membrane, matrix degradation, etc.

The disclosure provides antagonists that disrupt the hepsin/uPA interaction. For example, the disclosure provides a molecule that inhibits hepsin cleavage of pro-uPA (e.g., cleavage at the $Lys_{158}$-$Ile_{159}$ position). The molecule can exert its inhibitory function in any number of ways, including but not limited to binding to either hepsin or pro-uPA such that hepsin cleavage of pro-uPA is inhibited, binding to hepsin-pro-uPA complex such that cleavage of pro-uPA is inhibited, and/or binding to pro-uPA or hepsin (singly or in complex) such that effects of uPA cleavage by hepsin is inhibited (e.g., inhibition of release of uPA subsequent to cleavage by hepsin). In the disclosure, an antagonist molecule of the invention inhibits biological activities associated with pro-uPA activation.

In one aspect, an antagonist of the invention is derived from the discovery described herein that a fragment from hepatocyte growth factor activator inhibitors (HAI-1, HAI-1B, HAI-2) is a potent inhibitor of hepsin activation of pro-uPA. In the embodiment, the invention provides an antagonist of pro-uPA activation by hepsin, said antagonist comprising at least a portion (including all) of human HAI-1, HAI-1B or HAI-2. The portion comprises a Kunitz domain (KD) sequence capable of inhibiting pro-uPA activation by hepsin. Said Kunitz domain sequence is Kunitz domain 1 (KD1) of HAI-1 or HAI-1B. An antagonist of the disclosure comprises a variant KD1 sequence having at least about 70%, 75%, 80%, 85%, 90%, 95%, 97%, 98%, 99% sequence identity with wild type KD1 of human HAI-1, wherein said variant sequence has at least comparable ability as wild type KD1 in inhibiting hepsin cleavage of human pro-uPA. An antagonist of the disclosure comprises a variant KD1 sequence having between about 70% and 99%, about 75% and 98%, about 80% and 97%, 85% and 95% sequence identity with wild type KD1 of human HAI-1, wherein said sequence has at least comparable ability as wild type KD1 in inhibiting hepsin cleavage of human pro-uPA. Said Kunitz domain sequence could be one or both of the Kunitz domains of HAI-2. In one embodiment, an antagonist of the disclosure comprises a variant HAI-2 Kunitz domain sequence having at least about 70%, 75%, 80%, 85%, 90%, 95%, 97%, 98%, 99% sequence identity with the corresponding Kunitz domain(s) of wild type human HAI-2, wherein said variant sequence has at least comparable ability as wild type HAI-2 in inhibiting hepsin cleavage of human pro-uPA. In one embodiment, an antagonist of the disclosure comprises a variant HAI-2 Kunitz domain sequence having between about 70% and 99%, about 75% and 98%, about 80% and 97%, 85% and 95% sequence identity with the corresponding Kunitz domain(s) of wild type human HAI-2, wherein said sequence has at least comparable ability as wild type HAI-2 in inhibiting hepsin cleavage of human ro-uPA.

An antagonist of the disclosure is or comprises a small molecule, peptide, antibody, antibody fragment, aptamer, or a combination thereof. Antagonists as described herein can be routinely obtained using techniques known in the art (including those described in greater detail below) based on the discovery of the interaction of hepsin and pro-uP as described herein. For example, an antagonist of the disclosure competes with hepsin for binding to pro-uPA, but does not have ability to cleave pro-uPA at the hepsin cleavage site (e.g., at $Lys_{158}$-$Ile_{159}$). An antagonist of the disclosure competes with pro-uPA for binding to hepsin. The antagonist of the invention comprises of an amino acid sequence having at least about 60%, 70%, 80%, 90%, 95%, 98%, 99% sequence similarity or identity with respect to pro-uPA (e.g., human pro-uPA) and is capable of substantially binding hepsin, but lacks a hepsin cleavage site (e.g., pro-uPA $Lys_{158}$-$Ile_{159}$ peptide link). An antagonist of the disclosure comprises, consists or consists essentially of a pro-uPA fragment capable of binding hepsin, wherein said fragment is devoid of at least a portion of a uPA sequence associated with enzymatic activity.

Thus, the disclosure provides a pro-uPA mutant capable of substantially binding hepsin but has decreased uPA enzymatic activity compared to wild type uPA, e.g. an antagonist of uPA activity or a uPA variant exhibiting a reduction, but not an absence, of uPA enzymatic activity. An antagonist of the disclosure is capable of inhibiting the biological activity of wild type (in vitro or in vivo) uPA (such biological activity includes but is not limited to enzymatic activity with respect to plasminogen as a substrate). An antagonist of the disclosure provides reduced uPA enzymatic activity and/or plasminogen activation.

An antagonist of the disclosure is obtained by a screening or identification method of the invention as described herein.

An antagonist molecule of the disclosure is linked to a toxin such as a cytotoxic agent. These molecules can be formulated or administered in combination with an additive/enhancing agent, such as a radiation and/or chemotherapeutic agent.

The disclosure provides use of an antagonist of the invention in the preparation of a medicament for the therapeutic and/or prophylactic treatment of a disease, such as a cancer, a tumor, a cell proliferative disorder, or a disorder associated with disruption/degradation of basement membrane and/or matrix degradation. The antagonist can be of any form described herein, including antibody, antibody fragment, small molecule (e.g., an organic molecule), polypeptide (e.g., an oligopeptide), nucleic acid (e.g., an oligonucleotide, such as an antisense oligonucleotide or small interfering RNA), an aptamer, or combination thereof.

In one aspect, the disclosure provides use of a nucleic acid of the invention in the preparation of a medicament for the therapeutic and/or prophylactic treatment of a disease, such as a cancer, a tumor, a cell proliferative disorder, or a disorder associated with disruption/degradation of basement membrane and/or matrix degradation.

The disclosure provides a method of therapeutically treating a mammal having a cancerous tumor associated with pro-uPA activation, said method comprising administering to said mammal an effective amount of an antagonist of the invention, thereby effectively treating said mammal.

In one aspect, the invention provides compositions comprising one or more antagonists of the invention and a carrier. In one embodiment, the carrier is pharmaceutically acceptable.

The disclosure provides nucleic acids encoding an antagonist of the invention. In one embodiment, a nucleic acid of the invention encodes an antagonist which is or comprises a polypeptide (e.g., an oligopeptide). In one embodiment, a nucleic acid of the invention encodes an antagonist which is or comprises an antibody or fragment thereof.

Embodiments of the invention are:

1. An antagonist molecule that inhibits hepsin activation of pro-urokinase type plasminogen activator (pro-uPA) for use in a method of treating cancer in a subject, wherein the antagonist molecule comprises:

(i) a polypeptide comprising a Kunitz domain (KD) sequence;

(ii) at least a portion of human hepatocyte growth factor activator inhibitor-1, -IB, or -2 (HAI-1, HAI-IB or HAI-2);

(iii) an amino acid sequence having at least about 60%, 70%, 80%, 90%, 95% or 99% sequence identity to pro-uPA and which is capable of binding hepsin and which lacks a hepsin cleavage site.

2. The antagonist molecule as defined in claim 1 for use according to embodiment 1, wherein the Kunitz domain (KD) sequence comprises a Kunitz domain 1 sequence (KD-1).

3. The antagonist molecule as defined in claim 1 for use according to embodiment 1, wherein the portion of human hepatocyte growth factor activator inhibitor-1, -IB, or -2 (HAI-1, HAI-IB or HAI-2) comprises a Kunitz domain (KD) sequence.

4. The antagonist molecule as defined in claim 1 for use according to embodiment 3, wherein the Kunitz domain (KD) sequence is:

(i) Kunitz domain 1 sequence (KD-1) of human hepatocyte growth factor activator inhibitor-1, -IB (HAI-1, HAI-IB), or variant thereof; or

(ii) one or both of the Kunitz domains of human hepatocyte growth factor activator inhibitor-2 (HAI-2), or variant thereof.

5. The antagonist molecule as defined in claim 1 for use according to embodiment 1, wherein a hepsin cleavage site is pro-uPA LySI5s-11e159 peptide link.

6. The antagonist molecule as defined in claim 1 for use according to embodiment 1 or 5, wherein the amino acid sequence having at least about 60%, 70%, 80%, 90%, 95% or 99% sequence identity to pro-uPA and which is capable of binding hepsin and which lacks a hepsin cleavage site lacks uPA activity.

7. The antagonist molecule as defined in claim 1 for use according to embodiment 6, wherein the molecule is a mutant wherein the b chain is mutated or is devoid of at least a portion of uPA sequence associated with activity.

8. The antagonist molecule as defined in claim 1 for use according to any one of embodiments 1 to 7, wherein the molecule is linked to a toxin.

9. The antagonist molecule as defined in claim 1 for use according to embodiment 8, wherein the toxin is a cytotoxic agent.

10. The antagonist molecule as defined in claim 1 for use according to any one of embodiments 1 to 9, wherein the method further comprises administering radiation or a chemotherapeutic agent.

11. The antagonist molecule as defined in claim 1 for use according to any one of embodiments 1 to 10, wherein the cancer is prostate or ovarian.

12. A composition comprising a molecule as defined in any one of embodiments 1 to 9 and a carrier, for use in treatment as defined in any one of embodiments 1 or 11.

5

13. The composition for use according to embodiment 12, wherein the carrier is a pharmaceutically acceptable carrier.

## BRIEF DESCRIPTION OF THE DRAWINGS

FIGURE 1. Cleavage of pro-uPA by hepsin. (A) Pro-uPA (1.5 $\mu$M) was incubated with 15 nM hepsin or 15 nM plasmin or buffer (control) for 4 min and 60 min at room temperature. Converted uPA was analyzed by SDS-PAGE under reducing (R) and non-reducing (NR) conditions followed by protein staining. (B) Pro-tPA (1.5 $\mu$M) was incubated with 15 nM hepsin or 15 nM plasmin of buffer (control) for 10 min and 60 min at room temperature and analyzed by SDS-PAGE under reducing (R) conditions. The molecular weight standards are shown as $M_r$ x $10^3$.

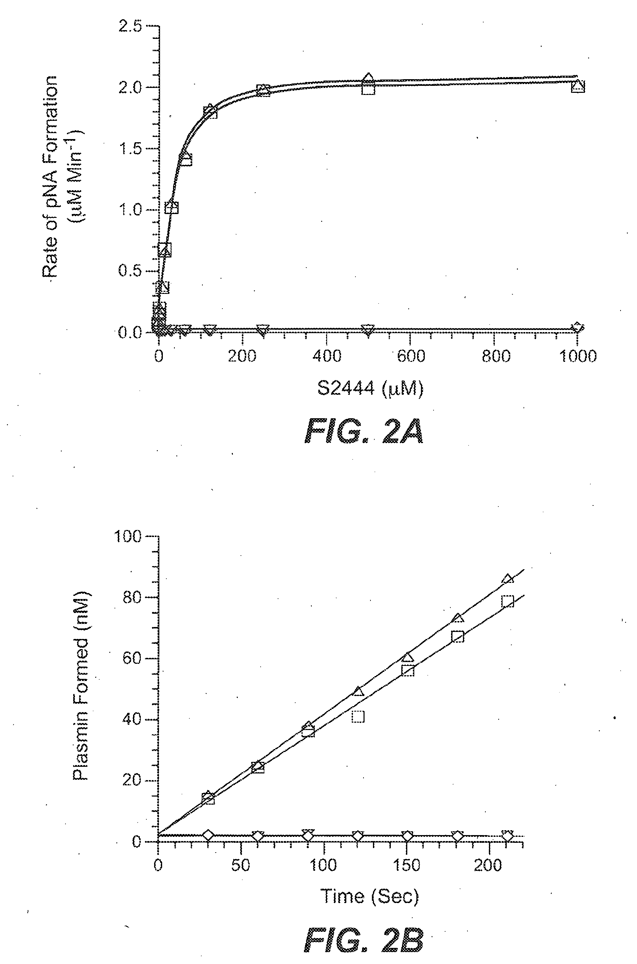

FIGURE 2. Enzymatic activity of pro-uPA cleaved by hepsin. Pro-uPA was completely converted to uPA by hepsin or plasmin and the products (uPA$_{Hepsin}$ and uPA$_{Plasmin}$) were analyzed for their enzymatic activity. The controls contained hepsin or plasmin but no pro-uPA (hepsin and plasmin control, respectively). (A) A representative experiment showing cleavage of the small synthetic para-nitroanilide (pNA) substrate S2444. The rates of pNA formation are shown as a function of [S2444]. triangles, uPA$_{Hepsin}$; squares, uPA$_{Plasmin}$; inverted triangles, hepsin control; diamonds, plasmin control; (B) A representative experiment showing the initial linear rates of plasminogen activation. Symbols as in (A).

FIGURE 3. First-order kinetics of pro-uPA activation by hepsin and other trypsin-like serine proteases. Pro-uPA (30 nM) was incubated with enzymes (3 nM) and the amount of cleaved pro-uPA was determined at various time points (see 'Experimental Procedures'). The figure shows the time-dependent decrease of [pro-uPA] by hepsin (triangles), plasmin (squares), matriptase (circles) and HGFA (diamonds); average of 5 experiments $\pm$ SD.

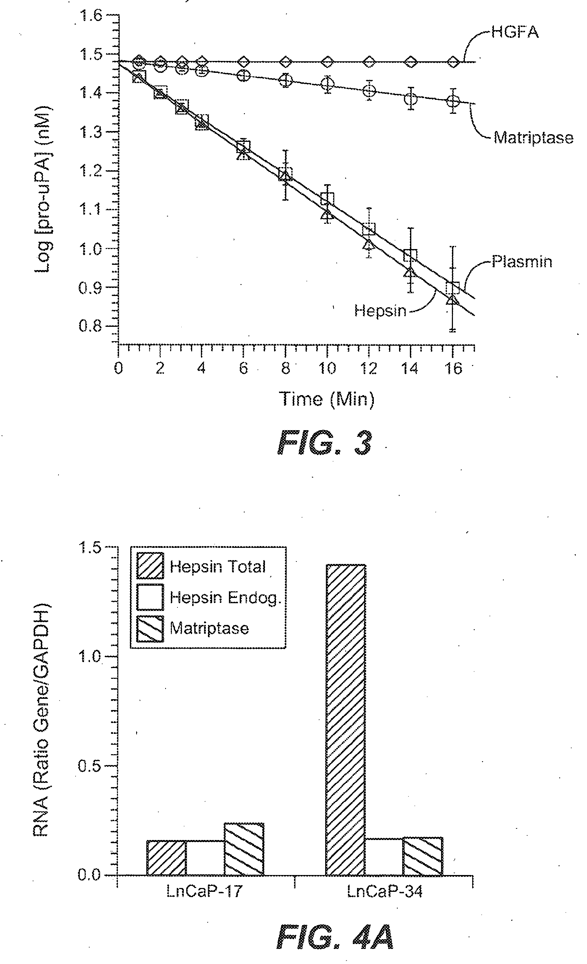

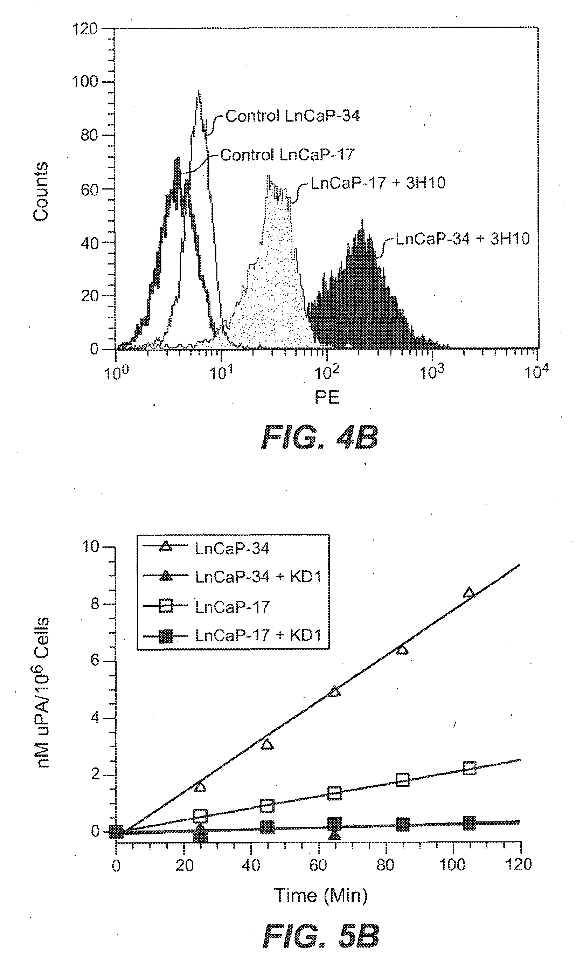

FIGURE 4. Expression of hepsin mRNA and protein by LnCaP cells. The cell lines LnCaP-17 and the hepsin-overexpressing LnCaP-34 were established as described in 'Experimental Procedures'. (A) RNA levels of total hepsin (= endogenous + transfected hepsin) (grey), endogenous hepsin (striped), and matriptase (black) as determined by real time RT-PCR. (B) Cell surface expression of full-length hepsin by LnCaP cells. Hepsin was detected by the monoclonal antibody 3H10 using FACS. Grey lines/no fill, LnCaP-17 control (secondary antibody only); grey fill, LnCaP-17 + 3H10; black lines/no fill, LnCaP-34 control (secondary antibody only); black fill, LnCaP-34 + 3H10.

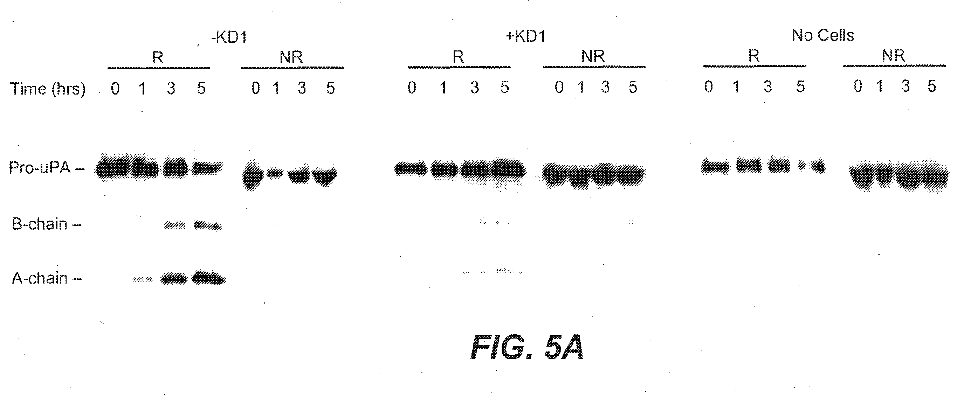

FIGURE 5. Pro-uPA conversion by LnCaP cells. (A) LnCaP-34 (hepsin-overexpressing) cells were allowed to process pro-uPA (30 nM) for 1 h, 3 h and 5 h in the presence or absence of KD1 inhibitor. The reaction product was analyzed by SDS-PAGE under reducing (R) and non-reducing (NR) conditions followed by immunoblotting using an anti-uPA polyclonal antibody. Left panel, cells/no KD1; middle panel, cells/+KD1; right panel, no cells/no KD1. The position of zymogen, uPA A-chain and uPA B-chain (protease domain) are indicated. (B) Kinetics of pro-uPA activation by LnCaP-17 and LnCaP-34 cells. Confluent cell layers were incubated with 100 nM pro-uPA at 37° C and aliquots taken at different time points. The concentration of formed uPA was determined in the second stage of the assay. Triangles, LnCaP-34; squares, LnCaP-17; filled triangles, LnCaP-34 + 1 $\mu$M KD1; filled squares, LnCaP-17 + 1 $\mu$M KD1.

FIGURE 6. Model of the $P_4$ - $P_1$ residues of pro-uPA in the hepsin active site.

FIGURE 7. One embodiment of an amino acid sequence of native human hepsin (SEQ ID NO:1).

FIGURE 8. (A) & (B) Another embodiment of an amino acid sequence of native human hepsin (SEQ ID NO:2).

FIGURE 9. One embodiment of an amino acid sequence of native human pro-urokinase plasminogen activator (pro-uPA)

FIGURE 10. Chemical structure of small molecule inhibitor HI-10331.

## MODES FOR CARRYING OUT THE INVENTION

The invention provides methods, compositions, kits and articles of manufacture comprising modulators of the HGF/c-met signaling pathway, including methods of using such modulators.

Details of these methods, compositions, kits and articles of manufacture are provided herein.

*General Techniques*

The practice of the present invention will employ, unless otherwise indicated, conventional techniques of molecular biology (including recombinant techniques), microbiology, cell biology, biochemistry, and immunology, which are within the skill of the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Current Protocols in Molecular Biology" (F. M. Ausubel et al., eds., 1987, and periodic updates); "PCR: The Polymerase Chain Reaction", (Mullis et al., ed.,

1994); "A Practical Guide to Molecular Cloning" (Perbal Bernard V., 1988); "Phage Display: A Laboratory Manual" (Barbas et al., 2001).

*Definitions*

The term "hepsin" as used herein encompasses native sequence polypeptides, polypeptide variants and fragments of a native sequence polypeptide and polypeptide variants (which are further defined herein) that is capable of pro-uPA cleavage in a manner similar to wild type hepsin. The hepsin polypeptide described herein may be that which is isolated from a variety of sources, such as from human tissue types or from another source, or prepared by recombinant or synthetic methods. The terms "hepsin", "hepsin polypeptide", "hepsin enzyme", and "hepsin protein" also include variants of a hepsin polypeptide as disclosed herein.

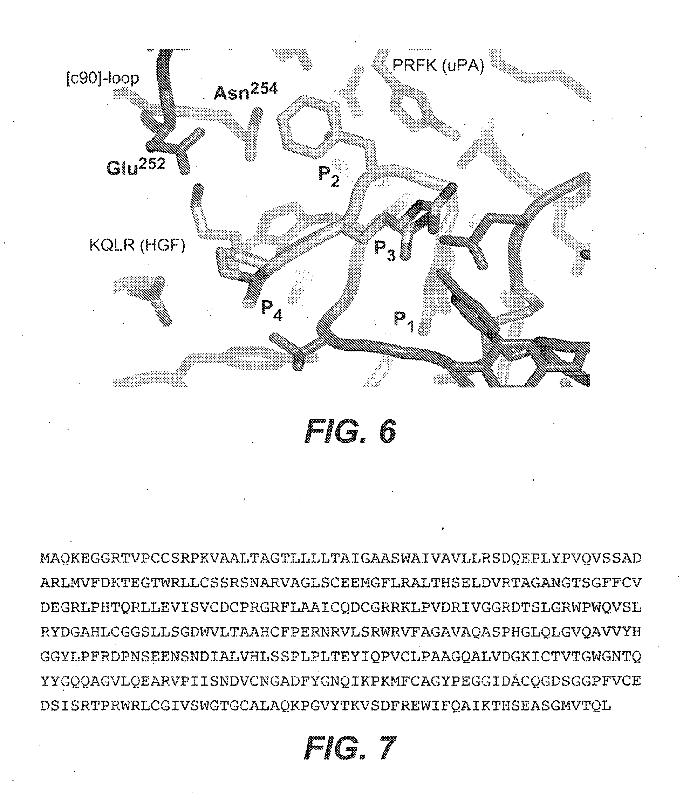

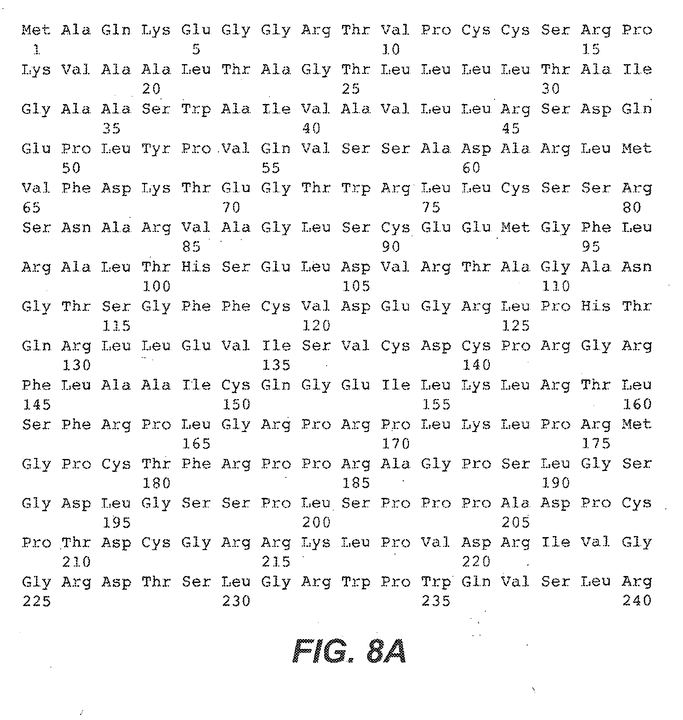

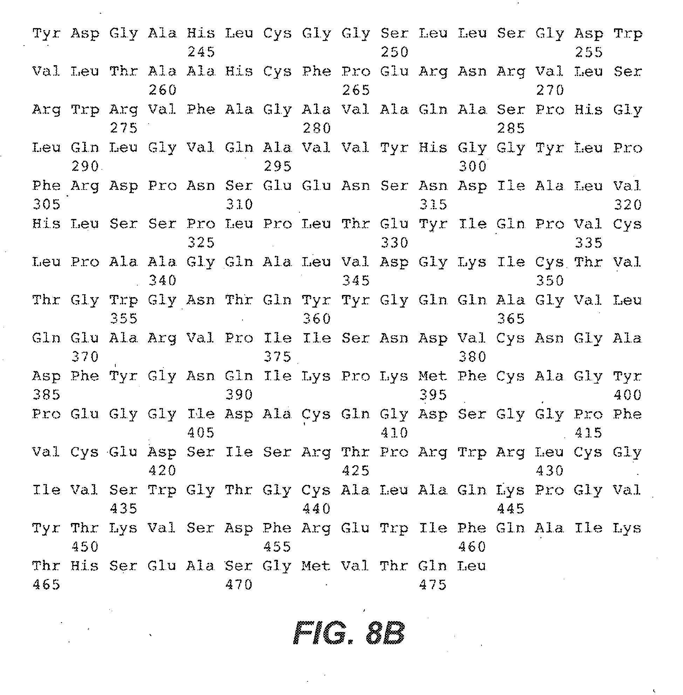

A "native sequence hepsin polypeptide" comprises a polypeptide having the same amino acid sequence as the corresponding hepsin polypeptide derived from nature. In one embodiment, a native sequence hepsin polypeptide comprises the amino acid sequence of SEQ ID NO:1 (see Figure 7). In one embodiment, a native sequence hepsin polypeptide comprises the amino acid sequence of SEQ ID NO:2 (see Figure 8). Such native sequence hepsin polypeptide can be isolated from nature or can be produced by recombinant or synthetic means. The term "native sequence hepsin polypeptide" specifically encompasses naturally-occurring truncated or secreted forms of the specific hepsin polypeptide (e.g., an extracellular domain sequence), naturally-occurring variant forms (*e.g.*, alternatively spliced forms) and naturally-occurring allelic variants of the polypeptide.

"Hepsin polypeptide variant", or variations thereof, means a hepsin polypeptide, generally an active hepsin polypeptide, as defined herein having at least about 80% amino acid sequence identity with any of the native sequence hepsin polypeptide sequences as disclosed herein. Such hepsin polypeptide variants include, for instance, hepsin polypeptides wherein one or more amino acid residues are added, or deleted, at the N- or C-terminus of a native amino acid sequence. Ordinarily, a hepsin polypeptide variant will have at least about 80% amino acid sequence identity, alternatively at least about 81%, 82%, 83%, 84%, 85%, 86%, 87%, 88%, 89%, 90%, 91%, 92%, 93%, 94%, 95%, 96%, 97%, 98%, or 99% amino acid sequence identity, to a native sequence hepsin polypeptide sequence as disclosed herein. Ordinarily, hepsin variant polypeptides are at least about 10 amino acids in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 amino acids in length, or more. Optionally, hepsin variant polypeptides will have no more than one conservative amino acid substitution as compared to a native hepsin polypeptide sequence, alternatively no more than 2, 3, 4, 5, 6, 7, 8, 9, or 10 conservative amino acid substitution as compared to the native hepsin polypeptide sequence.

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, as described in US Pat. No. 6,828,146.

The term "vector," as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "recombinant vectors"). In general, expression vectors of utility in recombinant DNA techniques are often in the form of plasmids. In the present specification, "plasmid" and "vector" may be used interchangeably as the plasmid is the most commonly used form of vector.

"Polynucleotide," or "nucleic acid," as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase, or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may be further modified after synthesis, such as by conjugation with a label. Other types of modifications include, for example, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example, proteins (e.g., nucleases, toxins, antibodies, signal peptides, ply-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl, 2'-fluoro- or 2'-azido-ribose, carbocyclic sugar analogs, alpha-anomeric sugars, epimeric sugars such as arabinose, xyloses or lyxoses, pyranose sugars, furanose sugars, sedoheptuloses, acyclic analogs and abasic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), "(O)NR$_2$ ("amidate"), P(O)R, P(O)OR', CO or CH$_2$ ("formacetal"), in which each R or R' is independently H or substituted or unsubstituted alkyl (1-20 C.) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, generally refers to short, generally single stranded, generally synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

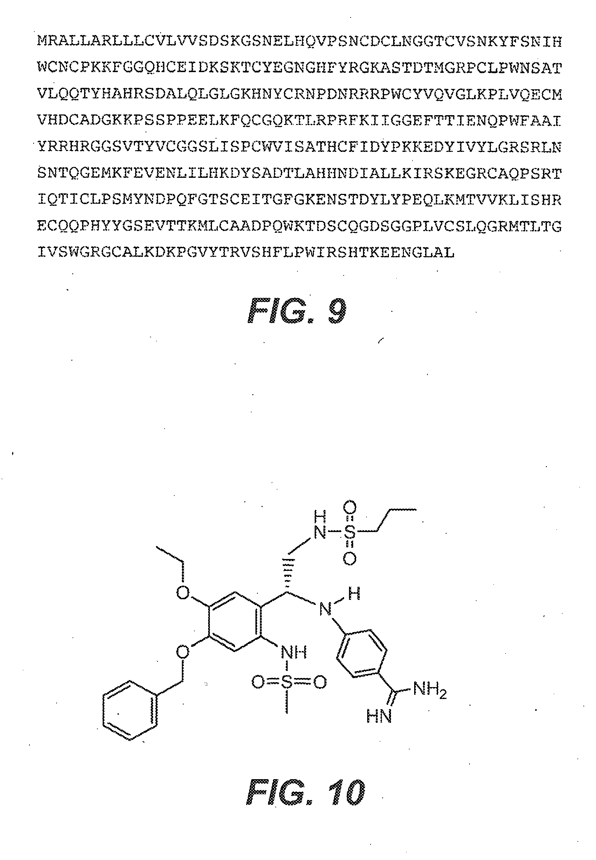

The term "urokinase-type plasminogen activator" and "uPA", or "pro-urokinase-type plasminogen activator" and "pro-uPA", as used herein, refers, unless specifically or contextually indicated otherwise, to any native or variant (whether naturally occurring or synthetic) uPA polypeptide that is capable of, or a uPA polypeptide that can be activated by hepsin into activated uPA that is capable of, activating plasminogen under conditions that permit such process to occur. The term "wild type uPA" generally refers to a polypeptide comprising the amino acid sequence of a naturally occurring uPA protein, for example as set forth in Figure 9 and listed at Accession No. NM_002658.

The terms "antibody" and "immunoglobulin" are used interchangeably in the broadest sense and include monoclonal antibodies (for *e.g.,* full length or intact monoclonal antibodies), polyclonal antibodies, multivalent antibodies, multispecific antibodies (e.g., bispecific antibodies so long as they exhibit the desired biological activity) and may also include certain antibody fragments (as described in greater detail herein). An antibody can be human, humanized and/or affinity matured.

"Antibody fragments" comprise only a portion of an intact antibody, wherein the portion preferably retains at least one, preferably most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half life modulation, ADCC function and complement binding. In one embodiment, an antibody fragment is a monovalent antibody that has an in vivo half life substantially similar to an intact antibody. For e.g., such an antibody fragment may comprise on antigen binding arm linked to an Fc sequence capable of conferring in vivo stability to the fragment.

The term "monoclonal antibody" as used herein refers to an antibody obtained from a population of substantially homogeneous antibodies, *i.e.*, the individual antibodies comprising the population comprise essentially identical amino acid sequence except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al., Proc. Natl. Acad. Sci. USA 81:6851-6855 (1984)).

"Humanized" forms of non-human (*e.g.,* murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FRs are those of a human immunoglobulin sequence. The humanized antibody optionally will also comprise at least a portion of an immunoglobulin constant region (Fc), typically that of a human immunoglobulin. For further details, see Jones et al., Nature 321:522-525 (1986); Riechmann et al., Nature 332:323-329 (1988); and Presta, Curr. Op. Struct. Biol. 2:593-596 (1992). See also the following review articles and references cited therein: Vaswani and Hamilton, Ann. Allergy, Asthma & Immunol. 1:105-1.15 (1998); Harris, Biochem. Soc. Transactions 23:1035-1038 (1995); Hurle and Gross, Curr. Op. Biotech. 5:428-433 (1994).

A "human antibody" is one which possesses an amino acid sequence which corresponds to that of an antibody produced by a human and/or has been made using any of the techniques for making human antibodies as disclosed herein. This definition of a human antibody specifically excludes a humanized antibody comprising non-human antigen-binding residues.

An "affinity matured" antibody is one with one or more alterations in one or more CDRs thereof which result in an improvement in the affinity of the antibody for antigen, compared to a parent antibody which does not possess those alteration(s). Preferred affinity matured antibodies will have nanomolar or even picomolar affinities for the target antigen. Affinity matured antibodies are produced by procedures known in the art. Marks et al. Bio/Technology 10:779-783 (1992) describes affinity maturation by VH and VL domain shuffling. Random mutagenesis of CDR and/or framework residues is described by: Barbas et al. Proc Nat. Acad. Sci, USA 91:3809-3813 (1994); Schier et al. Gene 169:147-155 (1995); Yelton et al. J. Immunol, 155:1994-2004 (1995); Jackson et al., J. Immunol. 154(7):3310-9 (1995); and Hawkins et al, J. Mol. Biol. 226:889-896 (1992).

A "blocking" antibody or an "antagonist" antibody is one which inhibits or reduces biological activity of the antigen it binds. Preferred blocking antibodies or antagonist antibodies substantially or completely inhibit the biological activity of the antigen.

An "agonist antibody", as used herein, is an antibody which mimics at least one of the functional activities of a polypeptide of interest.

A "disorder" is any condition that would benefit from treatment with a composition or method of the invention. This includes chronic and acute disorders or diseases including those pathological conditions which predispose the mammal to the disorder in question. Non-limiting examples of disorders to be treated herein include malignant and benign tumors; non-leukemias and lymphoid malignancies; neuronal, glial, astrocytal, hypothalamic and other glandular, macrophagal, epithelial, stromal and blastocoelic disorders; and other angiogenesis-related disorders.

The terms "cell proliferative disorder" and "proliferative disorder" refer to disorders that are associated with some degree of abnormal cell proliferation. In one embodiment, the cell proliferative disorder is cancer.

"Tumor", as used herein, refers to all neoplastic cell growth and proliferation, whether malignant or benign, and all pre-cancerous and cancerous cells and tissues. The terms "cancer", "cancerous", "cell proliferative disorder", "proliferative disorder" and "tumor" are not mutually exclusive as referred to herein.

The terms "cancer" and "cancerous" refer to or describe the physiological condition in mammals that is typically characterized by unregulated cell growth/proliferation and/or invasiness. Examples of cancer include but are not limited to, carcinoma, lymphoma, blastoma, sarcoma, and leukemia. More particular examples of such cancers include squamous cell cancer, small-cell lung cancer, non-small cell lung cancer, adenocarcinoma of the lung, squamous carcinoma of the lung, cancer of the peritoneum, hepatocellular cancer, gastrointestinal cancer, pancreatic cancer, glioblastoma, cervical cancer, ovarian cancer, liver cancer, bladder cancer, hepatoma, breast cancer, colon cancer, colorectal cancer, endometrial or uterine carcinoma, salivary gland carcinoma, kidney cancer, liver cancer, prostate cancer, vulval cancer, thyroid cancer, hepatic carcinoma and various types of head and neck cancer.

As used herein, "treatment" refers to clinical intervention in an attempt to alter the natural course of the individual or cell being treated, and can be performed either for prophylaxis or during the course of clinical pathology. Desirable effects of treatment include preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, compositions and/or methods of the invention are used to delay development of a disease or disorder.

An "effective amount" refers to an amount effective, at dosages and for periods of time necessary, to achieve the desired therapeutic or prophylactic result.

A "therapeutically effective amount" of a molecule (e.g., antagonist) of the invention may vary according to factors such as the disease state, age, sex, and weight of the individual, and the ability of the molecule (e.g., antagonist) to elicit a desired response in the individual. A therapeutically effective amount is also one in which any toxic or detrimental effects of the molecule (e.g., antagonist) are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "cytotoxic agent" as used herein refers to a substance that inhibits or prevents the function of cells and/or causes destruction of cells. The term is intended to include radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$ and radioactive isotopes of Lu), chemotherapeutic agents e.g. methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" is a chemical compound useful in the treatment of cancer. Examples of chemotherapeutic agents include alkylating agents such as thiotepa and CYTOXAN® cyclosphosphamide; alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethiylenethiophosphoramide and trimethylolomelamine; acetogenins (especially bullatacin and bullatacinone); delta-9-tetrahydrocannabinol (dronabinol, MARINOL®); beta-lapachone; lapachol; colchicines: betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CBI-TM1); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, and ranimnustine; antibiotics such as the enediyne antibiotics (e.g., calicheamicin, especially calicheamicin gammaII and calicheamicin omegaI1 (see, e.g., Agnew, Chem Intl. Ed. Engl., 33: 183-186 (1994)); dynemicin, including dynemicin A; an esperamicin; as well as neocarzinostatin chromophore and related chromoprotein enediyne antiobiotic chromophores), aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®) and deoxydoxorubicin), epirubicin, esorubicin, idarubicin, marcellomycin, mitomycins such as mitomycin C, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti- adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoids, e.g., paclitaxel (TAXOL®), albumin-engineered nanoparticle formulation of paclitaxel (ABRAXANE™), and doxetaxel (TAXOTERE®); chloranbucil; 6-thioguanine; mercaptopurine; methotrexate; platinum analogs such as cisplatin and carboplatin; vinblastine (VELBAN®); platinum; etoposide (VP-16); ifosfamide; mitoxantrone; vincristine (ONCOVIN®); oxaliplatin; leucovovin; vinorelbine (NAVELBINE®); novantrone; edatrexate; daunomycin; aminopterin; ibandronate; topoisomerase inhibitor RFS 2000; difluorometlhylornithine (DMFO); retinoids such as retinoic acid; pharmaceutically acceptable salts, acids or derivatives of any of the above; as well as combinations of two or more of the above such as CHOP, an abbreviation for a combined therapy of cyclophosphamide, doxorubicin, vincristine, and prednisolone, and FOLFOX, an abbreviation for a treatment regimen with oxaliplatin (ELOXATIN™) combined with 5-FU and leucovovin.

Also included in this definition are anti-hormonal agents that act to regulate, reduce, block, or inhibit the effects of hormones that can promote the growth of cancer, and are often in the form of systemic, or whole-body treatment. They may be hormones themselves. Examples include anti-estrogens and selective estrogen receptor modulators (SERMs), including, for example, tamoxifen (including NOLVADEX® tamoxifen), raloxifene (EVISTA®), droloxifene, 4-hydroxytamoxifen, trioxifene, keoxifene, LY117018, onapristone, and toremifene (FARESTON®); anti-progesterones; estrogen receptor down-regulators (ERDs); estrogen receptor antagonists such as fulvestrant (FASLODEX®); agents that function to suppress or shut down the ovaries, for example, leutinizing hormone-releasing hormone (LHRH) agonists such as leuprolide acetate (LUPRON® and ELIGARD®), goserelin acetate, buserelin acetate and tripterelin; other anti-androgens such as flutamide, nilutamide and bicalutamide; and aromatase inhibitors that inhibit the enzyme aromatase, which regulates estrogen production in the adrenal glands, such as, for example, 4(5)-imidazoles, aminoglutethimide, megestrol acetate (MEGASE®), exemestane (AROMASIN®), formestanie, fadrozole, vorozole (RIVISOR®), letrozole (FEMARA®), and anastrozole (ARIMIDEX®). In addition, such definition of chemotherapeutic agents includes bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); as well as troxacitabine (a 1,3-dioxolane nucleoside cytosine analog); antisense oligonucleotides, particularly those that inhibit expression of genes in signaling pathways implicated in abherant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); lapatinib ditosylate (an ErbB-2 and EGFR dual tyrosine kinase small-molecule inhibitor also known as GW572016); COX-2 inhibitors such as celecoxib (CELEBREX®; 4-(5-(4-methylphenyl)-3-(trifluoromethyl)-1H-pyrazol-1-yl) benzenesulfonamide; and pharmaceutically acceptable salts, acids or derivatives of any of the above.

A "growth inhibitory agent" when used herein refers to a compound or composition which inhibits growth of a cell whose growth is dependent upon uPA activation either *in vitro* or *in vivo.* Thus, the growth inhibitory agent may be one which significantly reduces the percentage of uPA-dependent cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

"Doxorubicin" is an anthracycline antibiotic. The full chemical name of doxorubicin is (8S-cis)-10-[(3-amino-2,3,6-trideoxy-$\alpha$-L-lyxo-hexapyranosyl)oxy]-7,8,9,10-tetrahydro-6,8,11-trihydroxy-8-(hydroxyacetyl)-1-methoxy-5,12-naphthacenedione.

Compositions and Methods of the Invention

A. Antibodies

In one embodiment, the present invention provides antagonist antibodies which may find use herein as therapeutic and/or diagnostic agents. Exemplary antibodies include polyclonal, monoclonal, humanized, bispecific, and heteroconjugate antibodies.

1. Polyclonal Antibodies

Polyclonal antibodies are preferably raised in animals by multiple subcutaneous (sc) or intraperitoneal (ip) injections of the relevant antigen and an adjuvant. It may be useful to conjugate the relevant antigen (especially when synthetic peptides are used) to a protein that is immunogenic in the species to be immunized. For example, the antigen can be conjugated to keyhole limpet hemocyanin (KLH), serum albumin, bovine thyroglobulin, or soybean trypsin inhibitor, using a bifunctional or derivatizing agent, e.g., maleimidobenzoyl sulfosuccinimide ester (conjugation through cysteine residues), N-hydroxysuccinimide (through lysine residues), glutaraldehyde, succinic anhydride, $SOCl_2$, or $R^1N=C=NR$,

where R and R$^1$ are different alkyl groups.

Animals are immunized against the antigen, immunogenic conjugates, or derivatives by combining, e.g., 100 μg or 5 μg of the protein or conjugate (for rabbits or mice, respectively) with 3 volumes of Freund's complete adjuvant and injecting the solution intradermally at multiple sites. One month later, the animals are boosted with 1/5 to 1/10 the original amount of peptide or conjugate in Freund's complete adjuvant by subcutaneous injection at multiple sites. Seven to 14 days later, the animals are bled and the serum is assayed for antibody titer. Animals are boosted until the titer plateaus. Conjugates also can be made in recombinant cell culture as protein fusions. Also, aggregating agents such as alum are suitably used to enhance the immune response.

2. Monoclonal Antibodies

Monoclonal antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as described above to elicit lymphocytes that produce or are capable of producing antibodies that will specifically bind to the protein used for immunization. Alternatively, lymphocytes may be immunized *in vitro.* After immunization, lymphocytes are isolated and then fused with a myeloma cell line using a suitable fusing agent, such as polyethylene glycol, to form a hybridoma cell (Goding, Monoclonal Antibodies: Principles and Practice, pp.59-103 (Academic Press, 1986)).

The hybridoma cells thus prepared are seeded and grown in a suitable culture medium which medium preferably contains one or more substances that inhibit the growth or survival of the unfused, parental myeloma cells (also referred to as fusion partner). For example, if the parental myeloma cells lack the enzyme hypoxanthine guanine phosphoribosyl transferase (HGPRT or HPRT), the selective culture medium for the hybridomas typically will include hypoxanthine, aminopterin, and thymidine (HAT medium), which substances prevent the growth of HGPRT-deficient cells.

Preferred fusion partner myeloma cells are those that fuse efficiently, support stable high-level production of antibody by the selected antibody-producing cells, and are sensitive to a selective medium that selects against the unfused parental cells. Preferred myeloma cell lines are murine myeloma lines, such as those derived from MOPC-21 and MPC-11 mouse tumors available from the Salk Institute Cell Distribution Center, San Diego, California USA, and SP-2 and derivatives e.g., X63-Ag8-653 cells available from the American Type Culture Collection, Manassas, Virginia, USA. Human myeloma and mouse-human heteromyeloma cell lines also have been described for the production of human monoclonal antibodies (Kozbor, J. Immunol., 133:3001 (1984); and Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen. Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The binding affinity of the monoclonal antibody can, for example, be determined by the Scatchard analysis described in Munson et al., Anal. Biochem., 107:220 (1980).

Once hybridoma cells that produce antibodies of the desired specificity, affinity, and/or activity are identified, the clones may be subcloned by limiting dilution procedures and grown by standard methods (Goding, Monoclonal Antibodies: Principles and Practice, pp.59-103 (Academic Press, 1986)). Suitable culture media for this purpose include, for example, D-MEM or RPMI-1640 medium. In addition, the hybridoma cells may be grown *in vivo* as ascites tumors in an animal e.g., by i.p. injection of the cells into mice.

The monoclonal antibodies secreted by the subclones are suitably separated from the culture medium, ascites fluid, or serum by conventional antibody purification procedures such as, for example, affinity chromatography (e.g., using protein A or protein G-Sepharose) or ion-exchange chromatography, hydroxylapatite chromatography, gel electrophoresis, dialysis, etc.

DNA encoding the monoclonal antibodies is readily isolated and sequenced using conventional procedures (e.g., by using oligonucleotide probes that are capable of binding specifically to genes encoding the heavy and light chains of murine antibodies). The hybridoma cells serve as a preferred source of such DNA. Once isolated, the DNA may be placed into expression vectors, which are then transfected into host cells such as *E. coli* cells, simian COS cells, Chinese Hamster Ovary (CHO) cells, or myeloma cells that do not otherwise produce antibody protein, to obtain the synthesis of monoclonal antibodies in the recombinant host cells. Review articles on recombinant expression in bacteria of DNA encoding the antibody include Skerra et al., Curr. Opinion in Immunol., 5:256-262 (1993) and Plückthun, Immunol. Revs. 130:151-188 (1992).

In a further embodiment, monoclonal antibodies or antibody fragments can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) describe the isolation of murine and human antibodies, respectively, using phage libraries. Subsequent publications describe the production of high affinity (nM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res. 21:2265-2266 (1993)). Thus, these techniques are viable alternatives to traditional monoclonal antibody hybridoma techniques for isolation of monoclonal antibodies.

The DNA that encodes the antibody may be modified to produce chimeric or fusion antibody polypeptides, for example, by substituting human heavy chain and light chain constant domain ($C_H$ and $C_L$) sequences for the homologous murine sequences (U.S. Patent No. 4,816,567; and Morrison, et al., Proc. Natl Acad. Sci. USA, 81:6851 (1984)), or by fusing the immunoglobulin coding sequence with all or part of the coding sequence for a non-immunoglobulin polypeptide (heterologous polypeptide). The non-immunoglobulin polypeptide sequences can substitute for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

### 3. Human and Humanized Antibodies

The antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity and HAMA response (human anti-mouse antibody) when the antibody is intended for human therapeutic use. According to the so-called "best-fit" method, the sequence of the variable domain of a rodent antibody is screened against the entire library of known human variable domain sequences. The human V domain sequence which is closest to that of the rodent is identified and the human framework region (FR) within it accepted for the humanized antibody (Sims et al., J. Immunol. 151:2296 (1993); Chothia et al., J. Mol. Biol., 196:901 (1987)). Another method uses a particular framework region derived from the consensus sequence of all human antibodies of a particular subgroup of light or heavy chains. The same framework may be used for several different humanized antibodies (Carter et al., Proc. Natl. Acad. Sci. USA, 89:4285 (1992); Presta et al., J. Immunol. 151:2623 (1993)).

It is further important that antibodies be humanized with retention of high binding affinity for the antigen and other favorable biological properties. To achieve this goal, according to a preferred method, humanized antibodies are prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

Various forms of a humanized antibody are contemplated. For example, the humanized antibody may be an antibody fragment, such as a Fab, which is optionally conjugated with one or more cytotoxic agent(s) in order to generate an immunoconjugate. Alternatively, the humanized antibody may be an intact antibody, such as an intact IgG1 antibody.

As an alternative to humanization, human antibodies can be generated. For example, it is now possible to produce transgenic animals (e.g., mice) that are capable, upon immunization, of producing a full repertoire of human antibodies in the absence of endogenous immunoglobulin production. For example, it has been described that the homozygous deletion of the antibody heavy-chain joining region ($J_H$) gene in chimeric and germ-line mutant mice results in complete inhibition of endogenous antibody production. Transfer of the human germ-line immunoglobulin gene array into such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggemann et al., Year in Immuno. 7:33 (1993); U.S. Patent Nos. 5,545,806, 5,569,825, 5,591,669 (all of GenPharm); 5,545,807; and WO 97/17852.

Alternatively, phage display technology (McCafferty et al., Nature 348:552-553 [1990]) can be used to produce human antibodies and antibody fragments *in vitro,* from immunoglobulin variable (V) domain gene repertoires from unimmunized donors. According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, e.g., Johnson, Kevin S. and Chiswell, David J., Current Opinion in Structural Biology 3:564-571 (1993). Several sources of V-gene segments can be used for phage display. Clackson et al., Nature, 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). See, also, U.S. Patent Nos. 5,565,332 and 5,573,905.

As discussed above, human antibodies may also be generated by *in vitro* activated B cells (see U.S. Patents 5,567,610 and 5,229,275).

### 4. Antibody fragments

In certain circumstances there are advantages of using antibody fragments, rather than whole antibodies. The smaller size of the fragments allows for rapid clearance, and may lead to improved access to solid tumors.

Various techniques have been developed for the production of antibody fragments. Traditionally, these fragments were derived via proteolytic digestion of intact antibodies (see, e.g., Morimoto et al., Journal of Biochemical and Biophysical Methods 24:107-117 (1992); and Brennan et al., Science, 229:81 (1985)). However, these fragments can now be produced directly by recombinant host cells. Fab, Fv and ScFv antibody fragments can all be expressed in and secreted from *E. coli,* thus allowing the facile production of large amounts of these fragments. Antibody fragments can be isolated from the antibody phage libraries discussed above. Alternatively, Fab'-SH fragments can be directly recovered from *E. coli* and chemically coupled to form F(ab')$_2$ fragments (Carter et al., Bio/Technology 10:163-167 (1992)). According to another approach, F(ab')$_2$ fragments can be isolated directly from recombinant host cell culture. Fab and F(ab')$_2$ fragment with increased in vivo half-life comprising a salvage receptor binding epitope residues are described in U.S. Patent No. 5,869,046. Other techniques for the production of antibody fragments will be apparent to the skilled practitioner. In other embodiments, the antibody of choice is a single chain Fv fragment (scFv). See WO 93/16185; U.S. Patent No. 5,571,894; and U.S. Patent No. 5,587,458. Fv and sFv are the only species with intact combining sites that are devoid of constant regions; thus, they are suitable for reduced nonspecific binding during in vivo use. sFv fusion proteins may be constructed to yield fusion of an effector protein at either the amino or the carboxy terminus of an sFv. See Antibody Engineering, ed. Borrebaeck, supra. The antibody fragment may also be a "linear antibody", e.g., as described in U.S. Patent 5,641,870 for example. Such linear antibody fragments may be monospecific or bispecific.

### 5. Bispecific Antibodies

Bispecific antibodies are antibodies that have binding specificities for at least two different epitopes. Exemplary bispecific antibodies may bind to two different epitopes of hepsin, uPA and/or hepsin:uPA complex as described herein. Other such antibodies may combine a binding site on these entities with a binding site for another polypeptide. Alternatively, an antibody arm may be combined with an arm which binds to a triggering molecule on a leukocyte such as a T-cell receptor molecule (e.g. CD3), or Fc receptors for IgG (FcγR), such as FcγRI (CD64), FcγRII (CD32) and FcγRIII (CD16), so as to focus and localize cellular defense mechanisms to the hepsin and/or uPA-expressing and/or binding cell. Bispecific antibodies may also be used to localize cytotoxic agents to cells which express and/or bind hepsin, uPA and/or hepsin:uPA complex. These antibodies possess a polypeptide binding arm and an arm which binds the cytotoxic agent (e.g., saporin, anti-interferon-$\alpha$, vinca alkaloid, ricin A chain, methotrexate or radioactive isotope hapten). Bispecific antibodies can be prepared as full length antibodies or antibody fragments (e.g., $F(ab')_2$ bispecific antibodies).

WO 96/16673 describes a bispecific anti-ErbB2/anti-Fc$\gamma$RIII antibody and U.S. Patent No. 5,837,234 discloses a bispecific anti-ErbB2/anti-FcγRI antibody. A bispecific anti-ErbB2/Fc$\alpha$ antibody is shown in WO98/02463. U.S. Patent No. 5,821,337 teaches a bispecific anti-ErbB2/anti-CD3 antibody.

Methods for making bispecific antibodies are known in the art. Traditional production of full length bispecific antibodies is based on the co-expression of two immunoglobulin heavy chain-light chain pairs, where the two chains have different specificities (Millstein et al., Nature 305:537-539 (1983)). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker et al., EMBO J. 10:3655-3659 (1991).

According to a different approach, antibody variable domains with the desired binding specificities (antibody-antigen combining sites) are fused to immunoglobulin constant domain sequences. Preferably, the fusion is with an Ig heavy chain constant domain, comprising at least part of the hinge, $C_H2$, and $C_H3$ regions. It is preferred to have the first heavy-chain constant region ($C_H1$) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments in embodiments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant affect on the yield of the desired chain combination.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details of generating bispecific antibodies see, for example, Suresh et al., Methods in Enzymology 121:210 (1986).

According to another approach described in U.S. Patent No. 5,731,168, the interface between a pair of antibody molecules can be engineered to maximize the percentage of heterodimers which are recovered from recombinant cell culture. The preferred interface comprises at least a part of the $C_H3$ domain. In this method, one or more small amino acid side chains from the interface of the first antibody molecule are replaced with larger side chains (e.g., tyrosine or tryptophan). Compensatory "cavities" of identical or similar size to the large side chain(s) are created on the interface of the second antibody molecule by replacing large amino acid side chains with smaller ones (e.g., alanine or threonine). This provides a mechanism for increasing the yield of the heterodimer over other unwanted end-products such as homodimers.

Bispecific antibodies include cross-linked or "heteroconjugate" antibodies. For example, one of the antibodies in the heteroconjugate can be coupled to avidin, the other to biotin. Such antibodies have, for example, been proposed to target immune system cells to unwanted cells (U.S. Patent No. 4,676,980), and for treatment of HIV infection (WO 91/00360, WO 92/200373, and EP 03089). Heteroconjugate antibodies may be made using any convenient cross-linking methods. Suitable cross-linking agents are well known in the art, and are disclosed in U.S. Patent No. 4,676,980, along with a number of cross-linking techniques.

Techniques for generating bispecific antibodies from antibody fragments have also been described in the literature. For example, bispecific antibodies can be prepared using chemical linkage. Brennan et al., Science 229:81 (1985) describe a procedure wherein intact antibodies are proteolytically cleaved to generate $F(ab')_2$ fragments. These fragments are reduced in the presence of the dithiol complexing agent, sodium arsenite, to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Recent progress has facilitated the direct recovery of Fab'-SH fragments from *E. coli,* which can be chemically coupled to form bispecific antibodies. Shalaby et al., J. Exp. Med. 175: 217-225 (1992) describe the production of a fully humanized bispecific antibody $F(ab')_2$ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets. Various techniques for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a $V_H$ connected to a $V_L$ by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See Gruber et al., J. Immunol., 152:5368 (1994).

Antibodies with more than two valencies are contemplated. For example, trispecific antibodies can be prepared. Tutt et al., J. Immunol. 147:60 (1991).

### 6. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 7. Multivalent Antibodies

A multivalent antibody may be internalized (and/or catabolized) faster than a bivalent antibody by a cell expressing an antigen to which the antibodies bind. The antibodies of the present invention can be multivalent antibodies (which are other than of the IgM class) with three or more antigen binding sites (e.g. tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises (or consists of) an Fc region or a hinge region. In this scenario, the antibody will comprise an Fc region and three or more antigen binding sites amino-terminal to the Fc region. The preferred multivalent antibody herein comprises (or consists of) three to about eight, but preferably four, antigen binding sites. The multivalent antibody comprises at least one polypeptide chain (and preferably two polypeptide chains), wherein the polypeptide chain(s) comprise two or more variable domains. For instance, the polypeptide chain(s) may comprise VD1-$(X1)_n$-VD2-$(X2)_n$-Fc, wherein VD1 is a first variable domain, VD2 is a second variable domain, Fc is one polypeptide chain of an Fc region, X1 and X2 represent an amino acid or polypeptide, and n is 0 or 1. For instance, the polypeptide chain(s) may comprise: VH-CH1-flexible linker-VH-CH1-Fc region chain; or VH-CH1-VH-CH1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### 8. Effector Function Engineering

It may be desirable to modify the antibody of the invention with respect to effector function, e.g., so as to enhance antigen-dependent cell-mediated cyotoxicity (ADCC) and/or complement dependent cytotoxicity (CDC) of the antibody. This may be achieved by introducing one or more amino acid substitutions in an Fc region of the antibody. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced antitumor activity may also be prepared using heterobifunctional cross-linkers as described in Wolff et al., Cancer Research 53:2560-2565 (1993). Alternatively, an antibody can be engineered which has dual Fc regions and may thereby have enhanced complement lysis and ADCC capabilities. See Stevenson et al., Anti-Cancer Drug Design 3:219-230 (1989). To increase the serum half life of the antibody, one may incorporate a salvage receptor binding epitope into the antibody (especially an antibody fragment) as described in U.S. Patent 5,739,277, for example. As used herein, the term "salvage receptor binding epitope" refers to an epitope of the Fc region of an IgG molecule (e.g., $IgG_1$, $IgG_2$, $IgG_3$, or $IgG_4$) that is responsible for increasing the *in vivo* serum half-life of the IgG molecule.

### 9. Immunoconjugates

The invention also pertains to immunoconjugates, or antibody-drug conjugates (ADC), comprising an antibody conjugated to a cytotoxic agent such as a chemotherapeutic agent, a drug, a growth inhibitory agent, a toxin (*e.g.,* an enzymatically active toxin of bacterial, fungal, plant, or animal origin, or fragments thereof), or a radioactive isotope (*i.e.,* a radioconjugate).

The use of antibody-drug conjugates for the local delivery of cytotoxic or cytostatic agents, i.e. drugs to kill or inhibit tumor cells in the treatment of cancer (Syrigos and Epenetos (1999) Anticancer Research 19:605-614; Niculescu-Duvaz and Springer (1997) Adv. Drg Del. Rev. 26:151-172; U.S. patent 4,975,278) theoretically allows targeted delivery of the drug moiety to tumors, and intracellular accumulation therein, where systemic administration of these unconjugated drug agents may result in unacceptable levels of toxicity to normal cells as well as the tumor cells sought to be eliminated (Baldwin et al., (1986) Lancet pp. (Mar. 15, 1986):603-05; Thorpe, (1985) "Antibody Carriers Of Cytotoxic Agents In Cancer Therapy: A Review," in Monoclonal Antibodies '84: Biological And Clinical Applications, A. Pinchera et al. (ed.s), pp. 475-506). Maximal efficacy with minimal toxicity is sought thereby. Both polyclonal antibodies and monoclonal antibodies have been reported as useful in these strategies (Rowland et al., (1986) Cancer Immunol. Immunother., 21:183-87). Drugs used in these methods include daunomycin, doxorubicin, methotrexate, and vindesine (Rowland et al., (1986) *supra*). Toxins used in antibody-toxin conjugates include bacterial toxins such as diphtheria toxin, plant toxins such as ricin, small molecule toxins such as geldanamycin (Mandler et al (2000) Jour. of the Nat. Cancer Inst. 92(19):1573-1581; Mandler et al (2000) Bioorganic & Med. Chem. Letters 10:1025-1028; Mandler et al (2002) Bioconjugate Chem. 13:786-791), maytansinoids (EP 1391213; Liu et al., (1996) Proc. Natl. Acad. Sci. USA 93:8618-8623), and calicheamicin (Lode et al (1998) Cancer Res. 58:2928; Hinman et al (1993) Cancer Res. 53:3336-3342). The toxins may effect their cytotoxic and cytostatic effects by mechanisms including tubulin binding, DNA binding, or topoisomerase inhibition. Some cytotoxic drugs tend to be inactive or less active when conjugated to large antibodies or protein receptor ligands.

ZEVALIN® (ibritumomab tiuxetan, Biogen/Idec) is an antibody-radioisotope conjugate composed of a murine IgG1 kappa monoclonal antibody directed against the CD20 antigen found on the surface of normal and malignant B lymphocytes and $^{111}In$ or $^{90}Y$ radioisotope bound by a thiourea linker-chelator (Wiseman et al (2000) Eur. Jour. Nucl. Med. 27(7):766-77; Wiseman et al (2002) Blood 99(12):4336-42; Witzig et al (2002) J. Clin. Oncol. 20(10):2453-63; Witzig et al (2002) J. Clin. Oncol. 20(15):3262-69). Although ZEVALIN has activity against B-cell non-Hodgkin's Lymphoma (NHL), administration results in severe and prolonged cytopenias in most patients. MYLOTARG™ (gemtuzumab ozogamicin, Wyeth Pharmaceuticals), an antibody drug conjugate composed of a hu CD33 antibody linked to calicheamicin, was approved in 2000 for the treatment of acute myeloid leukemia by injection (Drugs of the Future (2000) 25(7):686; US Patent Nos. 4970198; 5079233; 5585089; 5606040; 5693762; 5739116; 5767285; 5773001). Cantuzumab mertansine (Immunogen, Inc.), an antibody drug conjugate composed of the huC242 antibody linked via the disulfide linker SPP to the maytansinoid drug moiety, DM1, is advancing into Phase II trials for the treatment of cancers that express CanAg, such as colon, pancreatic, gastric, and others. MLN-2704 (Millennium Phann., BZL Biologics, Immunogen Inc.), an antibody drug conjugate composed of the anti-prostate specific membrane antigen (PSMA) monoclonal antibody linked to the maytansinoid drug moiety, DM1, is under development for the potential treatment of prostate tumors. The auristatin peptides, auristatin E (AE) and monomethylauristatin (MMAE), synthetic analogs of dolastatin, were conjugated to chimeric monoclonal antibodies cBR96 (specific to Lewis Y on carcinomas) and cAC10 (specific to CD30 on hematological malignancies) (Doronina et al (2003) Nature Biotechnology 21 (7):778-784) and are under therapeutic development.

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca american* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}Bi$, $^{131}I$, $^{131}In$, $^{90}Y$, and $^{186}Re$. Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCI), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026.

Conjugates of an antibody and one or more small molecule toxins, such as a calicheamicin, maytansinoids, a trichothecene, and CC1065, and the derivatives of these toxins that have toxin activity, are also contemplated herein.

Maytansine and maytansinoids

In one embodiment, an antibody (full length or fragments) of the invention is conjugated to one or more maytansinoid molecules.

Maytansinoids are mitototic inhibitors which act by inhibiting tubulin polymerization. Maytansine was first isolated from the east African shrub *Maytenus serrata* (U.S. Patent No. 3,896,111). Subsequently, it was discovered that certain microbes also produce maytansinoids, such as maytansinol and C-3 maytansinol esters (U.S. Patent No. 4,151,042). Synthetic maytansinol and derivatives and analogues thereof are disclosed, for example, in U.S. Patent Nos. 4,137,230; 4,248,870; 4,256,746; 4,260,608; 4,265,814; 4,294,757; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533.

Maytansinoid-antibody conjugates

In an attempt to improve their therapeutic index, maytansine and maytansinoids have been conjugated to antibodies specifically binding to tumor cell antigens. Immunoconjugates containing maytansinoids and their therapeutic use are disclosed, for example, in U.S. Patent Nos. 5,208,020, 5,416,064 and European Patent EP 0 425 235 B1. Liu et al., Proc. Natl. Acad. Sci. USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an *in vivo* tumor growth assay. Chari et al., Cancer Research 52:127-131 (1992) describe immunoconjugates in which a maytansinoid was conjugated via a disulfide linker to the murine antibody A7 binding to an antigen on human colon cancer cell lines, or to another murine monoclonal antibody TA.1 that binds the HER-2/*neu* oncogene. The cytotoxicity of the TA.1-maytansonoid conjugate was tested *in vitro* on the human breast cancer cell line SK-BR-3, which expresses 3 x $10^5$ HER-2 surface antigens per cell. The drug conjugate achieved a degree of cytotoxicity similar to the free maytansinoid drug, which could be increased by increasing the number of maytansinoid molecules per antibody molecule. The A7-maytansinoid conjugate showed low systemic cytotoxicity in mice.

Antibody-maytansinoid conjugates (immunoconjugates)

Antibody-maytansinoid conjugates are prepared by chemically linking an antibody to a maytansinoid molecule without significantly diminishing the biological activity of either the antibody or the maytansinoid molecule. An average of 3-4 maytansinoid molecules conjugated per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent No. 5,208,020 and in the other patents and nonpatent publications referred to hereinabove. Preferred maytansinoids are maytansinol and maytansinol analogues modified in the aromatic ring or at other positions of the maytansinol molecule, such as various maytansinol esters.

There are many linking groups known in the art for making antibody-maytansinoid conjugates, including, for example, those disclosed in U.S. Patent No. 5,208,020 or EP Patent 0 425 235 B1, and Chari et al., Cancer Research 52:127-131 (1992). The linking groups include disulfide groups, thioether groups, acid labile groups, photolabile groups, peptidase labile groups, or esterase labile groups, as disclosed in the above-identified patents, disulfide and thioether groups being preferred.

Conjugates of the antibody and maytansinoid may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). Particularly preferred coupling agents include N-succinimidyl-3-(2-pyridyldithio) propionate (SPDP) (Carlsson et al., Biochem. J. 173:723-737 [1978]) and N-succinimidyl-4-(2-pyridylthio)pentanoate (SPP) to provide for a disulfide linkage.

The linker may be attached to the maytansinoid molecule at various positions, depending on the type of the link. For example, an ester linkage may be formed by reaction with a hydroxyl group using conventional coupling techniques. The reaction may occur at the C-3 position having a hydroxyl group, the C-14 position modified with hydroxymethyl, the C-15 position modified with a hydroxyl group, and the C-20 position having a hydroxyl group. In a preferred embodiment, the linkage is formed at the C-3 position of maytansinol or a maytansinol analogue.

Calicheamicin

Another immunoconjugate of interest comprises an antibody conjugated to one or more calicheamicin molecules. The calicheamicin family of antibiotics are capable of producing double-stranded DNA breaks at sub-picomolar concentrations. For the preparation of conjugates of the calicheamicin family, see U.S. patents 5,712,374, 5,714,586, 5,739,116, 5,767,285, 5,770,701, 5,770,710, 5,773,001, 5,877,296 (all to American Cyanamid Company). Structural analogues of calicheamicin which may be used include, but are not limited to, $\gamma_1^I$, $\alpha_2^I$, $\alpha_3^I$, N-acetyl-$\gamma_1^I$, PSAG and $\theta_1^I$ (Hinman et al., Cancer Research 53:3336-3342 (1993), Lode et al., Cancer Research 58:2925-2928 (1998) and the aforementioned U.S. patents to American Cyanamid). Another anti-tumor drug that the antibody can be conjugated is QFA which is an antifolate. Both calicheamicin and QFA have intracellular sites of action and do not readily cross the plasma membrane. Therefore, cellular uptake of these agents through antibody mediated internalization greatly enhances their cytotoxic effects.

Other cytotoxic agents

Other antitumor agents that can be conjugated to the antibodies of the invention include BCNU, streptozoicin, vincristine and 5-fluorouracil, the family of agents known collectively LL-E33288 complex described in U.S. patents 5,053,394, 5,770,710, as well as esperamicins (U.S. patent 5,877,296).

Enzymatically active toxins and fragments thereof which can be used include diphtheria A chain, nonbinding active fragments of diphtheria toxin, exotoxin A chain (from *Pseucdonaonas aeruginosa*), ricin A chain, abrin A chain, modeccin A chain, alpha-sarcin, *Aleurites fordii* proteins, dianthin proteins, *Phytolaca american* proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published October 28, 1993.

The present invention further contemplates an immunoconjugate formed between an antibody and a compound with nucleolytic activity (e.g., a ribonuclease or a DNA endonuclease such as a deoxyribonuclease; DNase).

For selective destruction of the tumor, the antibody may comprise a highly radioactive atom. A variety of radioactive isotopes are available for the production of radioconjugated antibodies. Examples include $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu. When the conjugate is used for detection, it may comprise a radioactive atom for scintigraphic studies, for example $tc^{99m}$ or $I^{123}$, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

The radio- or other labels may be incorporated in the conjugate in known ways. For example, the peptide may be biosynthesized or may be synthesized by chemical amino acid synthesis using suitable amino acid precursors involving, for example, fluorine-19 in place of hydrogen. Labels such as $tc^{99m}$ or $I^{123}$, $Re^{186}$, $Re^{188}$ and $In^{111}$ can be attached via a cysteine residue in the peptide. Yttrium-90 can be attached via a lysine residue. The IODOGEN method (Fraker et al (1978) Biochem. Biophys. Res. Commun. 80: 49-57 can be used to incorporate iodine-123. "Monoclonal Antibodies in Immunoscintigraphy" (Chatal,CRC Press 1989) describes other methods in detail.

Conjugates of the antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate, iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, photolabile linker, dimethyl linker or disulfide-containing linker (Chari et al., Cancer Research 52:127-131 (1992); U.S. Patent No. 5,208,020) may be used.

The compounds of the invention expressly contemplate, but are not limited to, ADC prepared with cross-linker reagents: BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A). See pages 467-498, 2003-2004 Applications Handbook and Catalog.

PREPARATION OF ANTIBODY DRUG CONJUGATES

In the antibody drug conjugates (ADC) of the invention, an antibody (Ab) is conjugated to one or more drug moieties (D), e.g. about 1 to about 20 drug moieties per antibody, through a linker (L). The ADC of Formula I may be prepared by several routes, employing organic chemistry reactions, conditions, and reagents known to those skilled in the art, including: (1) reaction of a nucleophilic group of an antibody with a bivalent linker reagent, to form Ab-L, via a covalent bond, followed by reaction with a drug moiety D; and (2) reaction of a nucleophilic group of a drug moiety with a bivalent linker reagent, to form D-L, via a covalent bond, followed by reaction with the nucleophilic group of an antibody.

$$Ab\text{-}(L\text{-}D)_p \qquad I$$

Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol.

Antibody drug conjugates of the invention may also be produced by modification of the antibody to introduce electrophilic moieties, which can react with nucleophilic subsituents on the linker reagent or drug. The sugars of glycosylated antibodies may be oxidized, e.g. with periodate oxidizing reagents, to form aldehyde or ketone groups which may react with the amine group of linker reagents or drug moieties. The resulting imine Schiff base groups may form a stable linkage, or may be reduced, e.g. by borohydride reagents to form stable amine linkages. In one embodiment, reaction of the carbohydrate portion of a glycosylated antibody with either glactose oxidase or sodium meta-periodate may yield carbonyl (aldehyde and ketone) groups in the protein that can react with appropriate groups on the drug (Hermanson, Bioconjugate Techniques). In another embodiment, proteins containing N-terminal serine or threonine residues can react with sodium meta-periodate, resulting in production of an aldehyde in place of the first amino acid (Geoghegan & Stroh, (1992) Bioconjugate Chem. 3:138-146; US 5362852). Such aldehyde can be reacted with a drug moiety or linker nucleophile.

Likewise, nucleophilic groups on a drug moiety include, but are not limited to: amine, thiol, hydroxyl, hydrazide, oxime, hydrazine, thiosemicarbazone, hydrazine carboxylate, and arylhydrazide groups capable of reacting to form covalent bonds with electrophilic groups on linker moieties and linker reagents including: (i) active esters such as NHS esters, HOBt esters, haloformates, and acid halides; (ii) alkyl and benzyl halides such as haloacetamides; (iii) aldehydes, ketones, carboxyl, and maleimide groups.

Alternatively, a fusion protein comprising the antibody and cytotoxic agent may be made, e.g., by recombinant techniques or peptide synthesis. The length of DNA may comprise respective regions encoding the two portions of the conjugate either adjacent one another or separated by a region encoding a linker peptide which does not destroy the desired properties of the conjugate.

In yet another embodiment, the antibody may be conjugated to a "receptor" (such streptavidin) for utilization in tumor pre-targeting wherein the antibody-receptor conjugate is administered to the patient, followed by removal of unbound conjugate from the circulation using a clearing agent and then administration of a "ligand" (e.g., avidin) which is conjugated to a cytotoxic agent (e.g., a radionucleotide).

10. Immunoliposomes

The antibodies disclosed herein may also be formulated as immunoliposomes. A "liposome" is a small vesicle composed of various types of lipids, phospholipids and/or surfactant which is useful for delivery of a drug to a mammal. The components of the liposome are commonly arranged in a bilayer formation, similar to the lipid arrangement of biological membranes. Liposomes containing the antibody are prepared by methods known in the art, such as described in Epstein et al., Proc. Natl. Acad. Sci. USA 82:3688 (1985); Hwang et al., Proc. Natl Acad. Sci. USA 77:4030 (1980); U.S. Pat. Nos. 4,485,045 and 4,544,545; and WO97/38731 published October 23, 1997. Liposomes with enhanced circulation time are disclosed in U.S. Patent No. 5,013,556.

Particularly useful liposomes can be generated by the reverse phase evaporation method with a lipid composition comprising phosphatidylcholine, cholesterol and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem. 257:286-288 (1982) via a disulfide interchange reaction. A chemotherapeutic agent is optionally contained within the liposome. See Gabizon et al., J. National Cancer Inst. 81(19):1484 (1989).

B. Binding Oligopeptides

Binding oligopeptides of the invention are oligopeptides that bind, preferably specifically, to hepsin, uPA and/or hepsin:uPA complex as described herein. Binding oligopeptides may be chemically synthesized using known oligopeptide synthesis methodology or may be prepared and purified using recombinant technology. Binding oligopeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such oligopeptides that are capable of binding, preferably specifically, to a target of interest. Binding oligopeptides may be identified without undue experimentation using well known techniques. In this regard, it is noted that techniques for screening oligopeptide libraries for oligopeptides that are capable of specifically binding to a target are well known in the art (see, e.g., U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Natl. Acad. Sci. U.S.A., 81:3998-4002 (1984); Geysen et al.. Proc. Natl. Acad. Sci. U.S.A., 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natl. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363, and Smith, G. P. (1991) Current Opin. Biotechnol., 2:668).

In this regard, bacteriophage (phage) display is one well known technique which allows one to screen large oligopeptide libraries to identify member(s) of those libraries which are capable of specifically binding to a target. Phage display is a technique by which variant polypeptides are displayed as fusion proteins to the coat protein on the surface of bacteriophage particles (Scott, J.K. and Smith, G. P. (1990) Science, 249: 386). The utility of phage display lies in the fact that large libraries of selectively randomized protein variants (or randomly cloned cDNAs) can be rapidly and efficiently sorted for those sequences that bind to a target molecule with high affinity. Display of peptide (Cwirla, S. E. et al. (1990) Proc. Natl. Acad. Sci. USA, 87:6378) or protein (Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natl. Acad. Sci. USA, 88:8363) libraries on phage have been used for screening millions of polypeptides or oligopeptides for ones with specific binding properties (Smith, G. P. (1991) Current Opin. Biotechnol., 2:668). Sorting phage libraries of random mutants requires a strategy for constructing and propagating a large number of variants, a procedure for affinity purification using the target receptor, and a means of evaluating the results of binding enrichments. U.S. Patent Nos. 5,223,409, 5,403,484, 5,571,689, and 5,663,143.

Although most phage display methods have used filamentous phage, lambdoid phage display systems (WO 95/34683; U.S. 5,627,024), T4 phage display systems (Ren et al., Gene, 215: 439 (1998); Zhu et al., Cancer Research, 58(15): 3209-3214 (1998); Jiang et al., Infection & Immunity, 65(11): 4770-4777 (1997); Ren et al., Gene, 195(2):303-311 (1997); Ren, Protein Sci., 5: 1833 (1996); Efimov et al., Virus Genes, 10: 173 (1995)) and T7 phage display systems (Smith and Scott, Methods in Enzymology, 217: 228-257 (1993); U.S. 5,766,905) are also known.

Many other improvements and variations of the basic phage display concept have now been developed. These improvements enhance the ability of display systems to screen peptide libraries for binding to selected target molecules and to display functional proteins with the potential of screening these proteins for desired properties. Combinatorial reaction devices for phage display reactions have been developed (WO 98/14277) and phage display libraries have been used to analyze and control bimolecular interactions (WO 98/20169; WO 98/20159) and properties of constrained helical peptides (WO 98/20036). WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. WO 97/46251 describes a method of biopanning a random phage display library with an affinity purified antibody and then isolating binding phage, followed by a micropanning process using microplate wells to isolate high affinity binding phage. The use of *Staphlylococcus aureus* protein A as an affinity tag has also been reported (Li et al. (1998) Mol Biotech., 9:187). WO 97/47314 describes the use of substrate subtraction libraries to distinguish enzyme specificities using a combinatorial library which may be a phage display library. A method for selecting enzymes suitable for use in detergents using phage display is described in WO 97/09446. Additional methods of selecting specific binding proteins are described in U.S. Patent Nos. 5,498,538, 5,432,018, and WO 98/15833.

Methods of generating peptide libraries and screening these libraries are also disclosed in U.S. Patent Nos. 5,723,286, 5,432,018, 5,580,717, 5,427,908, 5,498,530, 5,770,434, 5,734,018, 5,698,426, 5,763,192, and 5,723,323.

C. Binding small molecules

Binding small molecules are preferably organic molecules other than oligopeptides or antibodies as defined herein that bind, preferably specifically, to hepsin, uPA and/or hepsin:uPA complex as described herein. Binding organic small molecules may be identified and chemically synthesized using known methodology (see, e.g., PCT Publication Nos. WO00/00823 and WO00/39585). Binding organic small molecules are usually less than about 2000 daltons in size, alternatively less than about 1500, 750, 500, 250 or 200 daltons in size, wherein such organic small molecules that are capable of binding, preferably specifically, to a target as described herein may be identified without undue experimentation using well known techniques. In this regard, it is noted that techniques for screening organic small molecule libraries for molecules that are capable of binding to a target are well known in the art (see, e.g., PCT Publication Nos. WO00/00823 and WO00/39585). Binding organic small molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

D. Screening for Antibodies, Binding Oligopeptides and Binding small molecules With the Desired Properties

Techniques for generating antibodies, oligopeptides and small moleculesof the invention have been described above. One may further select antibodies, oligopeptides or other small molecules with certain biological characteristics, as desired.

The inhibitory effects of an antibody, oligopeptide or other small molecule of the invention may be assessed by methods known in the art, e.g., using cells which express hepsin and/or pro-uPA either endogenously or following transfection with the respective gene(s). For example, appropriate tumor cell lines, and hepsin and/or pro-uPA polypeptide-transfected cells may be treated with a monoclonal antibody, oligopeptide or other small molecule of the invention at various concentrations for a few days (e.g., 2-7) days and analyzed for biological activity(ies) known to be associated with uPA activation, including the enzymatic activities assessed according to the Examples below and those described in Andreasen et al., Int. J. Cancer (1997), 72(1):1-22; Dano et al., Thromb. Haemost. (2005), 93(4):676-681; Helenius et al., Cancer Res. (2001), 61(14):5340-5344; Knudsen et al., Adv. Cancer Res. (2004), 91:31067; Shen'g, S., Cancer Metastasis Rev. (2001), 20(3-4):287-296; Van Veldhuizen et al., Am. J. Med. Sci. (1996), 312(1):8-11. The antibody, binding oligopeptide or binding organic small molecule will inhibit activity of a hepsin and/or uPA-expressing tumor cell *in vitro or in vivo* by about 25-100% compared to the untreated tumor cell, more preferably, by about 30-100%, and even more preferably by about 50-100% or 70-100%, in one embodiment, at an antibody concentration of about 0.5 to 30 $\mu$g/ml. Activity inhibition can be measured at an antibody concentration of about 0.5 to 30 $\mu$g/ml or about 0.5 nM to 200 nM in cell culture or other suitable experimental system, where the activity inhibition is determined 1-10 days after exposure of the tumor cells to the antibody. The antibody is inhibitory *in vivo* if administration of the antibody at about 1 $\mu$g/kg to about 100 mg/kg body weight results in reduction in tumor size, reduction of tumor cell invasiveness, etc., within about 5 days to 3 months from the first administration of the antibody, preferably within about 5 to 30 days.

To screen for antibodies, oligopeptides or other organic small molecules which bind to an epitope on a target of interest, a routine cross-blocking assay such as that described in Antibodies, A Laboratory Manual, Cold Spring Harbor Laboratory, Ed Harlow and David Lane (1988), can be performed. This assay can be used to determine if a test antibody, oligopeptide or other organic small molecule binds the same site or epitope as a known antibody. Alternatively, or additionally, epitope mapping can be performed by methods known in the art. For example, the antibody sequence can be mutagenized such as by alanine scanning, to identify contact residues. The mutant antibody is initially tested for binding with polyclonal antibody to ensure proper folding. In a different method, peptides corresponding to different regions of a polypeptide can be used in competition assays with the test antibodies or with a test antibody and an antibody with a characterized or known epitope.

E. Antibody Dependent Enzyme Mediated Prodrug Therapy (ADEPT)

The antibodies of the present invention may also be used in ADEPT by conjugating the antibody to a prodrug-activating enzyme which converts a prodrug (e.g., a peptidyl chemotherapeutic agent, see WO81/01145) to an active anti-cancer drug. See, for example, WO 88/07378 and U.S. Patent No. 4,975,278.

The enzyme component of the immunoconjugate useful for ADEPT includes any enzyme capable of acting on a prodrug in such a way so as to covert it into its more active, cytotoxic form.

Enzymes that are useful in the method of this invention include, but are not limited to, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug, 5-fluorouracil; proteases, such as serratia protease, thermolysin, subtilisin, carboxypeptidases and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as β-galactosidase and neuraminidase useful for converting glycosylated prodrugs into free drugs; β-lactamase useful for converting drugs derivatized with β-lactams into free drugs; and penicillin amidases, such as penicillin V amidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes", can be used to convert the prodrugs of the invention into free active drugs (see, e.g., Massey, Nature 328:457-458 (1987)). Antibody-abzyme conjugates can be prepared as described herein for delivery of the abzyme to a tumor cell population.

The enzymes of this invention can be covalently bound to the antibodies by techniques well known in the art such as the use of the heterobifunctional crosslinking reagents discussed above. Alternatively, fusion proteins comprising at least the antigen binding region of an antibody of the invention linked to at least a functionally active portion of an enzyme of the invention can be constructed using recombinant DNA techniques well known in the art (see, e.g., Neuberger et al., Nature 312:604-608 (1984).

F. Antibody Variants

In addition to the antibodies described herein, it is contemplated that antibody variants can be prepared. Antibody variants can be prepared by introducing appropriate nucleotide changes into the encoding DNA, and/or by synthesis of the desired antibody. Those skilled in the art will appreciate that amino acid changes may alter post-translational processes of the antibody, such as changing the number or position of glycosylation sites or altering the membrane anchoring characteristics.

Variations in the antibodies described herein can be made, for example, using any of the techniques and guidelines for conservative and non-conservative mutations set forth, for instance, in U.S. Patent No. 5,364,934. Variations may be a substitution, deletion or insertion of one or more codons encoding the antibody that results in a change in the amino acid sequence as compared with the native sequence antibody or polypeptide. Optionally the variation is by substitution of at least one amino acid with any other amino acid in one or more of the domains of the antibody. Guidance in determining which amino acid residue may be inserted, substituted or deleted without adversely affecting the desired activity may be found by comparing the sequence of the antibody with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the parent sequence.

Antibody and polypeptide fragments are provided herein. Such fragments may be truncated at the N-terminus or C-terminus, or may lack internal residues, for example, when compared with a full length native antibody or protein. Certain fragments lack amino acid residues that are not essential for a desired biological activity of the antibody or polypeptide.

Antibody and polypeptide fragments may be prepared by any of a number of conventional techniques. Desired peptide fragments may be chemically synthesized. An alternative approach involves generating antibody or polypeptide fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired antibody or polypeptide fragment, by polymerase chain reaction (PCR). Oligonucleotides that define the desired termini of the DNA fragment are employed at the 5' and 3' primers in the PCR. Preferably, antibody and polypeptide fragments share at least one biological and/or immunological activity with the native antibody or polypeptide disclosed herein.

In particular embodiments, conservative substitutions of interest are shown in the table below under the heading of preferred substitutions. If such substitutions result in a change in biological activity, then more substantial changes, denominated exemplary substitutions in this table, or as further described below in reference to amino acid classes, are introduced and the products screened.

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| He (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |

| | | |
|---|---|---|
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Substantial modifications in function or immunological identity of the antibody or polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Amino acids may be grouped according to similarities in the properties of their side chains (in A. L. Lehninger, in Biochemistry, second ed., pp. 73-75, Worth Publishers, New York (1975)):

(1) non-polar: Ala (A), Val (V), Leu (L), Ile (1), Pro (P), Phe (F), Trp (W), Met (M)
(2) uncharged polar: Gly (G), Ser (S), Thr (T), Cys (C), Tyr (Y), Asn (N), Gln (Q)
(3) acidic: Asp (D), Glu (E)
(4) basic: Lys (K), Arg (R), His(H)

Alternatively, naturally occurring residues may be divided into groups based on common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the antibody or polypeptide variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244:1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

Any cysteine residue not involved in maintaining the proper conformation of the antibody or polypeptide also may be substituted, generally with serine, to improve the oxidative stability of the molecule and prevent aberrant crosslinking. Conversely, cysteine bond(s) may be added to the antibody or polypeptide to improve its stability (particularly where the antibody is an antibody fragment such as an Fv fragment).

A particularly preferred type of substitutional variant involves substituting one or more hypervariable region residues of a parent antibody (e.g., a humanized or human antibody). Generally, the resulting variant(s) selected for further development will have improved biological properties relative to the parent antibody from which they are generated. A convenient way for generating such substitutional variants involves affinity maturation using phage display. Briefly, several hypervariable region sites (e.g., 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (e.g., binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and antigen polypeptide. Such contact residues and neighboring residues are candidates for substitution according to the techniques elaborated herein. Once such variants are generated, the panel of variants is subjected to screening as described herein and antibodies with superior properties in one or more relevant assays may be selected for further development.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

G. Modifications of Antibodies and Polypeptides

Covalent modifications of antibodies and polypeptides are included within the scope of this invention. One type of covalent modification includes reacting targeted amino acid residues of an antibody or polypeptide with an organic derivatizing agent that is capable of reacting with selected side chains or the N- or C- terminal residues of the antibody or polypeptide. Derivatization with bifunctional agents is useful, for instance, for crosslinking antibody or polypeptide to a water-insoluble support matrix or surface for use in the method for purifying antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the $\alpha$-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the antibody or polypeptide included within the scope of this invention comprises altering the native glycosylation pattern of the antibody or polypeptide. "Altering the native glycosylation pattern" is intended for purposes herein to mean deleting one or more carbohydrate moieties found in native sequence antibody or polypeptide (either by removing the underlying glycosylation site or by deleting the glycosylation by chemical and/or enzymatic means), and/or adding one or more glycosylation sites that are not present in the native sequence antibody or polypeptide. In addition, the phrase includes qualitative changes in the glycosylation of the native proteins, involving a change in the nature and proportions of the various carbohydrate moieties present.

Glycosylation of antibodies and other polypeptides is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody or polypeptide is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody or polypeptide (for O-linked glycosylation sites). The antibody or polypeptide amino acid sequence may optionally be altered through changes at the DNA level, particularly by mutating the DNA encoding the antibody or polypeptide at preselected bases such that codons are generated that will translate into the desired amino acids.

Another means of increasing the number of carbohydrate moieties on the antibody or polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the antibody or polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of antibody or polypeptide comprises linking the antibody or polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337. The antibody or polypeptide also may be entrapped in microcapsules prepared, for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Oslo, A., Ed., (1980).

The antibody or polypeptide of the present invention may also be modified in a way to form chimeric molecules comprising an antibody or polypeptide fused to another, heterologous polypeptide or amino acid sequence.

In one embodiment, such a chimeric molecule comprises a fusion of the antibody or polypeptide with a tag polypeptide which provides an epitope to which an anti-tag antibody can selectively bind. The epitope tag is generally placed at the amino- or carboxyl- terminus of the antibody or polypeptide. The presence of such epitope-tagged forms of the antibody or polypeptide can be detected using an antibody against the tag polypeptide. Also, provision of the epitope tag enables the antibody or polypeptide to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biotogy, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an $\alpha$-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the antibody or polypeptide with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of an antibody or polypeptide in place of at least one variable region within an Ig molecule. In a particularly preferred embodiment, the immunoglobulin fusion includes the hinge, $CH_2$ and $CH_3$, or the hinge, $CH_1$, $CH_2$ and $CH_3$ regions of an IgG1 molecule. For the production of immunoglobulin fusions see also US Patent No. 5,428,130 issued June 27, 1995.

H. Preparation of Antibodies and Polypeptides

The description below relates primarily to production of antibodies and polypeptides by culturing cells transformed or transfected with a vector containing antibody- and polypeptide-encoding nucleic acid. It is, of course, contemplated that alternative methods, which are well known in the art, may be employed to prepare antibodies and polypeptides. For instance, the appropriate amino acid sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the antibody or polypeptide may be chemically synthesized separately and combined using chemical or enzymatic methods to produce the desired antibody or polypeptide.

1. Isolation of DNA Encoding Antibody or Polypeptide

DNA encoding antibody or polypeptide may be obtained from a cDNA library prepared from tissue believed to possess the antibody or polypeptide mRNA and to express it at a detectable level. Accordingly, human antibody or polypeptide DNA can be conveniently obtained from a cDNA library prepared from human tissue. The antibody- or polypeptide-encoding gene may also be obtained from a genomic library or by known synthetic procedures (e.g., automated nucleic acid synthesis).

Libraries can be screened with probes (such as oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding antibody or polypeptide is to use PCR methodology [Sambrook et al., supra; Dieffenbach et al., PCR Primer: A Laboratory Manual (Cold Spring Harbor Laboratory Press, 1995)].

Techniques for screening a cDNA library are well known in the art. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like $^{32}$P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

2. Selection and Transformation of Host Cells

Host cells are transfected or transformed with expression or cloning vectors described herein for antibody or polypeptide production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl$_2$, CaPO$_4$, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al.,

Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia,* e.g., *E. coli, Enterobacter, Erwinia, Klebsiella, Proteus, Salmonella,* e.g., *Salmonella typhimurium, Serratia,* e.g., *Serratia marcescans,* and *Shigella,* as well as *Bacilli* such as *B. subtilis* and *B. licheniformis* (e.g., *B. licheniformis* 41P disclosed in DD 266,710 published 12 April 1989), *Pseudomonas* such as *P. aeruginosa,* and *Streptomyces.* These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA ; E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan$^r$; E. coli* W3110 strain 37D6, which has the complete genotype *tonA ptr3 phoA E15 (argF-lac)169 degP ompT rbs7 ilvG kan$^r$; E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

Full length antibody, antibody fragments, and antibody fusion proteins can be produced in bacteria, in particular when glycosylation and Fc effector function are not needed, such as when the therapeutic antibody is conjugated to a cytotoxic agent (e.g., a toxin) and the immunoconjugate by itself shows effectiveness in tumor cell destruction. Full-length antibodies have greater half life in circulation. Production in E. coli is faster and more cost efficient. For expression of antibody fragments and polypeptides in bacteria, see, e.g., U.S. 5,648,237 (Carter et. al.), U.S. 5,789,199 (Joly et al.), and U.S. 5,840,523 (Simmons et al.) which describes translation initiation regio (TIR) and signal sequences for optimizing expression and secretion. After expression, the antibody is isolated from the E. coli cell paste in a soluble fraction and can be purified through, e.g., a protein A or G column depending on the isotype. Final purification can be carried out similar to the process for purifying antibody expressed e.g" in CHO cells.

In addition to prokaryotes, eukaryotic microbes such as filamentous fungi or yeast are suitable cloning or expression hosts for antibody- or polypeptide-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactic* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans,* and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis,* and *Rhodotorula.* A list of specific species that are exemplary of this class of yeasts may be found in C. Anthony, The Biochemistry of Methylotrophs, 269 (1982).

Suitable host cells for the expression of glycosylated antibody or polypeptide are derived from multicellular organisms. Examples of invertebrate cells include insect cells such as Drosophila S2 and Spodoptera Sf9, as well as plant cells, such as cell cultures of cotton, corn, potato, soybean, petunia, tomato, and tobacco. Numerous baculoviral strains and variants and corresponding permissive insect host cells from hosts such as *Spocloptera frugiperda* (caterpillar), *Aedes aegypti* (mosquito), *Aedes albopictus* (mosquito), *Drosophila melanogaster* (fruitfly), and *Bombyx mori* have been identified. A variety of viral strains for transfection are publicly available, e.g., the L-1 variant of *Autographa californica* NPV and the Bm-5 strain of *Bombyx mori* NPV, and such viruses may be used as the virus herein according to the present invention, particularly for transfection of *Spodoptera frugiperda* cells.

However, interest has been greatest in vertebrate cells, and propagation of vertebrate cells in culture (tissue culture) has become a routine procedure. Examples of useful mammalian host cell lines are monkey kidney CV1 line transformed by SV40 (COS-7, ATCC CRL 1651); human embryonic kidney line (293 or 293 cells subcloned for growth in suspension culture, Graham et al., J. Gen Virol. 36:59 (1977)); baby hamster kidney cells (BHK, ATCC CCL 10); Chinese hamster ovary cells/-DHFR (CHO, Urlaub et al., Proc. Natl. Acad. Sci. USA 77:4216 (1980)); mouse sertoli cells (TM4, Mather, Biol. Reprod. 23:243-251 (1980)); monkey kidney cells (CV1 ATCC CCL 70); African green monkey kidney cells (VERO-76, ATCC CRL-1587); human cervical carcinoma cells (HELA, ATCC CCL 2); canine kidney cells (MDCK, ATCC CCL 34); buffalo rat liver cells (BRL 3A, ATCC CRL 1442); human lung cells (W138, ATCC CCL 75); human liver cells (Hep G2, HB 8065); mouse mammary tumor (MMT 060562, ATCC CCL51); TRI cells (Mather et al., Annals N.Y. Acad. Sci. 383:44-68 (1982)); MRC 5 cells; FS4 cells; and a human hepatoma line (Hep G2).

Host cells are transformed with the above-described expression or cloning vectors for antibody or polypeptide production and cultured in conventional nutrient media modified as appropriate for inducing promoters, selecting transformants, or amplifying the genes encoding the desired sequences.

3. Selection and Use of a Replicable Vector

The nucleic acid (e.g., cDNA or genomic DNA) encoding antibody or polypeptide may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The polypeptide may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the antibody- or polypeptide-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharonayces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2μ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the antibody- or polypeptide-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp1* gene present in the yeast plasmid YRp7 [Stinchcomb et al., Nature, 282:39 (1979); Kingsman et al., Gene, 7:141 (1979); Tschemper et al., Gene, 10:157 (1980)]. The *trp1* gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the antibody- or polypeptide-encoding nucleic acid sequence tq direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding antibody or polypeptide.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

Antibody or polypeptide transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the antibody or polypeptide by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, $\alpha$-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the antibody or polypeptide coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding antibody or polypeptide.

Still other methods, vectors, and host cells suitable for adaptation to the synthesis of antibody or polypeptide in recombinant vertebrate cell culture are described in Gething et al., Nature, 293:620-625 (1981); Mantei et al., Nature, 281:40-46 (1979); EP 117,060; and EP 117,058.

### 4. Culturing the Host Cells

The host cells used to produce the antibody or polypeptide of this invention may be cultured in a variety of media. Commercially available media such as Ham's F10 (Sigma), Minimal Essential Medium ((MEM), (Sigma), RPMI-1640 (Sigma), and Dulbecco's Modified Eagle's Medium ((DMEM), Sigma) are suitable for culturing the host cells. In addition, any of the media described in Ham et al., Meth. Enz. 58:44 (1979), Barnes et al., Anal. Biochem, 102:255 (1980), U.S. Pat. Nos. 4,767,704; 4,657,866; 4,927,762; 4,560,655; or 5,122,469; WO 90/03430; WO 87/00195; or U.S. Patent Re. 30,985 may be used as culture media for the host cells. Any of these media may be supplemented as necessary with hormones and/or other growth factors (such as insulin, transferrin, or epidermal growth factor), salts (such as sodium chloride, calcium, magnesium, and phosphate), buffers (such as HEPES), nucleotides (such as adenosine and thymidine), antibiotics (such as GENTAMYCIN™ drug), trace elements (defined as inorganic compounds usually present at final concentrations in the micromolar range), and glucose or an equivalent energy source. Any other necessary supplements may also be included at appropriate concentrations that would be known to those skilled in the art. The culture conditions, such as temperature, pH, and the like, are those previously used with the host cell selected for expression, and will be apparent to the ordinarily skilled artisan.

### 5. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or in situ hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence polypeptide or against a synthetic peptide based on the DNA sequence provided herein or against exogenous sequence fused to polypeptide DNA and encoding a specific antibody epitope.

6. Purification of Antibody and Polypeptide

Forms of antibody and polypeptide may be recovered from culture medium or from host cell lysates. If membrane-bound, it can be released from the membrane using a suitable detergent solution (e.g. Triton-X 100) or by enzymatic cleavage. Cells employed in expression of antibody and polypeptide can be disrupted by various physical or chemical means, such as freeze-thaw cycling, sonication, mechanical disruption, or cell lysing agents.

It may be desired to purify antibody and polypeptide from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the antibody and polypeptide. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular antibody or polypeptide produced.

When using recombinant techniques, the antibody can be produced intracellularly, in the periplasmic space, or directly secreted into the medium. If the antibody is produced intracellularly, as a first step, the particulate debris, either host cells or lysed fragments, are removed, for example, by centrifugation or ultrafiltration. Carter et al., Bio/Technology 10:163-167 (1992) describe a procedure for isolating antibodies which are secreted to the periplasmic space of *E. coli.* Briefly, cell paste is thawed in the presence of sodium acetate (pH 3.5), EDTA, and phenylmethylsulfonylfluoride (PMSF) over about 30 min. Cell debris can be removed by centrifugation. Where the antibody is secreted into the medium, supernatants from such expression systems are generally first concentrated using a commercially available protein concentration filter, for example, an Amicon or Millipore Pellicon ultrafiltration unit. A protease inhibitor such as PMSF may be included in any of the foregoing steps to inhibit proteolysis and antibiotics may be included to prevent the growth of adventitious contaminants.

The antibody composition prepared from the cells can be purified using, for example, hydroxylapatite chromatography, gel electrophoresis, dialysis, and affinity chromatography, with affinity chromatography being the preferred purification technique. The suitability of protein A as an affinity ligand depends on the species and isotype of any immunoglobulin Fc domain that is present in the antibody. Protein A can be used to purify antibodies that are based on human $\gamma$1, $\gamma$2 or $\gamma$4 heavy chains (Lindmark et al., J. Immunol. Meth. 62:1-13 (1983)). Protein G is recommended for all mouse isotypes and for human $\gamma$3 (Guss et al., EMBO J. 5:15671575 (1986)). The matrix to which the affinity ligand is attached is most often agarose, but other matrices are available. Mechanically stable matrices such as controlled pore glass or poly(styrenedivinyl)benzene allow for faster flow rates and shorter processing times than can be achieved with agarose. Where the antibody comprises a $C_H3$ domain, the Bakerbond ABX™resin (J. T. Baker, Phillipsburg, NJ) is useful for purification. Other techniques for protein purification such as fractionation on an ion-exchange column, ethanol precipitation, Reverse Phase HPLC, chromatography on silica, chromatography on heparin SEPHAROSE™ chromatography on an anion or cation exchange resin (such as a polyaspartic acid column), chromatofocusing, SDS-PAGE, and ammonium sulfate precipitation are also available depending on the antibody to be recovered.

Following any preliminary purification step(s), the mixture comprising the antibody of interest and contaminants may be subjected to low pH hydrophobic interaction chromatography using an elution buffer at a pH between about 2.5-4.5, preferably performed at low salt concentrations (e.g., from about 0-0.25M salt).

I. Pharmaceutical Formulations

Therapeutic formulations of the antibodies, binding oligopeptides, binding organic or inorganic small molecules and/or polypeptides used in accordance with the present invention are prepared for storage by mixing the antibody, polypeptide, oligopeptide or organic/inorganic small molecule having the desired degree of purity with optional pharmaceutically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as acetate, Tris, phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol;

and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; tonicifiers such as trehalose and sodium chloride; sugars such as sucrose, mannitol, trehalose or sorbitol; surfactant such as polysorbate; salt-forming counter-ions such as sodium; metal complexes (e.g., Zn-protein complexes); and/or non-ionic surfactants such as TWEEN®, PLURONICS® or polyethylene glycol (PEG). The formulation may comprise the antibody at a concentration of between 5-200 mg/ml, preferably between 10-100 mg/ml.

The formulations herein may also contain more than one active compound as necessary for the particular indication being treated, preferably those with complementary activities that do not adversely affect each other. For example, in addition to an antibody, binding oligopeptide, or binding organic or inorganic small molecule, it may be desirable to include in the one formulation, an additional antibody, e.g., a second antibody which binds a different epitope on the same polypeptide, or an antibody to some other target such as a growth factor that affects the growth of the particular cancer. Alternatively, or additionally, the composition may further comprise a chemotherapeutic agent, cytotoxic agent, cytokine, growth inhibitory agent, anti-hormonal agent, and/or cardioprotectant. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. Ed. (1980).

Sustained-release preparations may be prepared. Suitable examples of sustained-release preparations include semi-permeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT® (injectable microspheres composed of lactic acid-glycolic acid copolymer and leuprolide acetate), and poly-D-(-)-3-hydroxybutyric acid.

The formulations to be used for *in vivo* administration must be sterile. This is readily accomplished by filtration through sterile filtration membranes.

J. Treatment with Antibodies, Binding Oligopeptides and Binding Organic/Inorganic Small Molecules

To determine polypeptide (hepsin and/or uPA) expression in the cancer, various detection assays are available. In one embodiment, polypeptide overexpression may be analyzed by immunohistochemistry (IHC). Parrafin embedded tissue sections from a tumor biopsy may be subjected to the IHC assay and accorded a polypeptide staining intensity criteria as follows:

Score 0 - no staining is observed or membrane staining is observed in less than 10% of tumor cells.
Score 1+ - a faint/barely perceptible membrane staining is detected in more than 10% of the tumor cells. The cells are only stained in part of their membrane.
Score 2+ - a weak to moderate complete membrane staining is observed in more than 10% of the tumor cells.
Score 3+ - a moderate to strong complete membrane staining is observed in more than 10% of the tumor cells.

Those tumors with 0 or 1+ scores for polypeptide expression may be characterized as not overexpressing the polypeptide, whereas those tumors with 2+ or 3+ scores may be characterized as overexpressing the polypeptide.

Alternatively, or additionally, FISH assays such as the INFORM® (sold by Ventana, Arizona) or PATHVISION® (Vysis, Illinois) may be carried out on formalin-fixed, paraffin-embedded tumor tissue to determine the extent (if any) of polypeptide overexpression in the tumor.

Polypeptide overexpression or amplification may be evaluated using an *in vivo* detection assay, e.g., by administering a molecule (such as an antibody, oligopeptide or organic small molecule) which binds the molecule to be detected and is tagged with a detectable label (e.g., a radioactive isotope or a fluorescent label) and externally scanning the patient for localization of the label.

As described above, the antibodies, oligopeptides and organic/inorganic small molecules of the invention have various non-therapeutic applications. The antibodies, oligopeptides and organic/inorganic small molecules of the present invention can be useful for staging of polypeptide-expressing cancers (e.g., in radioimaging). The antibodies, oligopeptides and organic/inorganic small molecules are also useful for purification or immunoprecipitation of polypeptide from cells, for detection and quantitation of polypeptide *in vitro,* e.g., in an ELISA or a Western blot, to kill and eliminate polypeptide-expressing cells from a population of mixed cells as a step in the purification of other cells.

Currently, depending on the stage of the cancer, cancer treatment involves one or a combination of the following therapies: surgery to remove the cancerous tissue, radiation therapy, and chemotherapy. Antibody, oligopeptide or organic/inorganic small molecule therapy may be especially desirable in elderly patients who do not tolerate the toxicity and side effects of chemotherapy well and in metastatic disease where radiation therapy has limited usefulness. The tumor targeting antibodies, oligopeptides and organic/inorganic small molecules of the invention are useful to alleviate polypeptide-expressing cancers upon initial diagnosis of the disease or during relapse. For therapeutic applications, the antibody, oligopeptide or organic/inorganic small molecule can be used alone, or in combination therapy with, e.g., hormones, antiangiogcnics, or radiolabelled compounds, or with surgery, cryotherapy, and/or radiotherapy. Antibody, oligopeptide or organic/inorganic small molecule treatment can be administered in conjunction with other forms of conventional therapy, either consecutively with, pre- or post-conventional therapy. Chemotherapeutic drugs such as TAXOTERE® (docetaxel), TAXOL® (palictaxel), estramustine and mitoxantrone are used in treating cancer, in particular, in good risk patients. In the present method of the invention for treating or alleviating cancer, the cancer patient can be administered antibody, oligopeptide or organic/inorganic small molecule in conjunction with treatment with the one or more of the preceding chemotherapeutic agents. In particular, combination therapy with palictaxel and modified derivatives (see, e.g., EP0600517) is contemplated. The antibody, oligopeptide or organic/inorganic small molecule will be administered with a therapeutically effective dose of the chemotherapeutic agent. In another embodiment, the antibody, oligopeptide or organic/inorganic small molecule is administered in conjunction with chemotherapy to enhance the activity and efficacy of the chemotherapeutic agent, e.g., paclitaxel. The Physicians' Desk Reference (PDR) discloses dosages of these agents that have been used in treatment of various cancers. The dosing regimen and dosages of these aforementioned chemotherapeutic drugs that are therapeutically effective will depend on the particular cancer being treated, the extent of the disease and other factors familiar to the physician of skill in the art and can be determined by the physician.

In one particular embodiment, a conjugate comprising an antibody, oligopeptide or organic/inorganic small molecule conjugated with a cytotoxic agent is administered to the patient. Preferably, the immunoconjugate bound to the protein is internalized by the cell, resulting in increased therapeutic efficacy of the immunoconjugate in killing the cancer cell to which it binds. In a preferred embodiment, the cytotoxic agent targets or interferes with the nucleic acid in the cancer cell. Examples of such cytotoxic agents are described above and include maytansinoids, calicheamicins, ribonucleases and DNA endonucleases.

The antibodies, oligopeptides, organic/inorganic small molecules or toxin conjugates thereof are administered to a human patient, in accord with known methods, such as intravenous administration, e.g." as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody, oligopeptide or organic/inorganic small molecule is preferred.

Other therapeutic regimens may be combined with the administration of the antibody, oligopeptide or organic/inorganic small molecule. The combined administration includes co-administration, using separate formulations or a single pharmaceutical formulation, and consecutive administration in either order, wherein preferably there is a time period while both (or all) active agents simultaneously exert their biological activities. Preferably such combined therapy results in a synergistic therapeutic effect.

It may also be desirable to combine administration of the antibody or antibodies, oligopeptides or organic/inorganic small molecules, with administration of an antibody directed against another tumor antigen associated with the particular cancer.

In another embodiment, the therapeutic treatment methods of the present invention involves the combined administration of an antibody (or antibodies), oligopeptides or organic/inorganic small molecules and one or more chemotherapeutic agents or growth inhibitory agents, including co-administration of cocktails of different chemotherapeutic agents. Chemotherapeutic agents include estramustine phosphate, prednimustine, cisplatin, 5-fluorouracil, melphalan, cyclophosphamide, hydroxyurea and hydroxyureataxanes (such as paclitaxel and doxetaxel) and/or anthracycline antibiotics. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992).

The antibody, oligopeptide or organic/inorganic small molecule may be combined with an anti-hormonal compound; e.g., an anti-estrogen compound such as tamoxifen; an anti-progesterone such as onapristone (see, EP 616 812); or an anti-androgen such as flutamide, in dosages known for such molecules. Where the cancer to be treated is androgen independent cancer, the patient may previously have been subjected to anti-androgen therapy and, after the cancer becomes androgen independent, the antibody, oligopeptide or organic/inorganic small molecule (and optionally other agents as described herein) may be administered to the patient.

Sometimes, it may be beneficial to also co-administer a cardioprotectant (to prevent or reduce myocardial dysfunction associated with the therapy) or one or more cytokines to the patient. In addition to the above therapeutic regimes, the patient may be subjected to surgical removal of cancer cells and/or radiation therapy, before, simultaneously with, or post antibody, oligopeptide or organic/inorganic small molecule therapy. Suitable dosages for any of the above co-administered agents are those presently used and may be lowered due to the combined action (synergy) of the agent and antibody, oligopeptide or organic/inorganic small molecule.

For the prevention or treatment of disease, the dosage and mode of administration will be chosen by the physician according to known criteria. The appropriate dosage of antibody, oligopeptide or organic/inorganic small molecule will depend on the type of disease to be treated, as defined above, the severity and course of the disease, whether the antibody, oligopeptide or organic/inorganic small molecule is administered for preventive or therapeutic purposes, previous therapy, the patient's clinical history and response to the antibody, oligopeptide or organic/inorganic small molecule, and the discretion of the attending physician. The antibody, oligopeptide or organic/inorganic small molecule is suitably administered to the patient at one time or over a series of treatments. Preferably, the antibody, oligopeptide or organic/inorganic small molecule is administered by intravenous infusion or by subcutaneous injections. Depending on the type and severity of the disease, about 1 μg/kg to about 50 mg/kg body weight (e.g., about 0.1-15mg/kg/dose) of antibody can be an initial candidate dosage for administration to the patient, whether, for example, by one or more separate administrations, or by continuous infusion. A dosing regimen can comprise administering an initial loading dose of about 4 mg/kg, followed by a weekly maintenance dose of about 2 mg/kg of the antibody. However, other dosage regimens may be useful. A typical daily dosage might range from about 1 μg/kg to 100 mg/kg or more, depending on the factors mentioned above. For repeated administrations over several days or longer, depending on the condition, the treatment is sustained until a desired suppression of disease symptoms occurs. The progress of this therapy can be readily monitored by conventional methods and assays and based on criteria known to the physician or other persons of skill in the art.

Aside from administration of the antibody protein to the patient, the present application contemplates administration of the antibody by gene therapy. Such administration of nucleic acid encoding the antibody is encompassed by the expression "administering a therapeutically effective amount of an antibody". See, for example, WO96/07321 published March 14, 1996 concerning the use of gene therapy to generate intracellular antibodies.

There are two major approaches to getting the nucleic acid (optionally contained in a vector) into the patient's cells; *in vivo* and *ex vivo.* For *in vivo* delivery the nucleic acid is injected directly into the patient, usually at the site where the antibody is required. For *ex vivo* treatment, the patient's cells are removed, the nucleic acid is introduced into these isolated cells and the modified cells are administered to the patient either directly or, for example, encapsulated within porous membranes which are implanted into the patient (see, e.g., U.S. Patent Nos. 4,892,538 and 5,283,187). There are a variety of techniques available for introducing nucleic acids into viable cells. The techniques vary depending upon whether the nucleic acid is transferred into cultured cells *in vitro,* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. A commonly used vector for *ex vivo* delivery of the gene is a retroviral vector.

The currently preferred *in vivo* nucleic acid transfer techniques include transfection with viral vectors (such as adenovirus, Herpes simplex I virus, or adeno-associated virus) and lipid-based systems (useful lipids for lipid-mediated transfer of the gene are DOTMA, DOPE and DC-Chol, for example). For review of the currently known gene marking and gene therapy protocols see Anderson et al., Science 256:808-813 (1992). See also WO 93/25673 and the references cited therein.

The antibodies of the invention can be in the different forms encompassed by the definition of "antibody" herein. Thus, the antibodies include full length or intact antibody, antibody fragments, native sequence antibody or amino acid variants, humanized, chimeric or fusion antibodies, immunoconjugates, and functional fragments thereof. In fusion antibodies an antibody sequence is fused to a heterologous polypeptide sequence. The antibodies can be modified in the Fc region to provide desired effector functions. As discussed in more detail in the sections herein, with the appropriate Fc regions, the naked antibody bound on the cell surface can induce cytotoxicity, e.g., via antibody-dependent cellular cytotoxicity (ADCC) or by recruiting complement in complement dependent cytotoxicity, or some other mechanism. Alternatively, where it is desirable to eliminate or reduce effector function, so as to minimize side effects or therapeutic complications, certain other Fc regions may be used.

In one embodiment, the antibody competes for binding or bind substantially to, the same epitope as the antibodies of the invention. Antibodies having the biological characteristics of the present antibodies of the invention are also contemplated, specifically including the *in vivo* tumor targeting and any cell proliferation inhibition or cytotoxic characteristics.

Methods of producing the above antibodies are described in detail herein.

The present antibodies, oligopeptides and organic/inorganic small molecules are useful for treating a hepsin and/or uPA-expressing cancer, or alleviating one or more symptoms of the cancer in a mammal. Methods of the invention encompass usage of antagonists in the treatment and/or alleviation of symptoms of metastatic tumors associated with these cancers. The antibody, oligopeptide or organic/inorganic small molecule antagonist is able to bind to at least a portion of the cancer cells that express the polypeptide(s) (hepsin and/or uPA) in the mammal. In one embodiment, the antibody, oligopeptide or organic/inorganic small molecule is effective to cause destruction or killing of polypeptide-expressing and/or -responsive tumor cells, or inhibit the growth and/or invasiveness of such tumor cells, *in vitro* or *in vivo,* upon binding to the polypeptide. Such an antibody includes a naked antibody (not conjugated to any agent). Naked antibodies that have cytotoxic or other inhibition properties can be further harnessed with a cytotoxic agent to render them even more potent in tumor cell destruction. Cytotoxic properties can be conferred to an antibody by, e.g., conjugating the antibody with a cytotoxic agent, to form an immunoconjugate as described herein. In some embodiments, the cytotoxic agent or a growth inhibitory agent is a small molecule. In some embodiments, toxins such as calicheamicin or a maytansinoid and analogs or derivatives thereof, are used.

The invention provides a composition comprising an antibody, oligopeptide or organic/inorganic small molecule of the invention, and a carrier. For the purposes of treating cancer, compositions can be administered to the patient in need of such treatment, wherein the composition can comprise one or more antibodies present as an immunoconjugate or as the naked antibody. In a further embodiment, the compositions can comprise these antibodies, oligopeptides or organic/inorganic small molecules in combination with other therapeutic agents such as cytotoxic or growth inhibitory agents, including chemotherapeutic agents. The invention also provides formulations comprising an antibody, oligopeptide or organic/inorganic small molecule of the invention, and a carrier. In one embodiment, the formulation is a therapeutic formulation comprising a pharmaceutically acceptable carrier.

Another aspect of the invention is isolated nucleic acids encoding the antibodies. Nucleic acids encoding both the H and L chains and especially the hypervariable region residues, chains which encode the native sequence antibody as well as variants, modifications and humanized versions of the antibody, are encompassed.

The invention also provides methods useful for treating a cancer or alleviating one or more symptoms of the cancer in a mammal, comprising administering a therapeutically effective amount of an antibody, oligopeptide or organic/inorganic small molecule to the mammal. The antibody, oligopeptide or organic/inorganic small molecule therapeutic compositions can be administered short term (acute) or chronic, or intermittent as directed by physician. Also provided are methods of inhibiting the growth of, and killing a polypeptide (hepsin and/or uPA)-expressing and/or -responsive cell.

The invention also provides kits and articles of manufacture comprising at least one antibody, oligopeptide or organic/inorganic small molecule. Kits containing antibodies, oligopeptides or organic/inorganic small molecules find use, e.g., for cell killing assays, for inhibiting tumor cell invasion, and for purification or immunoprecipitation of polypeptide from cells. For example, for isolation and purification of a polypeptide, the kit can contain an antibody, oligopeptide or organic/inorganic small molecule coupled to beads (e.g., sepharose beads). Kits can be provided which contain the antibodies, oligopeptides or organic/inorganic small molecules for detection and quantitation of a polypeptide *in vitro,* e.g., in an ELISA or a Western blot. Such antibody, oligopeptide or organic/inorganic small molecule useful for detection may be provided with a label such as a fluorescent or radiolabel.

K. Articles of Manufacture and Kits

Another embodiment of the invention is an article of manufacture containing materials useful for the treatment of a polypeptide (hepsin and/or uPA) expressing cancer, such as prostate and ovarian cancer. The article of manufacture comprises a container and a label or package insert on or associated with the container. Suitable containers include, for example, bottles, vials, syringes, etc. The containers may be formed from a variety of materials such as glass or plastic. The container holds a composition which is effective for treating the cancer condition and may have a sterile access port (for example the container may be an intravenous solution bag or a vial having a stopper pierceable by a hypodermic injection needle). At least one active agent in the composition is an antibody, oligopeptide or organic/inorganic small molecule of the invention. The label or package insert indicates that the composition is used for treating cancer. The label or package insert will further comprise instructions for administering the antibody, oligopeptide or organic/inorganic small molecule composition to the cancer patient. Additionally, the article of manufacture may further comprise a second container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Kits are also provided that are useful for various purposes, e.g., for polypeptide-expressing or cell killing assays, for purification or immunoprecipitation of a polypeptide from cells. For isolation and purification of a polypeptide, the kit can contain an antibody, oligopeptide or organic/inorganic small molecule coupled to beads (e.g., sepharose beads). Kits can be provided which contain the antibodies, oligopeptides or organic/inorganic small molecules for detection and quantitation of a polypeptide *in vitro,* e.g., in an ELISA or a Western blot. As with the article of manufacture, the kit comprises a container and a label or package insert on or associated with the container. The container holds a composition comprising at least one antibody, oligopeptide or organic/inorganic small molecule of the invention. Additional containers may be included that contain, e.g., diluents and buffers, control antibodies. The label or package insert may provide a description of the composition as well as instructions for the intended in vitro or detection use.

L. Polypeptides and Polypeptide-Eneoding Nucleic Acids - Specific forms and applications

Nucleotide sequences (or their complement) encoding polypeptides of the invention have various applications in the art of molecular biology, as well as uses for therapy, etc. Polypeptide-encoding nucleic acid will also be useful for the preparation of polypeptides by the recombinant techniques described herein, wherein those polypeptides may find use, for example, in the preparation of antibodies as described herein.

A full-length native sequence polypeptide gene, or portions thereof, may be used as hybridization probes for a cDNA library to isolate other cDNAs (for instance, those encoding naturally-occurring variants of a polypeptide or a polypeptide from other species) which have a desired sequence identity to a native polypeptide sequence disclosed herein. Optionally, the length of the probes will be about 20 to about 50 bases. The hybridization probes may be derived from at least partially novel regions of the full length native nucleotide sequence wherein those regions may be determined without undue experimentation or from genomic sequences including promoters, enhancer elements and introns of native sequence polypeptide. By way of example, a screening method will comprise isolating the coding region of the polypeptide gene using the known DNA sequence to synthesize a selected probe of about 40 bases. Hybridization probes may be labeled by a variety of labels, including radionucleotides such as $^{32}$P or $^{35}$S, or enzymatic labels such as alkaline phosphatase coupled to the probe via avidin/biotin coupling systems. Labeled probes having a sequence complementary to that of the polypeptide gene of the present invention can be used to screen libraries of human cDNA, genomic DNA or mRNA to determine which members of such libraries the probe hybridizes to. Hybridization techniques are described in further detail in the Examples below. Any EST sequences disclosed in the present application may similarly be employed as probes, using the methods disclosed herein.

Other useful fragments of the polypeptide-encoding nucleic acids include antisense or sense oligonucleotides comprising a singe-stranded nucleic acid sequence (either RNA or DNA) capable of binding to target a polypeptide mRNA (sense) or a polypeptide DNA (antisense) sequence. Antisense or sense oligonucleotides, according to the present invention, comprise a fragment of the coding region of a DNA encoding hepsin, pro-uPA or binding fragments as described herein. Such a fragment generally comprises at least about 14 nucleotides, preferably from about 14 to 30 nucleotides. The ability to derive an antisense or a sense oligonucleotide, based upon a cDNA sequence encoding a given protein is described in, for example, Stein and Cohen (Cancer Res. 48:2659, 1988) and van der Krol et al. (BioTechniques 6:958, 1988).

Binding of antisense or sense oligonucleotides to target nucleic acid sequences results in the formation of duplexes that block transcription or translation of the target sequence by one of several means, including enhanced degradation of the duplexes, premature termination of transcription or translation, or by other means. Such methods are encompassed by the present invention. The antisense oligonucleotides thus may be used to block expression of a protein, wherein the protein may play a role in the induction of cancer in mammals. Antisense or sense oligonucleotides further comprise oligonucleotides having modified sugar-phosphodiester backbones (or other sugar linkages, such as those described in WO 91/06629) and wherein such sugar linkages are resistant to endogenous nucleases. Such oligonucleotides with resistant sugar linkages are stable *in vivo* (i.e., capable of resisting enzymatic degradation) but retain sequence specificity to be able to bind to target nucleotide sequences.

Preferred intragenic sites for antisense binding include the region incorporating the translation initiation/start codon (5'-AUG / 5'-ATG) or termination/stop codon (5'-UAA, 5'-UAG and 5-UGA / 5'-TAA, 5'-TAG and 5'-TGA) of the open reading frame (ORF) of the gene. These regions refer to a portion of the mRNA or gene that encompasses from about 25 to about 50 contiguous nucleotides in either direction (i.e., 5' or 3') from a translation initiation or termination codon. Other preferred regions for antisense binding include: introns; exons; intron-exon junctions; the open reading frame (ORF) or "coding region," which is the region between the translation initiation codon and the translation termination codon; the 5' cap of an mRNA which comprises an N7-methylated guanosine residue joined to the 5'-most residue of the mRNA via a 5'-5' triphosphate linkage and includes 5' cap structure itself as well as the first 50 nucleotides adjacent to the cap; the 5' untranslated region (5'UTR), the portion of an mRNA in the 5' direction from the translation initiation codon, and thus including nucleotides between the 5' cap site and the translation initiation codon of an mRNA or corresponding nucleotides on the gene; and the 3' untranslated region (3'UTR), the portion of an mRNA in the 3' direction from the translation termination codon, and thus including nucleotides between the translation termination codon and 3' end of an mRNA or corresponding nucleotides on the gene.

Specific examples of preferred antisense compounds useful for inhibiting expression of a polypeptide include oligonucleotides containing modified backbones or non-natural internucleoside linkages. Oligonucleotides having modified backbones include those that retain a phosphorus atom in the backbone and those that do not have a phosphorus atom in the backbone. For the purposes of this specification, and as sometimes referenced in the art, modified oligonucleotides that do not have a phosphorus atom in their internucleoside backbone can also be considered to be oligonucleosides. Preferred modified oligonucleotide backbones include, for example, phosphorothioates, chiral phosphorothioates, phosphorodithioates, phosphotriesters, aminoalkylphosphotri-esters, methyl and other alkyl phosphonates including 3'-alkylene phosphonates, 5'-alkylene phosphonates and chiral phosphonates, phosphinates, phosphoramidates including 3'-amino phosphoramidate and aminoalkylphosphoramidates, thionophosphoramidates, thionoalkylphosphonates, thionoalkylphosphotriesters, selenophosphates and borano-phosphates having normal 3'-5' linkages, 2'-5' linked analogs of these, and those having inverted polarity wherein one or more internucleotide linkages is a 3' to 3', 5' to 5' or 2' to 2' linkage. Preferred oligonucleotides having inverted polarity comprise a single 3' to 3' linkage at the 3'-most internucleotide linkage i.e. a single inverted nucleoside residue which may be abasic (the nucleobase is missing or has a hydroxyl group in place thereof). Various salts, mixed salts and free acid forms are also included. Representative United States patents that teach the preparation of phosphorus-containing linkages include, but are not limited to, U.S. Pat. Nos.: 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; 5,194,599; 5,565,555; 5,527,899; 5,721,218; 5,672,697 and 5,625,050.

Preferred modified oligonucleotide backbones that do not include a phosphorus atom therein have backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; riboacetyl backbones; alkene containing backbones; sulfamate backbones; methyleneimino and methylenehydrazino backbones; sulfonate and sulfonamide backbones; amide backbones; and others having mixed N, O, S, and CH.sub.2 component parts. Representative United States patents that teach the preparation of such oligonucleosides include, but are not limited to,. U.S. Pat. Nos.: 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; 5,792,608; 5,646,269 and 5,677,439.

In other preferred antisense oligonucleotides, both the sugar and the internucleoside linkage, i.e., the backbone, of the nucleotide units are replaced with novel groups. The base units are maintained for hybridization with an appropriate nucleic acid target compound. One such oligomeric compound, an oligonucleotide mimetic that has been shown to have excellent hybridization properties, is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone. Representative United States patents that teach the preparation of PNA compounds include, but are not limited to, U.S. Pat. Nos.: 5,539,082; 5,714,331; and 5,719,262. Further teaching of PNA compounds can be found in Nielsen et al., Science, 1991, 254, 1497-1500.

Preferred antisense oligonucleotides incorporate phosphorothioate backbones and/or heteroatom backbones, and in particular -$CH_2$-NH-O-$CH_2$-, -$CH_2$-N($CH_3$)-O-$CH_2$-[known as a methylene (methylimino) or MMI backbone], -$CH_2$-O-N($CH_3$)-$CH_2$-, -$CH_2$-N($CH_3$)-N($CH_3$)-$CH_2$- and -O-N($CH_3$)-$CH_2$-$CH_2$- [wherein the native phosphodiester backbone is represented as -O-P-O-$CH_2$-] described in the above referenced U.S. Pat. No. 5,489,677, and the amide backbones of the above referenced U.S. Pat. No. 5,602,240. Also preferred are antisense oligonucleotides having morpholino backbone structures of the above-referenced U.S. Pat. No. 5,034,506.

Modified oligonucleotides may also contain one or more substituted sugar moieties. Preferred oligonucleotides comprise one of the following at the 2' position: OH; F; O-alkyl, S-alkyl, or N-alkyl; O-alkenyl, S-alkeynyl, or N-alkenyl; O-alkynyl, S-alkynyl or N-alkynyl; or O-alkyl-O-alkyl, wherein the alkyl, alkenyl and alkynyl may be substituted or unsubstituted $C_1$ to $C_{10}$ alkyl or $C_2$ to $C_{10}$ alkenyl and alkynyl. Particularly preferred are $O[(CH_2)_nO]_mCH_3$, $O(CH_2)_nOCH_3$, $O(CH_2)_nNH_2$, $O(CH_2)_nCH_3$, $O(CH_2)_nONH_2$, and $O(CH_2)_nON[(CH_2)_nCH_3)]_2$, where n and m are from 1 to about 10. Other preferred antisense oligonucleotides comprise one of the following at the 2' position: $C_1$ to $C_{10}$ lower alkyl, substituted lower alkyl, alkenyl, alkynyl, alkaryl, aralkyl, O-alkaryl or O-aralkyl, SH, $SCH_3$, OCN, Cl, Br, CN, $CF_3$, $OCF_3$, $SOCH_3$, $SO_2 CH_3$, $ONO_2$, $NO_2$, $N_3$, $NH_2$, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. A preferred modification includes 2'-methoxyethoxy (2'-O-$CH_2CH_2OCH_3$, also known as 2'-O-(2-methoxyethyl) or 2'-MOE) (Martin et al., Helv. Chim. Acta, 1995, 78, 486-504) i.e., an alkoxyalkoxy group. A further preferred modification includes 2'-dimethylaminooxyethoxy, i.e., a $O(CH_2)_2ON(CH_3)_2$ group, also known as 2'-DMAOE, as described in examples hereinbelow, and 2'-dimethylaminoethoxyethoxy (also known in the art as 2'-O-dimethylaminoethoxyethyl or 2'-DMAEOE), i.e., 2'-O-$CH_2$-O-$CH_2$-N($CH_2$).

A further prefered modification includes Locked Nucleic Acids (LNAs) in which the 2'-hydroxyl group is linked to the 3' or 4' carbon atom of the sugar ring thereby forming a bicyclic sugar moiety. The linkage is preferably a methelyne (-$CH_2$-)$_n$ group bridging the 2' oxygen atom and the 4' carbon atom wherein n is 1 or 2. LNAs and preparation thereof are described in WO 98/39352 and WO 99/14226.

Other preferred modifications include 2'-methoxy (2-O-$CH_3$), 2'-aminopropoxy (2'-$OCH_2CH_2CH_2 NH_2$), 2'-allyl

(2'-CH$_2$-CH=CH$_2$), 2'-O-allyl (2'-O-CH$_2$-CH=CH$_2$) and 2'-fluoro (2'-F). The 2'-modification may be in the arabino (up) position or ribo (down) position. A preferred 2'-arabino modification is 2'-F. Similar modifications may also be made at other positions on the oligonucleotide, particularly the 3' position of the sugar on the 3' terminal nucleotide or in 2'-5' linked oligonucleotides and the 5' position of 5' terminal nucleotide. Oligonucleotides may also have sugar mimetics such as cyclobutyl moieties in place of the pentofuranosyl sugar. Representative United States patents that teach the preparation of such modified sugar structures include, but are not limited to, U.S. Pat. Nos.: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; and 5,700,920.

Oligonucleotides may also include nucleobase (often referred to in the art simply as "base") modifications or substitutions. As used herein, "unmodified" or "natural" nucleobases include the purine bases adenine (A) and guanine (G), and the pyrimidine bases thymine (T), cytosine (C) and uracil (U). Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH$_3$ or -CH$_2$-C≡CH) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine. Further modified nucleobases include tricyclic pyrimidines such as phenoxazine cytidine(1H-pyrimido[5,4-b][1,4]benzoxazin-2(3H)-one), phenothiazine cytidine (1H-pyrimido[5,4-b][1,4]benzothiazin-2(3H)-one), G-clamps such as a substituted phenoxazine cytidine (e.g. 9-(2-aminoethoxy)-H-pyrimido[5,4-b][1,4]benzoxazin-2(3H)-one), carbazole cytidine (2H-pyrimido[4,5-b]indol-2-one), pyridoindole cytidine (H-pyrido[3',2':4,5]pyrrolo[2,3-d]pyrimidin-2-one). Modified nucleobases may also include those in which the purine or pyrimidine base is replaced with other heterocycles, for example 7-deaza-adenine, 7-deazaguanosine, 2-aminopyridine and 2-pyridone. Further nucleobases include those disclosed in U.S. Pat. No. 3,687,808, those disclosed in The Concise Encyclopedia Of Polymer Science And Engineering, pages 858-859, Kroschwitz, J. I., ed. John Wiley & Sons, 1990, and those disclosed by Englisch et al., Angewandte Chemie, International Edition, 1991, 30, 613. Certain of these nucleobases are particularly useful for increasing the binding affinity of the oligomeric compounds of the invention. These include 5-substituted pyrimidines, 6-azapyrimidines and N-2, N-6 and O-6 substituted purines, including 2-aminopropyladenine, 5-propynyluracil and 5-propynylcytosine. 5-methylcytosine substitutions have been shown to increase nucleic acid duplex stability by 0.6-1.2.degree. C. (Sanghvi et al, Antisense Research and Applications, CRC Press, Boca Raton, 1993, pp. 276-278) and are preferred base substitutions, even more particularly when combined with 2'-O-methoxyethyl sugar modifications. Representative United States patents that teach the preparation of modified nucleobases include, but are not limited to: U.S. Pat. No. 3,687,808, as well as U.S. Pat. Nos.: 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; 5,645,985; 5,830,653; 5,763,588; 6,005,096; 5,681,941 and 5,750,692.

Another modification of antisense oligonucleotides chemically linking to the oligonucleotide one or more moieties or conjugates which enhance the activity, cellular distribution or cellular uptake of the oligonucleotide. The compounds of the invention can include conjugate groups covalently bound to functional groups such as primary or secondary hydroxyl groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugates groups include cholesterols, lipids, cation lipids, phospholipids, cationic phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve oligomer uptake, enhance oligomer resistance to degradation, and/or strengthen sequence-specific hybridization with RNA. Groups that enhance the pharmacokinetic properties, in the context of this invention, include groups that improve oligomer uptake, distribution, metabolism or excretion. Conjugate moieties include but are not limited to lipid moieties such as a cholesterol moiety (Letsinger et al., Proc. Natl. Acad. Sci. USA, 1989, 86, 6553-6556), cholic acid (Manoharan et al., Bioorg. Med. Chem. Let., 1994, 4, 1053-1060), a thioether, e.g., hexyl-S-tritylthiol (Manoharan et al., Ann. N.Y. Acad. Sci., 1992, 660, 306-309; Manoharan et al., Bioorg. Med. Chem. Let., 1993, 3, 2765-2770), a thiocholesterol (Oberhauser et al., Nucl. Acids Res., 1992, 20, 533-538), an aliphatic chain, e.g., dodecandiol or undecyl residues (Saison-Behmoaras et al., EMBO J., 199.1, 10, 1111-1118; Kabanov et al., FEBS Lett., 1990, 259, 327-330; Svinarchuk et al., Biochimie, 1993, 75, 49-54), a phospholipid, e.g., di-hexadecyl-rac-glycerol or triethylammonium 1,2-di-O-hexadecyl-rac-glycero-3-H-phosphonate (Manoharan et al., Tetrahedron Lett., 1995, 36, 3651-3654; Shea et al., Nucl. Acids Res., 1990, 18, 3777-3783), a polyamine or a polyethylene glycol chain (Manoharan et al., Nucleosides & Nucleotides, 1995, 14, 969-973), or adamantane acetic acid (Manoharan et al., Tetrahedron Lett., 1995, 36, 3651-3654), a palmityl moiety (Mishra et al., Biochim. Biophys. Acta, 1995, 1264, 229-237), or an octadecylamine or hexylamino-carbonyl-oxycholesterol moiety. Oligonucleotides of the invention may also be conjugated to active drug substances, for example, aspirin, warfarin, phenylbutazone, ibuprofen, suprofen, fenbufen, ketoprofen, (S)-(+)-pranoprofen, carprofen, dansylsarcosine, 2,3,5-triiodobenzoic acid, flufenamic acid, folinic acid, a benzothiadiazide, chlorothiazide, a diazepine, indomethicin, a barbiturate, a cephalosporin, a sulfa drug, an antidiabetic, an antibacterial or an antibiotic. Oligonucleotide-drug conjugates and their preparation are described in U.S. patent application Ser. No. 09/334,130 (filed Jun. 15, 1999) and United States patents Nos.: 4,828,979; 4,948,882; 5,218,105; 5,525,465; 5,541,313; 5,545,730; 5,552,538; 5,578,717, 5,580,731; 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241; 5,391,723; 5,416,203; 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941.

It is not necessary for all positions in a given compound to be uniformly modified, and in fact more than one of the aforementioned modifications may be incorporated in a single compound or even at a single nucleoside within an oligonucleotide. The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Preferred chimeric antisense oligonucleotides incorporate at least one 2' modified sugar (preferably 2'-O-$(CH_2)_2$-O-$CH_3$) at the 3' terminal to confer nuclease resistance and a region with at least 4 contiguous 2'-H sugars to confer RNase H activity. Such compounds have also been referred to in the art as hybrids or gapmers. Preferred gapmers have a region of 2' modified sugars (preferably 2'-O-$(CH_2)_2$-O-$CH_3$) at the 3'-terminal and at the 5' terminal separated by at least one region having at least 4 contiguous 2'-H sugars and preferably incorporate phosphorothioate backbone linkages. Representative United States patents that teach the preparation of such hybrid structures include, but are not limited to, U.S. Pat. Nos. 5,013,830; 5,149,797; 5,220,007; 5,256,775; 5,366,878; 5,403,711; 5,491,133; 5,565,350; 5,623,065; 5,652,355; 5,652,356; and 5,700,922.

The antisense compounds used in accordance with this invention may be conveniently and routinely made through the well-known technique of solid phase synthesis. Equipment for such synthesis is sold by several vendors including, for example, Applied Biosystems (Foster City, Calif.). Any other means for such synthesis known in the art may additionally or alternatively be employed. It is well known to use similar techniques to prepare oligonucleotides such as the phosphorothioates and alkylated derivatives. The compounds of the invention may also be admixed, encapsulated, conjugated or otherwise associated with other molecules, molecule structures or mixtures of compounds, as for example, liposomes, receptor targeted molecules, oral, rectal, topical or other formulations, for assisting in uptake, distribution and/or absorption. Representative United States patents that teach the preparation of such uptake, distribution and/or absorption assisting formulations include, but are not limited to, U.S. Pat. Nos. 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

Other examples of sense or antisense oligonucleotides include those oligonucleotides which are covalently linked to organic moieties, such as those described in WO 90/10048, and other moieties that increases affinity of the oligonucleotide for a target nucleic acid sequence, such as poly-(L-lysine). Further still, intercalating agents, such as ellipticine, and alkylating agents or metal complexes may be attached to sense or antisense oligonucleotides to modify binding specificities of the antisense or sense oligonucleotide for the target nucleotide sequence.

Antisense or sense oligonucleotides may be introduced into a cell containing the target nucleic acid sequence by any gene transfer method, including, for example, $CaPO_4$-mediated DNA transfection, electroporation, or by using gene transfer vectors such as Epstein-Barr virus. In a preferred procedure, an antisense or sense oligonucleotide is inserted into a suitable retroviral vector. A cell containing the target nucleic acid sequence is contacted with the recombinant retroviral vector, either *in vivo* or *ex vivo*. Suitable retroviral vectors include, but are not limited to, those derived from the murine retrovirus M-MuLV, N2 (a retrovirus derived from M-MuLV), or the double copy vectors designated DCT5A, DCT5B and DCT5C (see WO 90/13641).

Sense or antisense oligonucleotides also may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell.

Alternatively, a sense or an antisense oligonucleotide may be introduced into a cell containing the target nucleic acid sequence by formation of an oligonucleotide-lipid complex, as described in WO 90/10448. The sense or antisense oligonucleotide-lipid complex is preferably dissociated within the cell by an endogenous lipase.

Antisense or sense RNA or DNA molecules are generally at least about 5 nucleotides in length, alternatively at least about 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 210, 220, 230, 240, 250, 260, 270, 280, 290, 300, 310, 320, 330, 340, 350, 360, 370, 380, 390, 400; 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 730, 740, 750, 760, 770, 780, 790, 800, 810, 820, 830, 840, 850, 860, 870, 880, 890, 900, 910, 920, 930, 940, 950, 960, 970, 980, 990, or 1000 nucleotides in length, wherein in this context the term "about" means the referenced nucleotide sequence length plus or minus 10% of that referenced length.

The probes may also be employed in PCR techniques to generate a pool of sequences for identification of closely related polypeptide coding sequences.

Nucleotide sequences encoding a polypeptide can also be used to construct hybridization probes for mapping the gene which encodes that polypeptide and for the genetic analysis of individuals with genetic disorders. The nucleotide sequences provided herein may be mapped to a chromosome and specific regions of a chromosome using known techniques, such as *in situ* hybridization, linkage analysis against known chromosomal markers, and hybridization screening with libraries.

The polypeptide can be used in assays to identify other proteins or molecules involved in a binding interaction with the polypeptide. By such methods, inhibitors of the receptor/ligand binding interaction can be identified. Proteins involved in such binding interactions can also be used to screen for peptide or small molecule inhibitors of the binding interaction. Screening assays can be designed to find lead compounds that mimic the biological activity of a native polypeptide or a receptor for the polypeptide. Such screening assays will include assays amenable to high-throughput screening of chemical libraries, making them particularly suitable for identifying small molecule drug candidates. Small molecules contemplated include synthetic organic or inorganic compounds. The assays can be performed in a variety of formats, including protein-protein binding assays, biochemical screening assays, immunoassays and cell based assays, which are well characterized in the art.

Nucleic acids which encode a polypeptide or its modified forms can also be used to generate either transgenic animals or "knock out" animals which, in turn, are useful in the development and screening of therapeutically useful reagents. A transgenic animal (e.g., a mouse or rat) is an animal having cells that contain a transgene, which transgene was introduced into the animal or an ancestor of the animal at a prenatal, e.g., an embryonic stage. A transgene is a DNA which is integrated into the genome of a cell from which a transgenic animal develops. In one embodiment, cDNA encoding a polypeptide can be used to clone genomic DNA encoding the polypeptide in accordance with established techniques and the genomic sequences used to generate transgenic animals that contain cells which express DNA encoding the polypeptide. Methods for generating transgenic animals, particularly animals such as mice or rats, have become,conventional in the art and are described, for example, in U.S. Patent Nos. 4,736,866 and 4,870,009. Typically, particular cells would be targeted for polypeptide transgene incorporation with tissue-specific enhancers. Transgenic animals that include a copy of a transgene encoding a polypeptide introduced into the germ line of the animal at an embryonic stage can be used to examine the effect of increased expression of DNA encoding a polypeptide. Such animals can be used as tester animals for reagents thought to confer protection from, for example, pathological conditions associated with its overexpression. In accordance with this facet of the invention, an animal is treated with the reagent and a reduced incidence of the pathological condition, compared to untreated animals bearing the transgene, would indicate a potential therapeutic intervention for the pathological condition.

Alternatively, non-human homologues of a polypeptide can be used to construct a a gene "knock out" animal which has a defective or altered gene encoding the polypeptide as a result of homologous recombination between the endogenous gene encoding the polypeptide and altered genomic DNA encoding the polypeptide introduced into an embryonic stem cell of the animal. For example, cDNA encoding the polypeptide can be used to clone genomic DNA encoding the polypeptide in accordance with established techniques. A portion of the genomic DNA encoding the polypeptide can be deleted or replaced with another gene, such as a gene encoding a selectable marker which can be used to monitor integration. Typically, several kilobases of unaltered flanking DNA (both at the 5' and 3' ends) are included in the vector [see e.g., Thomas and Capecchi, Cell, 51:503 (1987) for a description of homologous recombination vectors]. The vector is introduced into an embryonic stem cell line (e.g., by electroporation) and cells in which the introduced DNA has homologously recombined with the endogenous DNA are selected [see e.g., Li et al., Cell, 69:915 (1992)]. The selected cells are then injected into a blastocyst of an animal (e.g., a mouse or rat) to form aggregation chimeras [see e.g., Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

Nucleic acid encoding the polypeptides may also be used in gene therapy. In gene therapy applications, genes are introduced into cells in order to achieve *in vivo* synthesis of a therapeutically effective genetic product, for example for replacement of a defective gene. "Gene therapy" includes both conventional gene therapy where a lasting effect is achieved by a single treatment, and the administration of gene therapeutic agents, which involves the one time or repeated administration of a therapeutically effective DNA or mRNA. Antisense RNAs and DNAs can be used as therapeutic agents for blocking the expression of certain genes *in vivo.* It has already been shown that short antisense oligonucleotides can be imported into cells where they act as inhibitors, despite their low intracellular concentrations caused by their restricted uptake by the cell membrane. *(*Zamecnik et al., Proc. Natl. Acad. Sci. USA 83:4143-4146 [1986]). The oligonucleotides can be modified to enhance their uptake, e.g. by substituting their negatively charged phosphodiester groups by uncharged groups.

There are a variety of techniques available for introducing nucleic acids into viable cells. The techniques vary depending upon whether the nucleic acid is transferred into cultured cells *in vitro*, or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred *in vivo* gene transfer techniques include transfection with viral (typically retroviral) vectors and viral coat protein-liposome mediated transfection (Dzau et al., Trends in Biotechnology 11, 205-210 [1993]). In some situations it is desirable to provide the nucleic acid source with an agent that targets the target cells, such as an antibody specific for a cell surface membrane protein or the target cell, a ligand for a receptor on the target cell, etc. Where liposomes are employed, proteins which bind to a cell surface membrane protein associated with endocytosis may be used for targeting and/or to facilitate uptake, e.g. capsid proteins or fragments thereof tropic for a particular cell type, antibodies for proteins which undergo internalization in cycling, proteins that target intracellular localization and enhance intracellular half-life. The technique of receptor-mediated endocytosis is described, for example, by Wu et al., J. Biol. Chem. 262, 4429-4432 (1987); and Wagner et al., Proc. Natl. Acad. Sci. USA 87, 3410-3414 (1990). For review of gene marking and gene therapy protocols see Anderson et al., Science 256, 808-813(1992).

The nucleic acid molecules encoding the polypeptides or fragments thereof described herein are useful for chromosome identification. In this regard, there exists an ongoing need to identify new chromosome markers, since relatively few chromosome marking reagents, based upon actual sequence data are presently available. Each nucleic acid molecule of the present invention can be used as a chromosome marker.

Polypeptides and nucleic acid molecules of the invention may be used diagnostically for tissue typing, wherein the polypeptides may be differentially expressed in one tissue as compared to another, preferably in a diseased tissue as compared to a normal tissue of the same tissue type. Nucleic acid molecules will find use for generating probes for PCR, Northern analysis, Southern analysis and Western analysis.

This invention encompasses methods of screening compounds to identify those that prevent the effect of the polypeptide (antagonists). Screening assays for antagonist drug candidates are designed to identify compounds that bind or complex with the polypeptides encoded by the genes identified herein, or otherwise interfere with the interaction of the encoded polypeptides with other cellular proteins, including e.g., inhibiting the expression of the polypeptide from cells. Such screening assays will include assays amenable to high-throughput screening of chemical libraries, making them particularly suitable for identifying small molecule drug candidates.

The assays can be performed in a variety of formats, including protein-protein binding assays, biochemical screening assays, immunoassays, and cell-based assays, which are well characterized in the art.

All assays for antagonists are common in that they call for contacting the drug candidate with a polypeptide or polypeptide complex under conditions and for a time sufficient to allow these components to interact.

In binding assays, the interaction is binding and the complex formed can be isolated or detected in the reaction mixture. In a particular embodiment, the polypeptide or the drug candidate is immobilized on a solid phase, e.g., on a microtiter plate, by covalent or non-covalent attachments. Non-covalent attachment generally is accomplished by coating the solid surface with a solution of the polypeptide and drying. Alternatively, an immobilized antibody, e.g., a monoclonal antibody, specific for the polypeptide to be immobilized can be used to anchor it to a solid surface. The assay is performed by adding the non-immobilized component, which may be labeled by a detectable label, to the immobilized component, e.g., the coated surface containing the anchored component. When the reaction is complete, the non-reacted components are removed, e.g., by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labeled antibody specifically binding the immobilized complex.

If the candidate compound interacts with but does not bind to a polypeptide, its interaction with that polypeptide can be assayed by methods well known for detecting protein-protein interactions. Such assays include traditional approaches, such as, e.g., cross-linking, co-immunoprecipitation, and co-purification through gradients or chromatographic columns. In addition, protein-protein interactions can be monitored by using a yeast-based genetic system described by Fields and co-workers (Fields and Song, Nature (London), 340:245-246 (1989); Chien et al., Proc. Natl. Acad. Sci. USA, 88:9578-9582 (1991)) as disclosed by Chevray and Nathans, Proc. Natl. Acad. Sci. USA, 89: 5789-5793 (1991). Many transcriptional activators, such as yeast GAL4, consist of two physically discrete modular domains, one acting as the DNA-binding domain, the other one functioning as the transcription-activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-lacZ reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER™) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

Compounds that interfere with the interaction of a gene encoding a polypeptide identified herein and other intra- or extracellular components can be tested as follows: usually a reaction mixture is prepared containing the product of the gene and the intra- or extracellular component under conditions and for a time allowing for the interaction and binding of the two products. To test the ability of a candidate compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described hereinabove. The formation of a complex in the control reaction(s) but not in the reaction mixture containing the test compound indicates that the test compound interferes with the interaction of the test compound and its reaction partner.

To assay for antagonists, the polypeptide may be added to a cell along with the compound to be screened for a particular activity and the ability of the compound to inhibit the activity of interest in the presence of the polypeptide indicates that the compound is an antagonist to the polypeptide. Alternatively, antagonists may be detected by combining the polypeptide and a potential antagonist with membrane-bound polypeptide receptors or encoded receptors under appropriate conditions for a competitive inhibition assay. The polypeptide can be labeled, such as by radioactivity, such that the number of polypeptide molecules bound to the receptor can be used to determine the effectiveness of the potential antagonist. The gene encoding the receptor can be identified by numerous methods known to those of skill in the art, for example, ligand panning and FACS sorting. Coligan et al., Current Protocols in Immun., 1(2): Chapter 5 (1991). Preferably, expression cloning is employed wherein polyadenylated RNA is prepared from a cell responsive to the polypeptide and a cDNA library created from this RNA is divided into pools and used to transfect COS cells or other cells that are not responsive to the polypeptide. Transfected cells that are grown on glass slides are exposed to labeled polypeptide. The polypeptide can be labeled by a variety of means including iodination or inclusion of a recognition site for a site-specific protein kinase. Following fixation and incubation, the slides are subjected to autoradiographic analysis. Positive pools are identified and sub-pools are prepared and re-transfected using an interactive sub-pooling and re-screening process, eventually yielding a single clone that encodes the putative receptor.

More specific examples of potential antagonists include antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the polypeptide that recognizes the receptor but imparts no effect, thereby competitively inhibiting the action of the polypeptide.

Another potential antagonist is an antisense RNA or DNA construct prepared using antisense technology, where, e.g., an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes the mature polypeptides herein, can be used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al., Science, 241: 456 (1988); Dervan et al., Science, 251:1360 (1991)), thereby preventing transcription and the production of the polypeptide. The antisense RNA oligonucleotide hybridizes to the mRNA *in vivo* and blocks translation of the mRNA molecule into the polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the polypeptide. When antisense DNA is used, oligodeoxyribonucleotides derived from the translation-initiation site, e.g., between about -10 and +10 positions of the target gene nucleotide sequence, are preferred.

Potential antagonists include small molecules that bind to the active site, the receptor binding site, or growth factor or other relevant binding site of the polypeptide, thereby blocking the normal biological activity of the polypeptide. Examples of small molecules include, but are not limited to, small peptides or peptide-like molecules, preferably soluble peptides, and synthetic non-peptidyl organic or inorganic compounds.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, e.g., Rossi, Current Biology, 4:469-471 (1994), and PCT publication No. WO 97/33551 (published September 18, 1997).

Nucleic acid molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details see, e.g., PCT publication No. WO 97/33551, *supra*.

These small molecules can be identified by any one or more of the screening assays discussed hereinabove and/or by any other screening techniques well known for those skilled in the art.

Isolated polypeptide-encoding nucleic acid can be used for recombinantly producing polypeptide using techniques well known in the art and as described herein. In turn, the produced polypeptides can be employed for generating antibodies using techniques well known in the art and as described herein.

Antibodies specifically binding a polypeptide identified herein, as well as other molecules identified by the screening assays disclosed hereinbefore, can be administered for the treatment of various disorders, including cancer, in the form of pharmaceutical compositions.

If the polypeptide is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g.,* Marasco et al., Proc. Natl. Acad. Sci. USA, 90: 7889-7893 (1993).

The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The following examples are offered for illustrative purposes only, and are not intended to limit the scope of the present invention in any way.

EXAMPLES

*Reagents*

Lys-plasmin and Lys-plasminogen were from Heamatologic Technologies Inc., (Essex Junction, VT). Pro-tPA was from Biodesign International (Saco, ME), uPA (high molecular weight form) from American Diagnostica (Greenwich, CT), pro-uPA from Cortex Biochem (San Leandro, CA) and PAI-1 from Molecular Innovations (Southfield, MI). The chromogenic substrates S2444, S2765 and S2366 were from Diapharma (Westchester, OH). T.in.Pro cells were from Expression System LLC (Woodland, CA). Nickel-nitrilotriacetic acid resin was from Qiagen Inc (Chatsworth, CA), Q-Sepharose and benzamidine-Sepharose 4 Fast Flow from GE Healthcare (Piscataway, NJ).

*Construction, expression and purification of recombinant proteins*

A soluble form of hepsin comprising the entire extracellular domain was produced by use of a baculovirus expression system. A secreted His-tagged hepsin cDNA was constructed by fusion of the cDNA coding for the signal sequence of honey bee melittin (Met$^1$-Tyr$^{20}$) with the cDNA coding for the extracelluar domain of human hepsin (Arg$^{45}$-Leu$^{417}$). The final cDNA construct was inserted in a baculovirus expression vector under the control of a polyhedrin promoter and expressed in T.in.Pro cells. Hepsin was purified by nickel-nitrilotriacetic acid affinity chromatography essentially as previously described (18). Hepsin-containing medium was conditioned with 1 mM sodium azide, 0.3 M NaCl and 15 mM Imidazole and was adjusted to pH 6.5 using NaOH. Pre-charged nickel-nitrilotriacetic acid resin was added to media (4 ml resin/l L medium). Batch absorption was performed by gently stirring at 4°C for 2 h. After allowing the resin to settle for 1 h, the supernatant was decanted and the resin packed into a column. The column was washed with a minimum of 10 column volumes of PBS/0.3 M NaCl pH 7.4, then followed by 10 column volumes of 25 mM Imidazole, 0.3 M NaCl, 1 mM sodium azide pH 8.0. Proteins were eluted with 250 mM Imidazole, 0.3 M NaCl, 1 mM sodium azide pH 8.0. Pooled fractions were purified further using either ion-exchange chromatography on a Q-Sepharose FF ion or affinity chromatography on a benzamidine-Sepharose 4 Fast Flow column.

The matriptase protease domain was expressed in *E.coli* and purified as previously described (20). HGFA and soluble HAI-2 were recombinantly expressed and purified as previously described (18,20). The active site concentration of hepsin, matriptase and HGFA was determined by using the potent Kunitz domain inhibitor KD1 derived from HAI-1B, which was produced in *E.coli* as previously described (21). The active site concentrations were used for all enzymatic assays. For plasmin, the concentration provided by the supplier (Haematologic Laboratories, Inc.) was used.

*Monoclonal anti-hepsin antibody*

Five Balb/c mice (Charles River Laboratories, MA, USA) were hyperimmunized with recombinant soluble hepsin in RIBI adjuvant (Ribi Immunochem Research Inc., MO, USA). B cells from lymph nodes from five mice were fused with mouse myeloma cells (X63.Ag8.653; American Type Culture Collection, MD, USA) as previously described (22). After 10 - 14 days, the supernatants were harvested and screened for antibody production with a hepsin binding ELISA. The clone 3H10 showed high immunobinding and specificity after the second round of single cell per well cloning (Elite 1 Sorter, Beckman Coulter, CA, USA) and was scaled up for purification in INTEGRA CELLine 1000 (Integra Biosciences, AG, Switzerland). The supernatant was purified by Protein A affinity chromatography, sterile-filtered and stored at 4°C in PBS. Isotyping with the mono-AB-ID SP Kit (Zymed Laboratories, CA, USA) showed that 3H10 is an IgG1 κ.

*Production of hepsin over-expressing LnCaP cells*

The human prostate carcinoma cell line, LnCaP-FGC (LnCaP), was obtained from American Type Culture Collection (Manassas, VA). The cells were cultured in RPMI 1640 medium (ATCC, Manassas, VA) plus 10% fetal bovine serum (Sigma-Aldrich, St. Louis, MO). A LnCaP clone that stably expressed the firefly luciferase gene (LnCaP-luc) was used for hepsin transfection experiments. To establish the LnCaP-luc cell line the luciferase gene was subcloned as an EcoRI/XhoI cDNA fragment inserted into the pMSCVneo expression vector (BD Biosciences-Clontech, Mountain View, CA). LnCaP cells were transfected with the luciferase construct using Lipofectamine 2000 (Invitrogen, Carlsbad, CA). The cells were selected with 500 μg/ml Geneticin (Invitrogen) and clones were screened for bioluminescence activity by using the Luclite kit (PerkinElmer, Boston, MA). The clone LnCaP-luc, which produced the strongest luminescence signal, was chosen for hepsin transfection experiments.

The cDNA of full-length hepsin was inserted into a mammalian expression vector containing the puromycin resistance gene for antibiotic selection (Genentech, South San Francisco, CA). The LnCaP-luc clone was transfected with the construct encoding full-length hepsin with a C-terminal Flag tag and the cells were selected with 0.5 μg/ml puromycin (Sigma-Adrich). The clones were analysed by FACS for hepsin surface expression using an anti-Flag monoclonal antibody (Sigma-Adrich). Two clones, the high hepsin expressor LnCaP-34 and the low hepsin expressor LnCaP-17, were selected for further experiments.

To measure total hepsin expression (= endogenous and transfected) on the cell surface, LnCaP-34 and LnCaP-17 cell suspensions in PBS/1% (v/v) FBS were incubated with 10 μg/ml of 3H10 antibody or without antibody (= control) for 40 min on ice. The cells were washed twice with PBS prior to incubation with PE-conjugated F(ab')$_2$ goat anti-mouse IgG (Jackson Immunoresearch Laboratories Inc. PA, USA) diluted 1:1000 in PBS/1%FBS (v/v). After 30 min on ice the cells were washed with PBS and cell pellets resuspended in 1% formalin (Richard Allen Scientific, MI, USA). Antibody binding was measured on a FAGSscan (BD Biosciences, San Jose, CA).

*Real-time reverse transcription-PCR*

Total RNA was isolated from LnCaP-17 and LnCaP-34 cells using RNeasy Mini Kit (Qiagen, Valencia, CA). Gene expression analysis was performed by real-time reverse transcriptase PCR (TaqMan) on a model 7500 sequence detector (ABI-Perkin Elmer, Foster City, CA). To specifically measure endogenous hepsin we used primers recognizing sequences in the 3' UTR of the hepsin gene. To measure total hepsin (= endogenous plus transfected hepsin) we used primers recognizing sequences in the ORF that is common to both hepsins. The sequences of primers and probes were as follows. Endogenous hepsin: forward 5'-CCCTCCAGGGTCCTCTCT-3' reverse 5'-AGTCCCAGACAGCAGAACAATA-3' probe 5'-(FAM) CAGCCCCGAGACCACCCAAC (TAMRA)-3' total hepsin: forward 5'-GCTGTGTGGCATTGTGAGT-3' reverse 5'-TGAGTCTTTATGGCCTGGAA-3' probe, 5'-(FAM) AAGCCAGGCGTCTACACCAAAGTCAC (TAMRA)-3' matriptase: forward 5'- CTTCGGAGCCTCCTCAGT-3', reverse 5'-GTCTCAGACCCGTCTGTTTTC-3' probe 5'-(FAM) CCTCCGAGCCTGGGCTTCCT (TAMRA)-3'; GAPDH: forward, 5'-GAAGGTGAAGGTCGGAGTC-3' reverse 5'-GAAGATGGTGATGGGATTTC-3' probe 5'-(FAM) CAAGCTTCCCGTTCTCAGCC (TAMRA)-3. The reverse transcription was carried out at 48°C for 30 min followed by heat activation of AmpliTaq Gold at 95°C for 10 min. The thermal cycling proceeded with 40 cycles of 95°C for 0.5 min and 60°C for 1 min. All samples were run in duplicates. The results were quantified using the standard curve method according to the manufacturer's instruction (ABI-Perkin Elmer). All gene expression levels were normalized to the house keeping gene GAPDH.

*Enzymatic assays with HAI-2 and the small molecule inhibitor HI-10331*

The reversible active site inhibitor HI-10331 (Genentech, Inc.) (having the molecule structure depicted in Figure 10, and disclosed in U.S. Pat. No. 6,472,393) or HAI-2 were incubated with 0.5 nM hepsin, 0.5 nM matriptase and 10 nM uPA for 30 min in Hepes buffer (20 mM Hepes, pH 7.5, 150 mM NaCl, 5 mM CaCl2, 0.01% Triton X-100) at room temperature. The chromogenic substrates S2366 (for hepsin), S2765 (for matriptase) and S2444 (for uPA) were added at a concentration corresponding to their respective $K_m$ values, which were determined in separate experiments. After substrate addition, the linear rates of the increase in absorbance at 405 nm was measured on a kinetic microplate reader (Molecular Devices, Sunnyvale, CA).

For HI-10331, the inhibitor concentration that gave a 50% inhibition of the enzymatic activity ($IC_{50}$) was determined by fitting the data to a four-parameter equation (Kaleidagraph version 3.6, Synergy Software, Reading, PA). The $K_i$ values were calculated according to the relationship $K_i = IC_{50}/(1 + [S]/K_m)$ (23) using the experimentally determined $IC_{50}$ and $K_m$ values for each enzyme-substrate pair. For HAI-2, the apparant $K_i$ values ($K_i^*$) were determined by fitting the data to the equation for tight binding inhibition (24,25).

*Pro-uPA activation by LnCaP cells*

Confluent LnCaP-34 and LnCaP-17 cells were washed with HBSA-Glucose buffer (20 mM Hepes pH 7.5, 150 mM NaCl, 5 mM CaCl2, 0.05 mg/ml BSA, 5 mM glucose) and 0.8 ml of 100 nM pro-uPA in pre-warmed HBSA-Glucose buffer was added to the cell layers. The inhibitors KD1 or HI-10331 were added to give final concentrations of 1 μM and 10 μM, respectively. The culture plates were kept at 37°C and 50 μl samples were withdrawn at different time points, supplemented with 0.2 ml of 0.625 M S2444 in Hepes buffer and the increase in absorbance at 405 nm measured on a kinetic microplate reader. Cell numbers were determined at the end of the experiments. The concentration of formed uPA in each sample was calculated from a standard curve of enzymatically converted pro-uPA and normalized to $10^6$ cells. After subtracting the background levels of uPA formed in the absence of cell layer, the linear rates of uPA formation/$10^6$ cells were determined. The pro-uPA activation was strictly dependent on the presence of the cells, since the samples taken at different time points did not convert any additional pro-uPA. Also, the enzymatic activity towards S2444 of the withdrawn samples was entirely due to uPA activity and not to hepsin or other proteases released from the cell surface, since the chromogenic activity of the samples was not inhibited by addition of the hepsin inhibitor KD1, but was completely inhibited by the uPA inhibitor PAI-1 (data not shown).

For immunoblotting experiments, confluent LnCaP-34 cell layers were washed as above and incubated with 30 nM pro-uPA at 37°C. After 1 hr, 3 hr and 5 hr, aliquots were taken and immediately added to SDS sample buffer. The proteins were separated by SDS-PAGE and transferred onto nitrocellulose filters using the Bio-Rad Semi Dry Transfer system. Pro-uPA and uPA were visualized by using a rabbit polyclonal anti-uPA antibody (Cell Sciences; Canton, MA) followed by HRP-conjugated anti-rabbit antibody (Jackson ImmunoResearch Laboratory;West Grove,PA ) and ECL (GE Healthcare;Piscataway, NJ) enhancement.

*Analysis of pro-uPA and pro-tPA activation by SDS-PAGE*

Pro-uPA at a concentration of 1.5 μM was incubated with 15 nM hepsin or 15 nM plasmin in Hepes buffer at room temperature. After 4 min and 60 min aliquots of the reaction mixture were taken and added to 6x SDS sample buffer. The samples were analyzed by SDS-PAGE using a 4-20% gradient gel (Invitrogen; Carlsbad,CA). Protein was visualized after staining with SimplyBlue Safe Stain (Invitrogen). Experiments with pro-tPA (1.5 μM) were carried out identically, except that the buffer used was 50 mM sodium phosphate pH 7.5, 200 mM arginine, 0.01 % Tween-20.

*Determination of first-order rate Constants*

The $K_m$ and $k_{cat}$ values for pro-uPA activation could not be accurately determined because the pro-uPA stock solution from the supplier (0.8 mg/ml) was not sufficiently high. Therefore, we chose to determine the first-order rate constant k as a measure of pro-uPA conversion efficiency. It was ensured that the pro-uPA concentration used (30 nM) was in the range of first-order kinetics for the enzymes tested, i.e. hepsin, plasmin and matriptase. The rate of uPA formation by these enzymes was linear up to 200 nM of pro-uPA. Pro-uPA (30 nM) was added to the enzymes (3 nM) in Hepes buffer to start the reaction at room temperature. At various time points, 50-$\mu$l aliquots were removed and added to 150 $\mu$l of 667 nM HAI-2 (final concentration in 250 $\mu$l was 400 nM) in Hepes buffer to stop further pro-uPA cleavage. At the concentration used, HAI-2 specifically inhibits plasmin (26), HGFA (27), hepsin and matriptase, but not the newly generated uPA (Table 1). Our results on HAI-2 inhibition of plasmin and HGFA (data not shown) were consistent with the published data (26,27). After addition of 50 $\mu$l of 2.5 mM S2444, the increase in absorbance at 405 nm was measured on a kinetic microplate reader. The concentration of uPA in each aliquot was calculated from a standard curve of enzymatically converted pro-uPA. The data were expressed as the decrease in the concentration of pro-uPA (log [pro-uPA]) as function of time. The first-order rate constant k was calculated from the slope using the equation

$$\log [S] = -(k/2.3)\, t + \log[S]_0$$

where [S] is the concentration of pro-uPA and $[S]_0$ is the initial pro-uPA concentration. The catalytic efficiency, $k_{cat}/K_m$, was t*hen calculated using the known enzyme concentration [E] and the relationship* k = $(k_{cat}/K_m)$ [E]. The values are the average of 5 experiments $\pm$ SD.

*Enzyme kinetics of uPA generated by hepsin- and plasmin-mediated cleavage of pro-uPA*

Pro-uPA (30 nM) was completely converted to uPA by 3 nM hepsin or plasmin during a 2 h reaction in Hepes buffer at room temperature. The controls were 3 nM hepsin or plasmin in Hepes buffer without pro-uPA. Sample aliquots of uPA generated by hepsin (uPA$_{Hepsin}$), by plasmin (uPA$_{Plasmin}$) and their respective controls were stored at -20°C until further analysis.

For chromogenic assays, thawed samples were 5-fold diluted in Hepes buffer containing uPA chromogenic substrate S2444 (1 nM-1000 nM) and 400 nM HAI-2 to inhibit hepsin and plasmin activities. The inital rates of S2444 cleavage were measured as change of absorbance at 405 nm on a kinetic microplate reader and expressed as $\mu$M paranitroanilide (pNA)/min using a pNA standard curve. The rates of pNA formation were presented as a function of S2444 concentration and the $K_m$ and $V_{max}$ were determined after fitting the data to a 4-parameter equation (Kaleidagraph version 3.6, Synergy Software).

For plasminogen activation assays, a dilution-jump method was used. The reaction was started by adding 4 $\mu$M plasminogen to an equal volume of 25-fold diluted samples (UPA$_{Hepsin}$, uPA$_{Plasmin}$ and respective controls). Aliquots of the reaction mixtures were taken at different time points and diluted 51-fold into 0.5 mM of the plasmin substrate S2366. Then, the increase in absorbance at 405 nM was measured on a kinetic microplate reader. By use of a plasmin standard curve the concentration of plasmin in each aliquot was determined and the initial rates of plasmin formation were calculated.

**Results**

*Cleavage of pro-uPA by hepsin produces enzymatically active uPA*

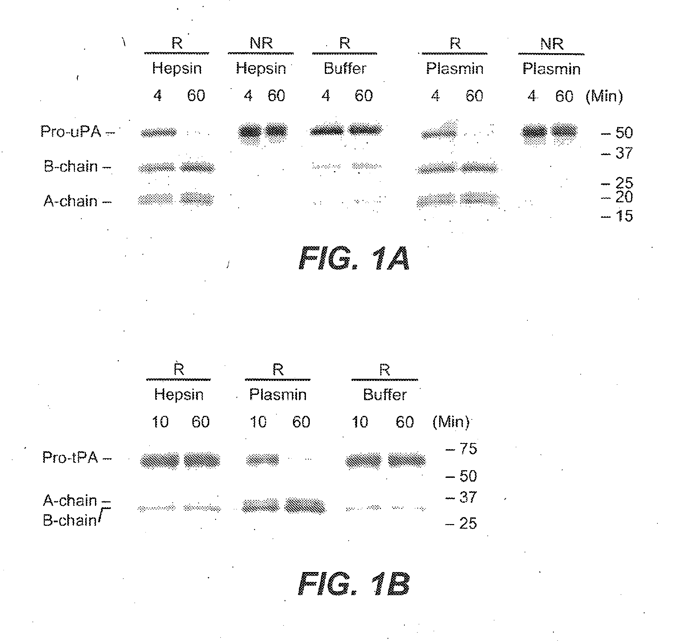

Incubation of pro-uPA with hepsin resulted in a time-dependent cleavage of the single-chain form into the two-chain form consisting of the 20 kDa A-chain and the 30 kDa B-chain (protease domain) (Fig. 1A). Both chains remained disulfide-linked, as shown by the single protein band of 55 kDa under non-reducing conditions (Fig.1A). The N-terminal sequence of the 20 kDa band was [1]SNELHQVPS[9] (=A-chain) and that of the 30 kDa band was [159]IIGGEFTTIENQ[170] (=B-chain), indicating that hepsin generated the high molecular weight form of uPA by processing pro-uPA at the consensus cleavage site Lys[158]-Ile[159]. In agreement, cleavage of pro-uPA by plasmin generated identical fragments and with a time-course similar to hepsin. (Fig.1A). Extended incubation of pro-uPA with hepsin did not produce the 32 kDa low molecular weight form of uPA (under non-reducing conditions) (28), nor any visible degradation products. Pro-tPA, which is structurally related to pro-uPA, was only cleaved by plasmin but not by hepsin (Fig.1B).

To assess the enzymatic function of uPA generated by hepsin, pro-uPA was completely converted by hepsin (to give uPA$_{Hepsin}$) or by plasmin (to give uPA$_{Plasmin}$) and then analyzed in two assay systems, i.e. cleavage of the small synthetic pNA substrate S2444 and of the macromolecular substrate plasminogen. First, the reaction velocity as a function of S2444 concentration was identical for uPA$_{Hepsin}$ and uPA$_{Plasmin}$ (Fig.2A). The determined $K_m$ for uPA$_{Hepsin}$ and uPA$_{Plasmin}$ were 34.1 $\pm$ 2.2 $\mu$M and 34.6 $\pm$ 1.5 $\mu$M (n =3; $\pm$ SD), respectively, and the $V_{max}$ values were 1.74 $\pm$

0.51 $\mu$M pNA min$^{-1}$ and 1.70 $\pm$ 0.51$\mu$ M pNA min$^{-1}$ (n =3; $\pm$ SD), respectively. Secondly, in plasminogen activation assays (Fig. 2B) the initial rates of plasmin generation by uPA$_{Hespin}$ and uPA$_{Plasmin}$ were 29.3 $\pm$ 5.0 nM plasmin min$^{-1}$ and 26.4 $\pm$ 4.5 nM plasmin min$^{-1}$ (n = 3; $\pm$ SD), respectively. In both assay systems, neither of the two controls (= appropriately diluted hepsin and plasmin) had any activity (Fig. 2A, B).

*Enzyme kinetics of pro-uPA activation by hepsin, plasmin and matriptase*

In order to compare the efficiency of hepsin to convert pro-uPA with those of other known pro-uPA activators, such as plasmin and another member of the TTSP family, matriptase (29,30), we determined the first-order rate constants of pro-uPA activation. The use of the bi-Kunitz inhibitor HAI-2 allowed us to stop the reaction at different time points and to measure the time-dependent uPA generation, because HAI-2 is a potent inhibitor of plasmin (26), hepsin and matriptase but not of uPA (Table I) (26). HGFA (31), which is known to only activate pro-HGF, was used as a negative control. The results in Figure 3 show the first-order reaction kinetics, indicating that hepsin and plasmin were equally active in processing pro-uPA, whereas matriptase cleaved pro-uPA at a significantly lower rate. During the experiment, hepsin and plasmin converted about 70% of the added pro-uPA. The determined first-order rate constant *k* as well as the calculated catalytic efficiencies ($k_{cat}/K_m$) are summarized in Table II showing that hepsin and plasmin were about 6-fold more efficient pro-uPA activators compared to matriptase. The catalytic efficiency of plasmin determined in our experiments was in the same range (1.9 - 6.2 x $10^5$ M$^{-1}$ s$^{-1}$) as determined by Lijnen et al. (32), Collen et al. (33) and Wolf et al. (34). A comparison of hepsin activity with other pro-uPA converting enzymes including mast cell tryptase (35), tryptase $\varepsilon$ (36), T-cell related serine protease (37), plasma kallikrein (38), mouse glandular kallikrein-6 (39), various cathepsins (40-42) and nerve growth factor-$\gamma$ (34) is difficult, since kinetic constants were not always reported. In comparison to the cases where data on catalytic efficiencies ($k_{cat}/K_m$) are available, such as tryptase $\varepsilon$ (2.4 x $10^5$ M$^{-1}$ s$^{-1}$) (36), mast cell tryptase (2.4 x $10^3$ M$^{-1}$ s$^{-1}$) (35), nerve growth factor-$\gamma$ (1.3 x $10^4$ M$^{-1}$ s$^{-1}$) (34) and glandular kallikrein (3.3 x $10^3$ M$^{-1}$ s$^{-1}$) (39), hepsin activity ($k_{cat}/K_m$ 4.84 x $10^5$ M$^{-1}$ s$^{-1}$) appears very high.

Table 1. *Equilibrium dissociation constants for the interaction of HAI-2 and the small molecule hepsin inhibitor HI-10331 with serine proteases*

| Protease | HAI-2 $K_i^{*\ a-}(nM)$ $\pm$ SD | HI-10331 $K_i$ (nM) $\pm$ SD |
|---|---|---|
| Hepsin | 0.26 $\pm$ 0.04 | 41.6 $\pm$ 1.1 |
| Matriptase | 0.85 $\pm$ 0.21 | >10,000 |
| u-PA | >1,000 | >10,000 |

$^a$ $K_i^*$, apparent equilibrium dissociation constant.

Table II. *Kinetic constant of pro-uPA activation by hepsin, plasmin and matriptase*

| Enzyme | $k$ ($10^{-3}$ min$^{-1}$) $\pm$ SD | $k_{cat}/K_m$($10^5$ M$^{-1}$ S$^{-1}$) $\pm$ SD |
|---|---|---|
| Hepsin | 87.1 $\pm$ 11.3 | 4.84 $\pm$ 0.63 |
| Plasmin | 81.5 $\pm$ 14.2 | 4.53 $\pm$ 0.79 |
| Matriptase | 14.2 $\pm$ 4.9 | 0.79 $\pm$ 0.27 |

*Pro-uPA activation by cell surface-expressed hepsin.*

In order to study pro-uPA processing on the cell surface, we established the LnCaP cell line LnCaP-34, which stably overexpressed full-length hepsin. Total hepsin RNA levels were about 9-fold higher than those of LnCaP-17, which only expressed endogenous hepsin as determined by RT-PCR Taqman using specific primer/probe sets (Fig. 4A). Neither the endogenous hepsin expression nor matriptase expression was changed in the LnCaP-34 cells (Fig.4A). Moreover, the monoclonal anti-hepsin antibody 3H10 allowed us to detect hepsin protein on the LnCaP cell surface. This antibody was raised against soluble hepsin extracellular domain and specifically recognized hepsin protein on LnCaP cells, but not on HPAC cells, which do not express any detectable hepsin mRNA (data not shown). The LnCaP-34 cells exhibited higher surface expression levels of hepsin compared to the LnCaP-17 cells, consistent with the different mRNA levels (Fig. 4B). The mean fluorescence intensity of the LnCaP-34 cells was 5-fold higher than the LnCaP-17 cells, indicating that LnCaP-34 expressed about 5-fold more hepsin on the cell surface.

The cell surface activity of hepsin was measured by incubating LnCaP-34 cells with 30 nM pro-uPA for 1 h, 3 h and 5 h at 37°C. Immunoblotting showed a time-dependent conversion of pro-uPA into the high molecular weight form as indicated by the appearance of a 30 kDa (B-chain) and 20 kDa (A-chain) band (Fig. 5A). The two chains were disulfide-linked as shown by the presence of a single 55 kDa band under nonreducing conditions, identical to the experiments with soluble hepsin (Fig. 1A). Addition of the HAI-1B-derived Kunitz domain inhibitor KD1 (21) strongly inhibited pro-uPA cleavage (Fig. 5A).

The rates of uPA formation by cell surface hepsin were quantified with an enzymatic assay. To measure cell surface hepsin activity, pro-uPA was added to the cell layers and the time-dependent formation of uPA was determined. Consistent with the different levels of hepsin expression, the rates of uPA formation were higher for LnCaP-34 compared with the LnCaP-17 (Fig. 5B, Table III). The KD1 inhibitor almost completely inhibited pro-uPA cleavage by both cell lines (Fig. 5B, Table III). Since KD1 does not inhibit uPA (21), it did not interfere with the determination of uPA concentrations in the second stage of the assay.

In addition to hepsin, both LnCaP-34 and LnCaP-17 express the pro-uPA activator matriptase (Fig. 4A), which can also be inhibited by KD1 (21). To find out whether matriptase activity contributed to uPA formation in this system, we used the small molecule inhibitor HI-10331, which inhibited hepsin more than 200-fold more potently than matriptase, but did not interfere with uPA activity (Table I). We found that HI-10331 reduced the pro-uPA activation rates by the hepsin over-expressing LnCaP-34 almost to the same level as KD1 (Table III), indicating that, compared to hepsin, there was little pro-uPA converting activity by matriptase (<10%). On the other hand, on LnCaP-17 cells, which had an about 3-fold lower pro-uPA converting activity, the potential contribution by matriptase was more substantial, since the rate of pro-uPA cleavage remained at about 30% of the control rate in the presence of HI-10331 (Table III).

Table III. *Pro-uPA activation by LnCaP cell lines*

| LnCaP clone | Rate of pro-uPA activation per $10^6$ cells (nM uPA hr$^{-1}$) $\pm$ SD | | |
| --- | --- | --- | --- |
| | Control | 1 $\mu$M KD1 | 10 $\mu$M HI-10331 |
| LnCaP-34 | 6.6 $\pm$ 1.9 | 0.2 $\pm$ 0.1 | 0.4 (0.4, 0.3)[a] |
| LnCaP-17 | 2.4 $\pm$ 0.9 | 0.2 $\pm$ 0.2 | 0.7 (0.8, 0.6)[a] |

[a] Results of two separate experiments are shown in parenthesis.

## Discussion

The herein reported pro-uPA converting activity of hepsin provides a molecular basis to link hepsin overexpression with prostate cancer progression. Hepsin itself does not directly convert plasminogen to plasmin (18), but instead amplifies plasmin generation by activating pro-uPA. uPA proteolytically cleaves plasminogen to plasmin, which in turn degrades components of basement membranes and the extracellular matrix either directly or indirectly by activating latent matrix metalloproteases (43,44). The association of the plasminogen activation system with tumor invasion and dissemination is well documented (43,44). Hepsin also activates the latent form of the invasive growth factor HGF (17,18), and both HGF and uPA have been implicated in prostate cancer growth and metastasis (45-48). Moreover, by activating pro-HGF, hepsin may directly regulate the local levels of its substrate pro-uPA, since HGF was shown to induce pro-uPA transcription and protein synthesis (49,50). Therefore, tumor cell surface expressed hepsin could be central in orchestrating invasive pathways at the tumor/stroma interface. Such a concept is consistent with the hepsin-mediated tumor progression and the associated basement membrane disruption in a mouse model of prostate cancer (16).

The ability of hepsin to activate pro-uPA as well as pro-HGF (17,18) is akin to the TTSP matriptase (20,29,30), which like hepsin is overexpressed in prostate and ovarian cancer (51-55). Therefore, hepsin and matriptase, may engage in overlapping functions that contribute to tumor progression. Their increased expression in prostate and ovarian tumors is not paralleled by HAI-1 (53-55), a bi-Kunitz inhibitor that is also expressed on tumor epithelium and potently inhibits both enzymes in vitro (17,18,20,56). In fact, the HAI-1 expression levels during prostate cancer progression were reported to remain unchanged (54) or to be reduced (55). In either case this would result in an enzyme:inhibitor imbalance that might favor tumor progression. An intriguing example that illustrates the consequences of an unbalanced enzyme:inhibitor system is matriptase overexpression in the mouse skin epidermis, where normally matriptase and its physiologic inhibitor HAI-1 are co-expressed in a regulated manner. Matriptase overexpression resulted in the spontaneous formation of neoplastic lesions, which could be completely prevented by the simultaneous overexpression of the inhibitor HAI-1 (57). Clearly, there are important functional differences between matriptase and hepsin. First, unlike matriptase, the in vivo overexpression of hepsin in mouse prostate did not lead to spontaneous neoplasia (16). Secondly, matriptase is more widely expressed in normal and tumor tissues and engages in physiologic functions, such as skin epidermal differentiation (58), in which hepsin is not involved. Nevertheless, the concerted upregulation of both proteases in prostate and ovarian cancer, combined with their similar substrate specificities suggests a functional cooperation in cancer progression.

Hepsin specifically cleaved pro-uPA at the consensus $Lys^{158}$-$Ile^{159}$ peptide bond to produce high molecular weight uPA, which was identical to the reference uPA material in respect to small synthetic and macromolecular substrate activation. Thus, hepsin converted pro-uPA into fully functional uPA in respect to both catalysis and uPA-receptor binding, since high molecular weight uPA contains the receptor binding site located in the N-terminal EGF and Kringle domains (59). Because the enzymatic assays were carried out with a soluble form of hepsin, it was important to assess whether pro-uPA conversion was recapitulated by full-length hepsin on the cell surface. Using the hepsin overexpressing LnCaP-34 cell line, we were able to demonstrate that like soluble hepsin, the cell surface-expressed hepsin converted pro-uPA into high molecular weight uPA. In agreement with the higher hepsin protein expression, the rate of uPA formation by LnCaP-34 cells (6.6 nM uPA $hr^{-1}$) was about 3-fold higher than the LnCaP-17 cells, which express about 5-fold less hepsin on the surface. Matriptase, which is also expressed by LnCaP-34 cells, did not significantly contribute to uPA generation. The lower matriptase activity towards pro-uPA in the cell-based system agrees well with the about 6-fold lower catalytic efficiency determined in purified enzyme assays.

The catalytic efficiency of hepsin for pro-uPA conversion was remarkably high ($k_{cat}/K_m$ 4.8 x $10^5$ $M^{-1}$ $s^{-1}$), being similar to plasmin, which is considered the most potent pro-uPA activator. This could imply that hepsin is an alternative pro-uPA activator, equivalent to plasmin in respect to efficiency. Hepsin thus differs from less potent pro-uPA convertases, which may only trigger the plasminogen/plasmin system by generating trace amounts of plasmin to allow for the efficient feedback activation of pro-uPA.

Except for pro-uPA, all known macromolecular substrates of hepsin have an Arg at the $P_1$ position (17-19), which is consistent with the results of a peptide library screening study that showed a strong preference of Arg over Lys as the $P_1$ residue (17). Yet, the dual recognition of macromolecular substrates with Arg or with Lys in $P_1$ is not without precedent for trypsin-like serine proteases, as exemplified by plasmin and plasma kallikrein. A molecular model of the $P_4$-$P_1$ sequence of pro-uPA (Pro-Arg-Phe-Lys) could be built without major difficulties using the published hepsin structure with the tetrapeptide Lys-Gln-Leu-Arg-cmk (17), derived from pro-HGF, in the active site (Fig. 6). The simple fit of Pro-Arg-Phe-Lys into this hepsin structure is imperfect, and would be consistent with a reduced affinity compared to the pro-HGF sequence. For instance, Lys at the $P_1$ position cannot make the same number of H-bonding interactions with the $S_1$ subsite that an Arg residue would, a Phe residue at position $P_2$ would require a small shift in the 90s loop ($Asn^{254}$) to make room for the bulkier side chain, and substitution of $P_4$ Lys with Pro eliminates weak interactions with $Glu^{252}$ and $Asn^{254}$ (Fig. 6). However, the substrate-binding clefts of trypsin family enzymes are not rigid, and a low energy conformational adjustment that would better match the pro-uPA sequence is very probably available. Indeed, given the rapid turnover of pro-uPA by hepsin, a suitable accommodation must be made.

Such reasoning fails to explain why another zymogen, pro-tPA, is a poor hepsin substrate, because the $P_4$-$P_1$ pro-tPA sequence is a hybrid of those from pro-HGF and pro-uPA (Table IV). In rationalizing the large difference in reactivity between pro-uPA and pro-tPA as substrates of hepsin, we conclude that factors beyond residues $P_4$-$P_1$ are probably responsible. Two possibilities present themselves. Firstly, there is a limit to how well short peptides represent intact proteins as enzyme substrates, since interactions outside the formal substrate binding cleft (exosites) can influence substrate turnover. Secondly, although their effects are less pronounced than residues at the N-terminal side of the scissile peptide bond, residues immediately C-terminal to the scissile bond can also influence substrate processing. Here we find that pro-tPA has a Lys at position $P_2$', instead of the almost invariably Val or Ile other among trypsin family members. The specific nature of substrate/enzyme interactions in the $P_1$' and $P_2$' positions are much less well characterized than in the $P_4$-$P_1$ positions. As a result, it is difficult to know if, or exactly how, a Lys at $P_2$' may affect substrate pro-tPA interactions with hepsin. Nonetheless, it is reasonable to speculate that a Lys would be poorly tolerated anywhere a Val or Ile has significant interactions.

Table IV. *Cleavage site sequences of uPA, tPA and HGF*

|         | $P_4$ | $P_3$ | $P_2$ | $P_1$ | $P_1$' | $P_2$' | $P_3$' |
|---------|-------|-------|-------|-------|--------|--------|--------|
| Pro-uPA | P     | R     | F     | K     | I      | I      | G      |
| Pro-tPA | P     | Q     | F     | R     | I      | K      | G      |
| Pro-HGF | K     | Q     | L     | R     | V      | V      | N      |

In conclusion, the activation of pro-uPA by hepsin provides a mechanistic basis to explain the basement membrane disorganization observed in the hepsin-overexpressing mouse prostate (16). The recent finding, though based on a small number of samples, that hepsin protein is strongly expressed in bone metastatic lesions and advanced primary prostate cancer (12) may indicate a role of hepsin during the later stages of disease. This would agree with the requirement of motility factors (e.g. HGF) and matrix degradation (uPA/plasmin), which could be influenced by hepsin enzymatic activity. The recognition that hepsin is a potent activator of pro-uPA and pro-HGF in vitro provides a conceptual framework to specifically investigate these questions in vivo. The recent development of potent protein-based inhibitors of hepsin, such as monoclonal antibodies (12) and HAI-1 derived Kunitz-domain inhibitor (21) may help to confirm hepsin's exact role(s) in cancer.

**Footnotes**

[1]TTSP, type II transmembrane serine protease; Kid1, Kunitz domain-1 derived from HAI-1B; pNA, para-nitroanilide; uPA, urokinase-type plasminogen activator; tPA, tissue-type plasminogen activator; pro-uPA, zymogen form of uPA; pro-tPA zymogen form of uPA; HGF, hepatocyte growth factor; pro-HGF, single chain form of HGF; HGFA, hepatocyte growth factor activator; HBSA-Glucose buffer (20 mM Hepes pH 7.5, 150 mM NaCl, 5 mM $CaCl_2$, 0.05 mg/ml BSA, 5 mM glucose); Hepes buffer (20 mM Hepes, pH 7.5, 150 mM NaCl, 5 mM $CaCl_2$, 0.01% Triton X-100).

**Partial List of References**

1. Somoza, J. R., Ho, J. D., Luong, C., Ghate, M., Sprengeler, P. A., Mortara, K., Shrader, W. D., Sperandio, D., Chan, H., McGrath, M. E., and Katz, B. A. (2003) Structure 11(9), 1.123-1131
2. Vu, T. K., Liu, R. W., Haaksma, C. J., Tomasek, J. J., and Howard, E. W. (1997) J Biol Chem 272(50), 31315-31320
3. Yu, I. S., Chen, H. J., Lee, Y. S., Huang, P. H., Lin, S. R., Tsai, T. W., and Lin, S. W. (2000) Thromb Haemost 84(5), 865-870
4. Wu, Q., Yu, D., Post, J., Halks-Miller, M., Sadler, J. E., and Morser, J. (1998) J Clin Invest 101(2), 321-326
5. Tanimoto, H., Yan, Y., Clarke, J., Korourian, S., Shigemasa, K., Parmley, T. H., Parham, G. P., and O'Brien, T. J. (1997) Cancer Res 57(14), 2884-2887
6. Dhanasekaran, S. M., Barrette, T. R., Ghosh, D., Shah, R., Varambally, S., Kurachi, K., Pienta, K. J., Rubin, M. A., and Chinnaiyan, A. M. (2001) Nature 412(6849), 822-826
7. Luo, J., Duggan, D. J., Chen, Y., Sauvageot, J., Ewing, C. M., Bittner, M. L., Trent, J. M., and Isaacs, W. B. (2001) Cancer Res 61(12), 4683-4688
8. Magee, J. A., Araki, T., Patil, S., Ehrig, T., True, L., Humphrey, P. A., Catalona, W. J., Watson, M. A., and Milbrandt, J. (2001) Cancer Res 61(15), 5692-5696
9. Stamey, T. A., Warrington, J. A., Caldwell, M. C., Chen, Z., Fan, Z., Mahadevappa, M., McNeal, J. E., Nolley, R., and Zhang, Z. (2001) J Urol 166(6), 2171-2177
10. Stephan, C., Yousef, G. M., Scorilas, A., Jung, K., Jung, M., Kristiansen, G., Hauptmann, S., Kishi, T., Nakamura, T., Loening, S. A., and Diamandis, E. P. (2004) J Urol 171(1), 187-191
11. Welsh, J. B., Sapinoso, L. M., Su, A. I., Kern, S. G., Wang-Rodriguez, J., Moskaluk, C. A., Frierson, H. F., Jr., and Hampton, G. M. (2001) Cancer Res 61(16), 5974-5978
12. xuan, J. A., Schneider, D., Toy, P., Lin, R., Newton, A., Zhu, Y., Finster, S., Vogel, D., Mintzer, B., Dinter, H., Light, D., Parry, R., Polokoff, M., Whitlow, M., Wu, Q., and Parry, G. (2006) Cancer Res 66(7), 3611-3619
13. Chen, Z., Fan, Z., McNeal, J. E., Nolley, R., Caldwell, M. C., Mahadevappa, M., Zhang, Z., Warrington, J. A., and Stamey, T. A. (2003) J Urol 169(4), 1316-1319
14. Srikantan, V., Valladares, M., Rhim, J. S., Moul, J. W., and Srivastava, S. (2002) Cancer Res 62(23), 6812-6816
15. Torres-Rosado, A., O'Shea, K. S., Tsuji, A., Chou, S. H., and Kurachi, K. (1993) Proc Natl Acad Sci U S A 90(15), 7181-7185
16. Klezovitch, O., Chevillet, J., Mirosevich, J., Roberts, R. L., Matusik, R. J., and Vasioukhin, V. (2004) Cancer Cell 6(2), 185-195
17. Herter, S., Piper, D. E., Aaron, W., Gabriele, T., Cutler, G., Cao, P., Bhatt, A. S., Choc, Y., Craik, C. S., Walker, N., Meininger, D., Hoey, T., and Austin, R. J. (2005) Biochem J 390(Pt 1), 125-136
18. Kirchhofer, D., Peek, M., Lipari, M. T., Billeci, K., Fan, B., and Moran, P. (2005) FEBS Lett 579(9), 1945-1950
19. Kazama, Y., Hamamoto, T., Foster, D. C., and Kisiel, W. (1995) J Biol Chem 270(1), 66-72
20. Kirchhofer, D., Peek, M., Li, W., Stamos, J., Eigenbrot, C., Kadkhodayan, S., Elliott, J. M., Corpuz, R. T., Lazarus, R. A., and Moran, P. (2003) J Biol Chem 278(38), 36341-36349
21. Shia, S., Stamos, J., Kirchhofer, D., Fan, B., Wu, J., Corpuz, R. T., Santell, L., Lazarus, R. A., and Eigenbrot, C. (2005) J Mol Biol 346(5), 1335-1349
22. Hongo, J. A., Mora-Worms, M., Lucas, C., and Fendly, B. M. (1995) Hybridoma 14(3), 253-260
23. Cheng, Y., and Prusoff, W. H. (1973) Biochem Pharmacol 22(23), 3099-3108
24. Morrison, J. F. (1969) Biochim Biophys Acta 185(2), 269-286
25. Olivero, A. G., Eigenbrot, C., Goldsmith, R., Robarge, K., Artis, D. R., Flygare, J., Rawson, T., Sutherlin, D. P., Kadkhodayan, S., Beresini, M., Elliott, L. O., DeGuzman, G. G., Banner, D. W., Ultsch, M., Marzec, U., Hanson, S.

R., Refino, C, Bunting, S., and Kirchhofer, D. (2005) J Biol Chem 280(10), 9160-9169

26. Delaria, K. A., Muller, D. K., Marlor, C. W., Brown, J. E., Das, R. C., Roczniak, S. O., and Tamburini, P. P. (1997) J Biol Chem 272(18), 12209-12214

27. Kawaguchi, T., Qin, L., Shimomura, T., Kondo, J., Matsumoto, K., Denda, K., and Kitamura, N. (1997) J Biol Chem 272(44), 27558-27564

28. Bachmann, F. (1994) *Colman, R.W., Hirsh, J, Marder V.J. and Salzman, E.W., Editors (Lippincott Company, Philadelphia),* 1592-1622

29. Lee, S. L., Dickson, R. B., and Lin, C. Y. (2000) J Biol Chem. 275(47), 36720-36725

30. Takeuchi, T., Harris, J. L., Huang, W., Yan, K. W., Coughlin, S. R., and Craik, C. S. (2000) J Biol Chem 275(34), 26333-26342

31. Miyazawa, K., Shimomura, T., Kitamura, A., Kondo, J., Morimoto, Y., and Kitamura, N. (1993) J Biol Chem 268(14), 10024-10028

32. Lijnen, H. R., Van Hoef, B., and Collen, D. (1987) Eur J Biochem 169(2), 359-364

33. Collen, D., Zamarron, C., Lijnen, H. R., and Hoylaerts, M. (1986) J Biol Chem 261(3), 1259-1266

34. Wolf, B. B., Vasudevan, J., Henkin, J., and Gonias, S. L. (1993) J Biol Chem 268(22), 16327-16331

35. Stack, M. S., and Johnson, D. A. (1994) J Biol Chem 269(13), 9416-9419

36. Yasuda, S., Morokawa, N., Wong, G. W., Rossi, A., Madhusudhan, M. S., Sali, A., Askew, Y. S., Adachi, R., Silverman, G. A., Krilis, S. A., and Stevens, R. L. (2005) Blood 105(10), 3893-3901

37. Brunner, G., Vettel, U., Jobstmann, S., Kramer, M. D., and Schirrmacher, V. (1992) Blood 79(8), 2099-2106

38. Ichinose, A., Fujikawa, K., and Suyama, T. (1986) J Biol Chem 261(8), 3486-3489

39. List, K., Jensen, O. N., Bugge, T. H., Lund, L. R., Ploug, M., Dano, K., and Behrendt, N. (2000) Biochemistry 39(3), 508-515

40. Goretzki, L., Schmitt, M., Mann, K., Calvete, J., Chucholowski, N., Kramer, M., Gunzler, W. A., Janicke, F., and Graeff, H. (1992) FEBS Lett 297(1-2), 112-118

41. Kobayashi, H., Schmitt, M., Goretzki, L., Chucholowski, N., Calvete, J., Kramer, M., Gunzler, W. A., Janicke, F., and Graeff, H. (1991) J Biol Chem 266(8), 5147-5152

42. Nauland, U., and Rijken, D. C. (1994) Eur J Biochem 223(2), 497-501

43. Andreasen, P. A., Kjoller, L., Christensen, L., and Duffy, M. J. (1997) Int J Cancer 72(1), 1-22

44. Dano, K., Behrendt, N., Hoyer-Hansen, G., Johnsen, M., Lund, L. R., Ploug, M., and Romer, J. (2005) Thromb Haemost 93(4), 676-681

45. Helenius, M. A., Saramaki, O. R., Linja, M. J., Tammela, T. L., and Visakorpi, T. (2001) Cancer Res 61(14), 5340-5344

46. Knudsen, B. S., and Edlund, M. (2004) Adv Cancer Res 91, 31-67

47. Sheng, S. (2001) Cancer Metastasis Rev 20(3-4), 287-296

48. Van Veldhuizen, P. J., Sadasivan, R., Cherian, R., and Wyatt, A. (1996) Am J Med Sci 312(1), 8-11

49. Ried, S., Jager, C., Jeffers, M., Vande Woude, G. F., Graeff, H., Schmitt, M., and Lengyel, E. (1999) J Biol Chem 274(23), 16377-16386

50. Hall, C. L., Tsan, R., Mugnai, G., Mazar, A., Radinsky, R., and Pettaway, C. A. (2004) Prostate 59(2), 167-176

51. Jin, J. S., Hsieh, D. S., Loh, S. H., Chen, A., Yao, C. W., and Yen, C. Y. (2006) Mod Pathol 19(3), 447-452

52. Johnson, M. D., Oberst, M. D., Lin, C. Y., and Dickson, R. B. (2003) Expert Rev Mol Diagn 3(3), 331-338

53. Oberst, M. D., Johnson, M. D., Dickson, R. B., Lin, C. Y., Singh, B., Stewart, M., Williams, A., al-Nafussi, A., Smyth, J. F., Gabra, H., and Sellar, G. C. (2002) Clin Cancer Res 8(4), 1101-1107

54. Riddick, A. C., Shukla, C. J., Pennington, C. J., Bass, R., Nuttall, R. K., Hogan, A., Sethia, K. K., Ellis, V., Collins, A. T., Maitland, N. J., Ball, R. Y., and Edwards, D. R. (2005) Br J Cancer 92(12), 2171-2180

55. Saleem, M., Adhami, V. M., Zhong, W., Longley, B. J., Lin, C. Y., Dickson, R. B., Reagan-Shaw, S., Jarrard, D. F., and Mukhtar, H. (2006) Cancer Epidemiol Biomarkers Prev 15(2), 217-227

56. Lin, C. Y., Anders, J., Johnson, M., and Dickson, R. B. (1999) J Biol Chem 274(26), 18237-18242

57. List, K., Szabo, R., Molinolo, A., Sriuranpong, V., Redeye, V., Murdock, T., Burke, B., Nielsen, B. S., Gutkind, J. S., and Bugge, T. H. (2005) Genes Dev 19(16), 1934-1950

58. List, K., Haudenschild, C. C., Szabo, R., Chen, W., Wahl, S. M., Swaim, W., Engelholm, L. H., Behrendt, N., and Bugge, T. H. (2002) Oncogene 21(23), 3765-3779

59. Huai, Q., Mazar, A. P., Kuo, A., Parry, G. C., Shaw, D. E., Callahan, J., Li, Y., Yuan, C., Bian, C., Chen, L., Furie, B., Furie, B. C., Cines, D. B., and Huang, M. (2006) Science 311(5761), 656-659

60. Petersen, L. C., Lund, L. R., Nielsen, L. S., Dano, K., and Skriver, L. (1988) J Biol Chem 263(23), 11189-11195

## Claims

1. An antagonist molecule that inhibits hepsin activation of pro-urokinase type plasminogen activator (pro-uPA) for use in a method of treating cancer in a subject, wherein the antagonist molecule comprises:

   (i) a polypeptide comprising a Kunitz domain (KD) sequence;
   (ii) at least a portion of human hepatocyte growth factor activator inhibitor-1, - 1 B, or -2 (HAI-1, HAI-1B or HAI-2);
   (iii) an amino acid sequence having at least 60%, 70%, 80%, 90%, 95% or 99% sequence identity to pro-uPA and which is capable of binding hepsin and which lacks a hepsin cleavage site.

2. The antagonist molecule as defined in claim 1 for use according to claim 1, wherein the Kunitz domain (KD) sequence comprises a Kunitz domain 1 sequence (KD-1).

3. The antagonist molecule as defined in claim 1 for use according to claim 1, wherein the portion of human hepatocyte growth factor activator inhibitor-1, -1 B, or -2 (HAI-1, HAI-1B or HAI-2) comprises a Kunitz domain (KD) sequence.

4. The antagonist molecule as defined in claim 1 for use according to claim 3, wherein the Kunitz domain (KD) sequence is:

   (i) Kunitz domain 1 sequence (KD-1) of human hepatocyte growth factor activator inhibitor-1, -1 B (HAI-1, HAI-1B), or variant thereof; or
   (ii) one or both of the Kunitz domains of human hepatocyte growth factor activator inhibitor-2 (HAI-2), or variant thereof.

5. The antagonist molecule as defined in claim 1 for use according to claim 1, wherein a hepsin cleavage site is pro-uPA $Lys_{158}$-$Ile_{159}$ peptide link.

6. The antagonist molecule as defined in claim 1 for use according to claim 1 or 5, wherein the amino acid sequence having at least 60%, 70%, 80%, 90%, 95% or 99% sequence identity to pro-uPA and which is capable of binding hepsin and which lacks a hepsin cleavage site lacks uPA activity.

7. The antagonist molecule as defined in claim 1 for use according to claim 6, wherein the molecule is a mutant wherein the $\beta$ chain is mutated or is devoid of at least a portion of uPA sequence associated with activity.

8. The antagonist molecule as defined in claim 1 for use according to any one of claims 1 to 7, wherein the molecule is linked to a toxin.

9. The antagonist molecule as defined in claim 1 for use according to claim 8, wherein the toxin is a cytotoxic agent.

10. The antagonist molecule as defined in claim 1 for use according to any one of claims 1 to 9, wherein the method further comprises administering radiation or a chemotherapeutic agent.

11. The antagonist molecule as defined in claim 1 for use according to any one of claims 1 to 10, wherein the cancer is prostate or ovarian.

12. A composition comprising a molecule as defined in any one of claims 1 to 9 and a carrier, for use in treatment as defined in any one of claims 1 or 11.

13. The composition for use according to claim 12, wherein the carrier is a pharmaceutically acceptable carrier.

## Patentansprüche

1. Antagonistenmolekül, das Hepsinaktivierung eines Plasminogenaktivators vom Prourokinasetyp (pro-uPA) hemmt, zur Verwendung in einem Verfahren zur Behandlung von Krebs bei einem Individuum, wobei das Antagonistenmolekül Folgendes umfasst:

   (i) ein Polypeptid, das eine Kunitz-Domänen- (KD-) Sequenz umfasst;

(ii) zumindest einen Abschnitt von menschlichem Hepatozyten-Wachstumsfaktor-Aktivator-Inhitior-1, -1 B oder -2 (HAI-1, HAI-1B oder HAI-2);

(iii) eine Aminosäuresequenz mit zumindest 60 %, 70 %, 80 %, 90 %, 95 % oder 99 % Sequenzidentität mit pro-uPA, die in der Lage ist, Hepsin zu binden, und keine Hepsin-Spaltstelle aufweist.

2. Antagonistenmolekül wie in Anspruch 1 definiert zur Verwendung nach Anspruch 1, wobei die Kunitz-Domänen-(KD-) Sequenz eine Kunitz-Domäne-1-Sequenz (KD-1) umfasst.

3. Antagonistenmolekül wie in Anspruch 1 definiert zur Verwendung nach Anspruch 1, wobei der Abschnitt von menschlichem Hepatozyten-Wachstumsfaktor-Aktivator-Inhitior-1, -1 B oder -2 (HAI-1, HAI-1 B oder HAI-2) eine Kunitz-Domänen-(KD-) Sequenz umfasst.

4. Antagonistenmolekül wie in Anspruch 1 definiert zur Verwendung nach Anspruch 3, wobei die Kunitz-Domänen-(KD-) Sequenz Folgendes ist:

(i) eine Kunitz-Domäne-1-Sequenz (KD-1) von menschlichem Hepatozyten-Wachstumsfaktor-Aktivator-Inhitior-1, -1 B (HAI-1, HAI-1 B) oder eine Variante davon; oder

(ii) eine oder beide der Kunitz-Domänen von menschlichem Hepatozyten-Wachstumsfaktor-Aktivator-Inhitior-2 (HAI-2) oder eine Variante davon.

5. Antagonistenmolekül wie in Anspruch 1 definiert zur Verwendung nach Anspruch 1, wobei eine Hepsin-Spaltstelle eine pro-uPA-Lys$_{158}$-Ile$_{159}$-Peptidbindung ist.

6. Antagonistenmolekül wie in Anspruch 1 definiert zur Verwendung nach Anspruch 1 oder 5, wobei die Aminosäuresequenz zumindest 60 %, 70 %, 80 %, 90 %, 95 % oder 99 % Sequenzidentität mit pro-uPA aufweist und in der Lage ist, Hepsin zu binden, und keine Hepsin-Spaltstelle aufweist,

7. Antagonistenmolekül wie in Anspruch 1 definiert zur Verwendung nach Anspruch 6, wobei das Molekül eine Mutante ist, worin die β-Kette mutiert ist oder dieser zumindest ein Abschnitt einer uPA-Sequenz fehlt, die mit Aktivität assoziiert ist.

8. Antagonistenmolekül wie in Anspruch 1 definiert zur Verwendung nach einem der Ansprüche 1 bis 7, wobei das Molekül an ein Toxin gebunden ist.

9. Antagonistenmolekül wie in Anspruch 1 definiert zur Verwendung nach Anspruch 8, wobei das Toxin ein zytotoxisches Mittel ist.

10. Antagonistenmolekül wie in Anspruch 1 definiert zur Verwendung nach einem der Ansprüche 1 bis 9, wobei das Verfahren weiters die Verabreichung von Strahlung oder eines Chemotherapeutikums umfasst.

11. Antagonistenmolekül wie in Anspruch 1 definiert zur Verwendung nach einem der Ansprüche 1 bis 10, wobei der Krebs Prostata- oder Ovarialkrebs ist.

12. Zusammensetzung, die ein Molekül wie in einem der Ansprüche 1 bis 9 definiert und einen Träger umfasst, zur Verwendung bei einer Behandlung wie in einem der Ansprüche 1 oder 11 definiert.

13. Zusammensetzung zur Verwendung nach Anspruch 12, wobei der Träger ein pharmazeutisch annehmbarer Träger ist.

**Revendications**

1. Molécule antagoniste qui inhibe l'activation par l'hepsine de l'activateur du plasminogène de type pro-urokinase (pro-uPA) pour une utilisation dans un procédé de traitement du cancer chez un sujet, ladite molécule antagoniste comprenant :

(i) un polypeptide comprenant une séquence de domaine de Kunitz (KD) ;
(ii) au moins une portion de l'inhibiteur-1, -1B ou -2 d'activateur du facteur de croissance des hépatocytes humains (HAI-1, HAI-1B ou HAI-2) ;

(iii) une séquence d'amino-acides ayant une identité de séquence d'au moins 60 %, 70 %, 80 %, 90 %, 95 % ou 99 % avec le pro-uPA et qui est capable de se lier à l'hepsine et qui est dépourvue de sites de clivage d'hepsine.

2. Molécule antagoniste telle que définie dans la revendication 1 pour une utilisation suivant la revendication 1, dans laquelle la séquence de domaine de Kunitz (KD) comprend une séquence de domaine de Kunitz 1 (KD-1).

3. Molécule antagoniste telle que définie dans la revendication 1 pour une utilisation suivant la revendication 1, dans laquelle la portion de l'inhibiteur -1, -1B ou -2 d'activateur du facteur de croissance des hépatocytes humains (HAI-1, HAI-1B ou HAI-2) comprend une séquence de domaine de Kunitz (KD).

4. Molécule antagoniste telle que définie dans la revendication 1 pour une utilisation suivant la revendication 3, dans laquelle la séquence du domaine de Kunitz (KD) est :

(i) la séquence du domaine de Kunitz 1 (KD-1) de l'inhibiteur 1 ou -1B d'activateur du facteur de croissance des hépatocytes humains (HAI-1, HAI-1B), ou son variant ; ou
(ii) l'un des ou les deux domaines de Kunitz de l'inhibiteur 2 d'activateur de facteur de croissance des hépatocytes humains (HAI-2), ou leurs variants.

5. Molécule antagoniste telle que définie dans la revendication 1 pour une utilisation suivant la revendication 1, dans laquelle un site de clivage d'hepsine est le segment peptidique de liaison $Lys_{158}$-$Ile_{159}$ de pro-uPA.

6. Molécule antagoniste telle que définie dans la revendication 1 pour une utilisation suivant la revendication 1 ou 5, dans laquelle la séquence d'amino-acides ayant une identité de séquence d'au moins 60 %, 70 %, 80 %, 90 %, 95 % ou 99 % avec le pro-uPA et qui est capable de se lier à l'hepsine et qui est dépourvue de site de clivage d'hepsine est dépourvue d'activité de uPA.

7. Molécule antagoniste telle que définie dans la revendication 1 pour une utilisation suivant la revendication 6, ladite molécule étant un mutant dans lequel la chaîne β est mutée ou est dépourvue d'au moins une portion de la séquence de uPA associée à l'activité.

8. Molécule antagoniste telle que définie dans la revendication 1 pour une utilisation suivant l'une quelconque des revendications 1 à 7, ladite molécule étant liée à une toxine.

9. Molécule antagoniste telle que définie dans la revendication 1 pour une utilisation suivant la revendication 8, la toxine étant un agent cytotoxique.

10. Molécule antagoniste telle que définie dans la revendication 1 pour une utilisation suivant l'une quelconque des revendications 1 à 9, le procédé comprenant en outre l'administration d'un rayonnement ou d'un agent chimiothérapeutique.

11. Molécule antagoniste telle que définie dans la revendication 1 pour une utilisation suivant l'une quelconque des revendications 1 à 10, le cancer étant le cancer de la prostate ou le cancer des ovaires.

12. Composition comprenant une molécule telle que définie dans l'une quelconque des revendications 1 à 9 et un support, pour une utilisation dans un traitement tel que défini dans l'une quelconque des revendications 1 et 11.

13. Composition pour une utilisation suivant la revendication 12, dans laquelle le support est un support pharmaceutiquement acceptable.

*FIG. 1A*

*FIG. 1B*

FIG. 2A

FIG. 2B

**FIG. 3**

**FIG. 4A**

*FIG. 4B*

*FIG. 5B*

FIG. 5A

FIG. 6

MAQKEGGRTVPCCSRPKVAALTAGTLLLLTAIGAASWAIVAVLLRSDQEPLYPVQVSSAD
ARLMVFDKTEGTWRLLCSSRSNARVAGLSCEEMGFLRALTHSELDVRTAGANGTSGFFCV
DEGRLPHTQRLLEVISVCDCPRGRFLAAICQDCGRRKLPVDRIVGGRDTSLGRWPWQVSL
RYDGAHLCGGSLLSGDWVLTAAHCFPERNRVLSRWRVFAGAVAQASPHGLQLGVQAVVYH
GGYLPFRDPNSEENSNDIALVHLSSPLPLTEYIQPVCLPAAGQALVDGKICTVTGWGNTQ
YYGQQAGVLQEARVPIISNDVCNGADFYGNQIKPKMFCAGYPEGGIDACQGDSGGPFVCE
DSISRTPRWRLCGIVSWGTGCALAQKPGVYTKVSDFREWIFQAIKTHSEASGMVTQL

FIG. 7

```
Met Ala Gln Lys Glu Gly Gly Arg Thr Val Pro Cys Cys Ser Arg Pro
1               5               10              15
Lys Val Ala Ala Leu Thr Ala Gly Thr Leu Leu Leu Leu Thr Ala Ile
            20              25              30
Gly Ala Ala Ser Trp Ala Ile Val Ala Val Leu Leu Arg Ser Asp Gln
        35              40              45
Glu Pro Leu Tyr Pro Val Gln Val Ser Ser Ala Asp Ala Arg Leu Met
    50              55              60
Val Phe Asp Lys Thr Glu Gly Thr Trp Arg Leu Leu Cys Ser Ser Arg
65              70              75              80
Ser Asn Ala Arg Val Ala Gly Leu Ser Cys Glu Glu Met Gly Phe Leu
            85              90              95
Arg Ala Leu Thr His Ser Glu Leu Asp Val Arg Thr Ala Gly Ala Asn
        100             105             110
Gly Thr Ser Gly Phe Phe Cys Val Asp Glu Gly Arg Leu Pro His Thr
        115             120             125
Gln Arg Leu Leu Glu Val Ile Ser Val Cys Asp Cys Pro Arg Gly Arg
    130             135             140
Phe Leu Ala Ala Ile Cys Gln Gly Glu Ile Leu Lys Leu Arg Thr Leu
145             150             155             160
Ser Phe Arg Pro Leu Gly Arg Pro Arg Pro Leu Lys Leu Pro Arg Met
            165             170             175
Gly Pro Cys Thr Phe Arg Pro Pro Arg Ala Gly Pro Ser Leu Gly Ser
        180             185             190
Gly Asp Leu Gly Ser Ser Pro Leu Ser Pro Pro Ala Asp Pro Cys
        195             200             205
Pro Thr Asp Cys Gly Arg Arg Lys Leu Pro Val Asp Arg Ile Val Gly
    210             215             220
Gly Arg Asp Thr Ser Leu Gly Arg Trp Pro Trp Gln Val Ser Leu Arg
225             230             235             240
```

FIG. 8A

61

Tyr Asp Gly Ala His Leu Cys Gly Gly Ser Leu Leu Ser Gly Asp Trp
                    245                 250                 255
Val Leu Thr Ala Ala His Cys Phe Pro Glu Arg Asn Arg Val Leu Ser
                260                 265                 270
Arg Trp Arg Val Phe Ala Gly Ala Val Ala Gln Ala Ser Pro His Gly
        275                 280                 285
Leu Gln Leu Gly Val Gln Ala Val Val Tyr His Gly Gly Tyr Leu Pro
    290                 295                 300
Phe Arg Asp Pro Asn Ser Glu Glu Asn Ser Asn Asp Ile Ala Leu Val
305                 310                 315                 320
His Leu Ser Ser Pro Leu Pro Leu Thr Glu Tyr Ile Gln Pro Val Cys
                325                 330                 335
Leu Pro Ala Ala Gly Gln Ala Leu Val Asp Gly Lys Ile Cys Thr Val
        340                 345                 350
Thr Gly Trp Gly Asn Thr Gln Tyr Tyr Gly Gln Gln Ala Gly Val Leu
        355                 360                 365
Gln Glu Ala Arg Val Pro Ile Ile Ser Asn Asp Val Cys Asn Gly Ala
    370                 375                 380
Asp Phe Tyr Gly Asn Gln Ile Lys Pro Lys Met Phe Cys Ala Gly Tyr
385                 390                 395                 400
Pro Glu Gly Gly Ile Asp Ala Cys Gln Gly Asp Ser Gly Gly Pro Phe
                405                 410                 415
Val Cys Glu Asp Ser Ile Ser Arg Thr Pro Arg Trp Arg Leu Cys Gly
                420                 425                 430
Ile Val Ser Trp Gly Thr Gly Cys Ala Leu Ala Gln Lys Pro Gly Val
        435                 440                 445
Tyr Thr Lys Val Ser Asp Phe Arg Glu Trp Ile Phe Gln Ala Ile Lys
    450                 455                 460
Thr His Ser Glu Ala Ser Gly Met Val Thr Gln Leu
465                 470                 475

FIG. 8B

MRALLARLLLCVLVVSDSKGSNELHQVPSNCDCLNGGTCVSNKYFSNIH
WCNCPKKFGGQHCEIDKSKTCYEGNGHFYRGKASTDTMGRPCLPWNSAT
VLQQTYHAHRSDALQLGLGKHNYCRNPDNRRRPWCYVQVGLKPLVQECM
VHDCADGKKPSSPPEELKFQCGQKTLRPRFKIIGGEFTTIENQPWFAAI
YRRHRGGSVTYVCGGSLISPCWVISATHCFIDYPKKEDYIVYLGRSRLN
SNTQGEMKFEVENLILHKDYSADTLAHHNDIALLKIRSKEGRCAQPSRT
IQTICLPSMYNDPQFGTSCEITGFGKENSTDYLYPEQLKMTVVKLISHR
ECQQPHYYGSEVTTKMLCAADPQWKTDSCQGDSGGPLVCSLQGRMTLTG
IVSWGRGCALKDKPGVYTRVSHFLPWIRSHTKEENGLAL

## FIG. 9

## FIG. 10

## REFERENCES CITED IN THE DESCRIPTION

### Patent documents cited in the description

- US 60805584 B
- WO 200162271 A
- WO 2006014928 A
- WO 0157194 A
- US 2004009911 A
- US 6828146 B
- US 4816567 A
- US 5545806 A
- US 5569825 A
- US 5591669 A
- US 5545807 A
- WO 9717852 A
- US 5565332 A
- US 5573905 A
- US 5567610 A
- US 5229275 A
- US 5869046 A
- WO 9316185 A
- US 5571894 A
- US 5587458 A
- US 5641870 A
- WO 9616673 A
- US 5837234 A
- WO 9802463 A
- US 5821337 A
- WO 9308829 A
- WO 9404690 A
- US 5731168 A
- US 4676980 A
- WO 9100360 A
- WO 92200373 A
- EP 03089 A
- US 5739277 A
- US 4975278 A
- EP 1391213 A
- US 4970198 A
- US 5079233 A
- US 5585089 A
- US 5606040 A
- US 5693762 A
- US 5739116 A
- US 5767285 A
- US 5773001 A
- WO 9411026 A
- US 3896111 A
- US 4151042 A
- US 4137230 A
- US 4248870 A
- US 4256746 A
- US 4260608 A
- US 4265814 A
- US 4294757 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5208020 A
- US 5416064 A
- EP 0425235 B1
- US 5712374 A
- US 5714586 A
- US 5770701 A
- US 5770710 A
- US 5877296 A
- US 5053394 A
- WO 9321232 A
- US 5362852 A
- US 4485045 A
- US 4544545 A
- WO 9738731 A
- US 5013556 A
- US 5556762 A
- US 5750373 A
- US 4708871 A
- US 4833092 A
- US 5223409 A
- US 5403484 A
- US 5571689 A
- US 5663143 A
- WO 8403506 A
- WO 8403564 A
- WO 9534683 A
- US 5627024 A
- US 5766905 A
- WO 9814277 A
- WO 9820169 A
- WO 9820159 A

- WO 9820036 A
- WO 9735196 A
- WO 9746251 A
- WO 9747314 A
- WO 9709446 A
- US 5498538 A
- US 5432018 A
- WO 9815833 A
- US 5723286 A
- US 5580717 A
- US 5427908 A
- US 5498530 A
- US 5770434 A
- US 5734018 A
- US 5698426 A
- US 5763192 A
- US 5723323 A
- WO 0000823 A
- WO 0039585 A
- WO 8101145 A
- WO 8807378 A
- US 5364934 A
- WO 8705330 A
- US 4640835 A
- US 4496689 A
- US 4301144 A
- US 4670417 A
- US 4791192 A
- US 4179337 A
- US 5428130 A
- WO 8905859 A
- US 4399216 A
- DD 266710
- US 4946783 A
- US 5648237 A, Carter
- US 5789199 A, Joly
- US 5840523 A, Simmons
- EP 139383 A
- US 4943529 A
- EP 402226 A
- EP 183070 A
- EP 244234 A
- EP 394538 A
- WO 9100357 A
- US 5010182 A
- EP 362179 A
- WO 9013646 A
- EP 36776 A
- EP 73657 A
- GB 2211504 A
- EP 117060 A
- EP 117058 A
- US 4767704 A
- US 4657866 A
- US 4927762 A
- US 4560655 A
- US 5122469 A
- WO 9003430 A
- WO 8700195 A
- US RE30985 E
- US 3773919 A
- EP 0600517 A
- EP 616812 A
- WO 9607321 A
- US 4892538 A
- US 5283187 A
- WO 9325673 A
- WO 9106629 A
- US 3687808 A
- US 4469863 A
- US 4476301 A
- US 5023243 A
- US 5177196 A
- US 5188897 A
- US 5264423 A
- US 5276019 A
- US 5278302 A
- US 5286717 A
- US 5321131 A
- US 5399676 A
- US 5405939 A
- US 5453496 A
- US 5455233 A
- US 5466677 A
- US 5476925 A
- US 5519126 A
- US 5536821 A
- US 5541306 A
- US 5550111 A
- US 5563253 A
- US 5571799 A
- US 5587361 A
- US 5194599 A
- US 5565555 A
- US 5527899 A
- US 5721218 A
- US 5672697 A
- US 5625050 A
- US 5034506 A
- US 5166315 A
- US 5185444 A
- US 5214134 A
- US 5216141 A
- US 5235033 A
- US 5264562 A
- US 5264564 A
- US 5405938 A
- US 5434257 A
- US 5470967 A
- US 5489677 A
- US 5541307 A
- US 5561225 A
- US 5596086 A
- US 5602240 A
- US 5610289 A
- US 5608046 A

- US 5618704 A
- US 5623070 A
- US 5663312 A
- US 5633360 A
- US 5677437 A
- US 5792608 A
- US 5646269 A
- US 5677439 A
- US 5539082 A
- US 5714331 A
- US 5719262 A
- WO 9839352 A
- WO 9914226 A
- US 4981957 A
- US 5118800 A
- US 5319080 A
- US 5359044 A
- US 5393878 A
- US 5446137 A
- US 5466786 A
- US 5514785 A
- US 5519134 A
- US 5567811 A
- US 5576427 A
- US 5591722 A
- US 5597909 A
- US 5610300 A
- US 5627053 A
- US 5639873 A
- US 5646265 A
- US 5658873 A
- US 5670633 A
- US 5792747 A
- US 5700920 A
- US 4845205 A
- US 5130302 A
- US 5134066 A
- US 5175273 A
- US 5367066 A
- US 5432272 A
- US 5457187 A
- US 5459255 A
- US 5484908 A
- US 5502177 A
- US 5525711 A
- US 5552540 A
- US 5587469 A
- US 5594121 A
- US 5596091 A
- US 5614617 A
- US 5645985 A
- US 5830653 A
- US 5763588 A
- US 6005096 A
- US 5681941 A
- US 5750692 A
- US 33413099 A
- US 4828979 A
- US 4948882 A
- US 5218105 A
- US 5525465 A
- US 5541313 A
- US 5545730 A
- US 5552538 A
- US 5578717 A
- US 5580731 A
- US 5591584 A
- US 5109124 A
- US 5118802 A
- US 5138045 A
- US 5414077 A
- US 5486603 A
- US 5512439 A
- US 5578718 A
- US 4587044 A
- US 4605735 A
- US 4667025 A
- US 4762779 A
- US 4789737 A
- US 4824941 A
- US 4835263 A
- US 4876335 A
- US 4904582 A
- US 4958013 A
- US 5082830 A
- US 5112963 A
- US 5214136 A
- US 5245022 A
- US 5254469 A
- US 5258506 A
- US 5262536 A
- US 5272250 A
- US 5292873 A
- US 5317098 A
- US 5371241 A
- US 5391723 A
- US 5416203 A
- US 5451463 A
- US 5510475 A
- US 5512667 A
- US 5565552 A
- US 5567810 A
- US 5574142 A
- US 5585481 A
- US 5587371 A
- US 5595726 A
- US 5597696 A
- US 5599923 A
- US 5599928 A
- US 5688941 A
- US 5013830 A
- US 5149797 A
- US 5220007 A
- US 5256775 A
- US 5366878 A
- US 5403711 A

- US 5491133 A
- US 5565350 A
- US 5623065 A
- US 5652355 A
- US 5652356 A
- US 5700922 A
- US 5108921 A
- US 5354844 A
- US 5416016 A
- US 5459127 A
- US 5521291 A
- US 5543158 A
- US 5547932 A
- US 5583020 A
- US 5591721 A
- US 4426330 A
- US 4534899 A
- US 5213804 A
- US 5227170 A
- US 5264221 A

- US 5356633 A
- US 5395619 A
- US 5417978 A
- US 5462854 A
- US 5469854 A
- US 5512295 A
- US 5527528 A
- US 5534259 A
- US 5543152 A
- US 5556948 A
- US 5580575 A
- US 5595756 A
- WO 9010048 A
- WO 9013641 A
- WO 9104753 A
- WO 9010448 A
- US 4736866 A
- US 4870009 A
- WO 9733551 A
- US 6472393 B

**Non-patent literature cited in the description**

- **SAMBROOK et al.** Molecular Cloning: A Laboratory Manual. 1989
- Oligonucleotide Synthesis. 1984
- Animal Cell Culture. 1987
- Methods in Enzymology. Academic Press, Inc,
- Current Protocols in Molecular Biology. 1987
- PCR: The Polymerase Chain Reaction. 1994
- **PERBAL BERNARD V.** A Practical Guide to Molecular Cloning. 1988
- **BARBAS et al.** Phage Display: A Laboratory Manual. 2001
- **MORRISON et al.** *Proc. Natl. Acad. Sci. USA,* 1984, vol. 81, 6851-6855
- **JONES et al.** *Nature,* 1986, vol. 321, 522-525
- **RIECHMANN et al.** *Nature,* 1988, vol. 332, 323-329
- **PRESTA.** *Curr. Op. Struct. Biol.,* 1992, vol. 2, 593-596
- **VASWANI ; HAMILTON.** *Ann. Allergy, Asthma & Immunol.,* 1998, vol. 1, 105-1.15
- **HARRIS.** *Biochem. Soc. Transactions,* 1995, vol. 23, 1035-1038
- **HURLE ; GROSS.** *Curr. Op. Biotech.,* 1994, vol. 5, 428-433
- **MARKS et al.** *Bio/Technology,* 1992, vol. 10, 779-783
- **BARBAS et al.** *Proc Nat. Acad. Sci, USA,* 1994, vol. 91, 3809-3813
- **SCHIER et al.** *Gene,* 1995, vol. 169, 147-155
- **YELTON et al.** *J. Immunol,* 1995, vol. 155, 1994-2004

- **JACKSON et al.** *J. Immunol.,* 1995, vol. 154 (7), 3310-9
- **HAWKINS et al.** *J. Mol. Biol.,* 1992, vol. 226, 889-896
- **AGNEW.** *Chem Intl. Ed. Engl.,* 1994, vol. 33, 183-186
- Cell cycle regulation, oncogenes, and antineoplastic drugs. **MURAKAMI et al.** The Molecular Basis of Cancer. WB Saunders, 1995, 13
- **KOHLER et al.** *Nature,* 1975, vol. 256, 495
- **GODING.** Monoclonal Antibodies: Principles and Practice. Academic Press, 1986, 59-103
- **KOZBOR.** *J. Immunol.,* 1984, vol. 133, 3001
- **BRODEUR et al.** Monoclonal Antibody Production Techniques and Applications. Marcel Dekker, Inc, 1987, 51-63
- **MUNSON et al.** *Anal. Biochem.,* 1980, vol. 107, 220
- **SKERRA et al.** *Curr. Opinion in Immunol.,* 1993, vol. 5, 256-262
- **PLÜCKTHUN.** *Immunol. Revs.,* 1992, vol. 130, 151-188
- **MCCAFFERTY et al.** *Nature,* 1990, vol. 348, 552-554
- **CLACKSON et al.** *Nature,* 1991, vol. 352, 624-628
- **MARKS et al.** *J. Mol. Biol.,* 1991, vol. 222, 581-597
- **WATERHOUSE et al.** *Nuc. Acids. Res.,* 1993, vol. 21, 2265-2266
- **MORRISON et al.** *Proc. Natl Acad. Sci. USA,* 1984, vol. 81, 6851

- **RIECHMANN et al.** *Nature,* 1988, vol. 332, 323-327
- **VERHOEYEN et al.** *Science,* 1988, vol. 239, 1534-1536
- **SIMS et al.** *J. Immunol.,* 1993, vol. 151, 2296
- **CHOTHIA et al.** *J. Mol. Biol.,* 1987, vol. 196, 901
- **CARTER et al.** *Proc. Natl. Acad. Sci. USA,* 1992, vol. 89, 4285
- **PRESTA et al.** *J. Immunol.,* 1993, vol. 151, 2623
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immuno.,* 1993, vol. 7, 33
- **MCCAFFERTY et al.** *Nature,* 1990, vol. 348, 552-553
- **JOHNSON, KEVIN S. ; CHISWELL, DAVID J.** *Current Opinion in Structural Biology,* 1993, vol. 3, 564-571
- **GRIFFITH et al.** *EMBO J.,* 1993, vol. 12, 725-734
- **MORIMOTO et al.** *Journal of Biochemical and Biophysical Methods,* 1992, vol. 24, 107-117
- **BRENNAN et al.** *Science,* 1985, vol. 229, 81
- **CARTER et al.** *Bio/Technology,* 1992, vol. 10, 163-167
- **MILLSTEIN et al.** *Nature,* 1983, vol. 305, 537-539
- **TRAUNECKER et al.** *EMBO J.,* 1991, vol. 10, 3655-3659
- **SURESH et al.** *Methods in Enzymology,* 1986, vol. 121, 210
- **SHALABY et al.** *J. Exp. Med,* 1992, vol. 175, 217-225
- **KOSTELNY et al.** *J. Immunol.,* 1992, vol. 148 (5), 1547-1553
- **HOLLINGER et al.** *Proc. Natl. Acad. Sci. USA,* 1993, vol. 90, 6444-6448
- **GRUBER et al.** *J. Immunol.,* 1994, vol. 152, 5368
- **TUTT et al.** *J. Immunol.,* 1991, vol. 147, 60
- **CARON et al.** *J. Exp Med.,* 1992, vol. 176, 1191-1195
- **SHOPES, B.** *J. Immunol.,* 1992, vol. 148, 2918-2922
- **WOLFF et al.** *Cancer Research,* 1993, vol. 53, 2560-2565
- **STEVENSON et al.** *Anti-Cancer Drug Design,* 1989, vol. 3, 219-230
- **SYRIGOS ; EPENETOS.** *Anticancer Research,* 1999, vol. 19, 605-614
- **NICULESCU-DUVAZ ; SPRINGER.** *Adv. Drg Del. Rev.,* 1997, vol. 26, 151-172
- **BALDWIN et al.** *Lancet,* 15 March 1986, 603-05
- Antibody Carriers Of Cytotoxic Agents In Cancer Therapy: A Review. **THORPE et al.** Monoclonal Antibodies '84: Biological And Clinical Applications. 1984, 475-506
- **ROWLAND et al.** *Cancer Immunol. Immunother.,* 1986, vol. 21, 183-87
- **MANDLER et al.** *Jour. of the Nat. Cancer Inst.,* 2000, vol. 92 (19), 1573-1581
- **MANDLER et al.** *Bioorganic & Med. Chem. Letters,* 2000, vol. 10, 1025-1028
- **MANDLER et al.** *Bioconjugate Chem.,* 2002, vol. 13, 786-791
- **LIU et al.** *Proc. Natl. Acad. Sci. USA,* 1996, vol. 93, 8618-8623
- **LODE et al.** *Cancer Res.,* 1998, vol. 58, 2928
- **HINMAN et al.** *Cancer Res.,* 1993, vol. 53, 3336-3342
- **WISEMAN et al.** *Eur. Jour. Nucl. Med.,* 2000, vol. 27 (7), 766-77
- **WISEMAN et al.** *Blood,* 2002, vol. 99 (12), 4336-42
- **WITZIG et al.** *J. Clin. Oncol.,* 2002, vol. 20 (10), 2453-63
- **WITZIG et al.** *J. Clin. Oncol.,* 2002, vol. 20 (15), 3262-69
- *Drugs of the Future,* 2000, vol. 25 (7), 686
- **DORONINA et al.** *Nature Biotechnology,* 2003, vol. 21 (7), 778-784
- **VITETTA et al.** *Science,* 1987, vol. 238, 1098
- **CHARI et al.** *Cancer Research,* 1992, vol. 52, 127-131
- **CARLSSON et al.** *Biochem. J.,* 1978, vol. 173, 723-737
- **HINMAN et al.** *Cancer Research,* 1993, vol. 53, 3336-3342
- **LODE et al.** *Cancer Research,* 1998, vol. 58, 2925-2928
- **FRAKER et al.** *Biochem. Biophys. Res. Commun.,* 1978, vol. 80, 49-57
- **CHATAL.** Monoclonal Antibodies in Immunoscintigraphy. CRC Press, 1989
- Applications Handbook and Catalog. 2003, 467-498
- **HERMANSON.** *Bioconjugate Techniques*
- **GEOGHEGAN ; STROH.** *Bioconjugate Chem.,* 1992, vol. 3, 138-146
- **EPSTEIN et al.** *Proc. Natl. Acad. Sci. USA,* 1985, vol. 82, 3688
- **HWANG et al.** *Proc. Natl Acad. Sci. USA,* 1980, vol. 77, 4030
- **MARTIN et al.** *J. Biol. Chem.,* 1982, vol. 257, 286-288
- **GABIZON et al.** *J. National Cancer Inst.,* 1989, vol. 81 (19), 1484

- **GEYSEN et al.** *Proc. Natl. Acad. Sci. U.S.A.,* 1984, vol. 81, 3998-4002
- **GEYSEN et al.** *Proc. Natl. Acad. Sci. U.S.A.,* 1985, vol. 82, 178-182
- **GEYSEN et al.** *Synthetic Peptides as Antigens,* 1986, 130-149
- **GEYSEN et al.** *J. Immunol. Meth.,* 1987, vol. 102, 259-274
- **SCHOOFS et al.** *J. Immunol,* 1988, vol. 140, 611-616
- **CWIRLA, S. E. et al.** *Proc. Natl. Acad. Sci. USA,* 1990, vol. 87, 6378
- **LOWMAN, H.B. et al.** *Biochemistry,* 1991, vol. 30, 10832
- **CLACKSON, T. et al.** *Nature,* 1991, vol. 352, 624
- **MARKS, J. D. et al.** *J. Mol. Biol.,* 1991, vol. 222, 581
- **KANG, A.S. et al.** *Proc. Natl. Acad. Sci. USA,* 1991, vol. 88, 8363
- **SMITH, G. P.** *Current Opin. Biotechnol.,* 1991, vol. 2, 668
- **SCOTT, J.K. ; SMITH, G. P.** *Science,* 1990, vol. 249, 386
- **REN et al.** *Gene,* 1998, vol. 215, 439
- **ZHU et al.** *Cancer Research,* 1998, vol. 58 (15), 3209-3214
- **JIANG et al.** *Infection & Immunity,* 1997, vol. 65 (11), 4770-4777
- **REN et al.** *Gene,* 1997, vol. 195 (2), 303-311
- **REN.** *Protein Sci.,* 1996, vol. 5, 1833
- **EFIMOV et al.** *Virus Genes,* 1995, vol. 10, 173
- **SMITH ; SCOTT.** *Methods in Enzymology,* 1993, vol. 217, 228-257
- **LI et al.** *Mol Biotech.,* 1998, vol. 9, 187
- **ANDREASEN et al.** *Int. J. Cancer,* 1997, vol. 72 (1), 1-22
- **DANO et al.** *Thromb. Haemost.,* 2005, vol. 93 (4), 676-681
- **HELENIUS et al.** *Cancer Res.,* 2001, vol. 61 (14), 5340-5344
- **KNUDSEN et al.** *Adv. Cancer Res.,* 2004, vol. 91, 31067
- **SHEN'G, S.** *Cancer Metastasis Rev.,* 2001, vol. 20 (3-4), 287-296
- **VAN VELDHUIZEN et al.** *Am. J. Med. Sci.,* 1996, vol. 312 (1), 8-11
- **ED HARLOW ; DAVID LANE.** Antibodies, A Laboratory Manual. Cold Spring Harbor Laboratory, 1988
- **MASSEY.** *Nature,* 1987, vol. 328, 457-458
- **NEUBERGER et al.** *Nature,* 1984, vol. 312, 604-608
- **A. L. LEHNINGER.** Biochemistry. Worth Publishers, 1975, 73-75
- **CARTER et al.** *Nucl. Acids Res.,* 1986, vol. 13, 4331
- **ZOLLER et al.** *Nucl. Acids Res.,* 1987, vol. 10, 6487
- **WELLS et al.** *Gene,* 1985, vol. 34, 315
- **WELLS et al.** *Philos. Trans. R. Soc. London SerA,* 1986, vol. 317, 415
- **CUNNINGHAM ; WELLS.** *Science,* 1989, vol. 244, 1081-1085
- **CREIGHTON.** The Proteins. W.H. Freeman & Co,
- **CHOTHIA.** *J. Mol. Biol.,* 1976, vol. 150, 1
- **T.E. CREIGHTON.** Proteins: Structure and Molecular Properties. W.H. Freeman & Co, 1983, 79-86
- **APLIN ; WRISTON.** *CRC Crit. Rev. Biochem.,* 1981, 259-306
- **HAKIMUDDIN et al.** *Arch. Biochem. Biophys,* 1987, vol. 259, 52
- **EDGE et al.** *Anal. Biochem.,* 1981, vol. 118, 131
- **THOTAKURA et al.** *Meth. Enzymol.,* 1987, vol. 138, 350
- Remington's Pharmaceutical Sciences. 1980
- **FIELD et al.** *Mol. Cell. Biol.,* 1988, vol. 8, 2159-2165
- **EVAN et al.** *Molecular and Cellular Biotogy,* 1985, vol. 5, 3610-3616
- **PABORSKY et al.** *Protein Engineering,* 1990, vol. 3 (6), 547-553
- **HOPP et al.** *BioTechnology,* 1988, vol. 6, 1204-1210
- **MARTIN et al.** *Science,* 1992, vol. 255, 192-194
- **SKINNER et al.** *J. Biol. Chem.,* 1991, vol. 266, 15163-15166
- **LUTZ-FREYERMUTH et al.** *Proc. Natl. Acad. Sci. USA,* 1990, vol. 87, 6393-6397
- **STEWART et al.** Solid-Phase Peptide Synthesis. W.H. Freeman Co, 1969
- **MERRIFIELD.** *J. Am. Chem. Soc.,* 1963, vol. 85, 2149-2154
- **SAMBROOK et al.** Molecular Cloning: A Laboratory Manual. Cold Spring Harbor Laboratory Press, 1989
- **DIEFFENBACH et al.** PCR Primer: A Laboratory Manual. Cold Spring Harbor Laboratory Press, 1995
- Mammalian Cell Biotechnology: a Practical Approach. IRL Press, 1991
- **SHAW et al.** *Gene,* 1983, vol. 23, 315
- **GRAHAM ; VAN DER EB.** *Virology,* 1978, vol. 52, 456-457
- **VAN SOLINGEN et al.** *J. Bact.,* 1977, vol. 130, 946
- **HSIAO et al.** *Proc. Natl. Acad. Sci. (USA),* 1979, vol. 76, 3829
- **KEOWN et al.** *Methods in Enzymology,* 1990, vol. 185, 527-537

- **MANSOUR et al.** *Nature,* 1988, vol. 336, 348-352
- **BEACH ; NURSE.** *Nature,* 1981, vol. 290, 140
- **FLEER et al.** *Bio/Technology,* 1991, vol. 9, 968-975
- **LOUVENCOURT et al.** *J. Bacteriol.,* 1983, vol. 154 (2), 737-742
- **VAN DEN BERG et al.** *Bio/Technology,* 1990, vol. 8, 135
- **SREEKRISHNA et al.** *J. Basic Microbiol.,* 1988, vol. 28, 265-278
- **CASE et al.** *Proc. Natl. Acad. Sci. USA,* 1979, vol. 76, 5259-5263
- **BALLANCE et al.** *Biochem. Biophys. Res. Commun.,* 1983, vol. 112, 284-289
- **TILBURN et al.** *Gene,* 1983, vol. 26, 205-221
- **YELTON et al.** *Proc. Natl. Acad. Sci. USA,* 1984, vol. 81, 1470-1474
- **KELLY ; HYNES.** *EMBO J.,* 1985, vol. 4, 475-479
- **C. ANTHONY.** *The Biochemistry of Methylotrophs,* 1982, 269
- **GRAHAM et al.** *J. Gen Virol.,* 1977, vol. 36, 59
- **URLAUB et al.** *Proc. Natl. Acad. Sci. USA,* 1980, vol. 77, 4216
- **MATHER.** *Biol. Reprod.,* 1980, vol. 23, 243-251
- **MATHER et al.** Annals N.Y. Acad. Sci. 1982, vol. 383, 44-68
- **STINCHCOMB et al.** *Nature,* 1979, vol. 282, 39
- **KINGSMAN et al.** *Gene,* 1979, vol. 7, 141
- **TSCHEMPER et al.** *Gene,* 1980, vol. 10, 157
- **JONES.** *Genetics,* 1977, vol. 85, 12
- **CHANG et al.** *Nature,* 1978, vol. 275, 615
- **GOEDDEL et al.** *Nature,* 1979, vol. 281, 544
- **GOEDDEL.** *Nucleic Acids Res.,* 1980, vol. 8, 4057
- **DEBOER et al.** *Proc. Natl. Acad. Sci. USA,* 1983, vol. 80, 21-25
- **HITZEMAN et al.** *J. Biol. Chem.,* 1980, vol. 255, 2073
- **HESS et al.** *J. Adv. Enzyme Reg.,* 1968, vol. 7, 149
- **HOLLAND.** *Biochemistry,* 1978, vol. 17, 4900
- **GETHING et al.** *Nature,* 1981, vol. 293, 620-625
- **MANTEI et al.** *Nature,* 1979, vol. 281, 40-46
- **HAM et al.** *Meth. Enz.,* 1979, vol. 58, 44
- **BARNES et al.** *Anal. Biochem,* 1980, vol. 102, 255
- **THOMAS.** *Proc. Natl. Acad. Sci. USA,* 1980, vol. 77, 5201-5205
- **DEUTSCHER.** *Methods in Enzymology,* 1990, 182
- **SCOPES.** Protein Purification: Principles and Practice. Springer-Verlag, 1982
- **LINDMARK et al.** *J. Immunol. Meth.,* 1983, vol. 62, 1-13
- **GUSS et al.** *EMBO J.,* 1966, vol. 5, 15671575
- Chemotherapy Service. Williams & Wilkins, 1992
- **ANDERSON et al.** *Science,* 1992, vol. 256, 808-813
- **STEIN ; COHEN.** *Cancer Res.,* 1988, vol. 48, 2659
- **VAN DER KROL et al.** *BioTechniques,* 1988, vol. 6, 958
- **NIELSEN et al.** *Science,* 1991, vol. 254, 1497-1500
- **MARTIN et al.** *Helv. Chim. Acta,* 1995, vol. 78, 486-504
- The Concise Encyclopedia Of Polymer Science And Engineering. John Wiley & Sons, 1990, 858-859
- **ENGLISCH et al.** *Angewandte Chemie, International Edition,* 1991, vol. 30, 613
- **SANGHVI et al.** Antisense Research and Applications. CRC Press, 1993, 276-278
- **LETSINGER et al.** *Proc. Natl. Acad. Sci. USA,* 1989, vol. 86, 6553-6556
- **MANOHARAN et al.** *Bioorg. Med. Chem. Let.,* 1994, vol. 4, 1053-1060
- **MANOHARAN et al.** *Ann. N.Y. Acad. Sci.,* 1992, vol. 660, 306-309
- **MANOHARAN et al.** *Bioorg. Med. Chem. Let.,* 1993, vol. 3, 2765-2770
- **OBERHAUSER et al.** *Nucl. Acids Res.,* 1992, vol. 20, 533-538
- **SAISON-BEHMOARAS et al.** *EMBO J.,* 1991, vol. 10, 1111-1118
- **KABANOV et al.** *FEBS Lett.,* 1990, vol. 259, 327-330
- **SVINARCHUK et al.** *Biochimie,* 1993, vol. 75, 49-54
- **MANOHARAN et al.** *Tetrahedron Lett.,* 1995, vol. 36, 3651-3654
- **SHEA et al.** *Nucl. Acids Res.,* 1990, vol. 18, 3777-3783
- **MANOHARAN et al.** *Nucleosides & Nucleotides,* 1995, vol. 14, 969-973
- **MISHRA et al.** *Biochim. Biophys. Acta,* 1995, vol. 1264, 229-237
- **THOMAS ; CAPECCHI.** *Cell,* 1987, vol. 51, 503
- **LI et al.** *Cell,* 1992, vol. 69, 915
- **BRADLEY.** Teratocarcinomas and Embryonic Stem Cells: A Practical Approach. IRL, 1987, 113-152
- **ZAMECNIK et al.** *Proc. Natl. Acad. Sci. USA,* 1986, vol. 83, 4143-4146
- **DZAU et al.** *Trends in Biotechnology,* 1993, vol. 11, 205-210
- **WU et al.** *J. Biol. Chem.,* 1987, vol. 262, 4429-4432

- **WAGNER et al.** *Proc. Natl. Acad. Sci. USA,* 1990, vol. 87, 3410-3414
- **FIELDS ; SONG.** *Nature,* 1989, vol. 340, 245-246
- **CHIEN et al.** *Proc. Natl. Acad. Sci. USA,* 1991, vol. 88, 9578-9582
- **CHEVRAY ; NATHANS.** *Proc. Natl. Acad. Sci. USA,* 1991, vol. 89, 5789-5793
- **COLIGAN et al.** Current Protocols in Immun. 1991, vol. 1
- **LEE et al.** *Nucl. Acids Res.,* 1979, vol. 6, 3073
- **COONEY et al.** *Science,* 1988, vol. 241, 456
- **DERVAN et al.** *Science,* 1991, vol. 251, 1360
- **OKANO.** *Neurochem.,* 1991, vol. 56, 560
- Oligodeoxynucleotides as Antisense Inhibitors of Gene Expression. CRC Press, 1988
- **ROSSI.** *Current Biology,* 1994, vol. 4, 469-471
- **MARASCO et al.** *Proc. Natl. Acad. Sci. USA,* 1993, vol. 90, 7889-7893
- **SOMOZA, J. R. ; HO, J. D. ; LUONG, C. ; GHATE, M. ; SPRENGELER, P. A. ; MORTARA, K. ; SHRADER, W. D. ; SPERANDIO, D. ; CHAN, H. ; MCGRATH, M. E.** *Structure,* 2003, vol. 11 (9), 1.123-1131
- **VU, T. K. ; LIU, R. W. ; HAAKSMA, C. J. ; TOMASEK, J. J. ; HOWARD, E. W.** *J Biol Chem,* 1997, vol. 272 (50), 31315-31320
- **YU, I. S. ; CHEN, H. J. ; LEE, Y. S. ; HUANG, P. H. ; LIN, S. R., TSAI, T. W. ; LIN, S. W.** *Thromb Haemost,* 2000, vol. 84 (5), 865-870
- **WU, Q. ; YU, D. ; POST, J. ; HALKS-MILLER, M. ; SADLER, J. E. ; MORSER, J.** *J Clin Invest,* 1998, vol. 101 (2), 321-326
- **TANIMOTO, H. ; YAN, Y. ; CLARKE, J. ; KOROURIAN, S. ; SHIGEMASA, K., PARMLEY, T. H. ; PARHAM, G. P. ; O'BRIEN, T. J.** *Cancer Res,* 1997, vol. 57 (14), 2884-2887
- **DHANASEKARAN, S. M. ; BARRETTE, T. R. ; GHOSH, D. ; SHAH, R. ; VARAMBALLY, S. ; KURACHI, K. ; PIENTA, K. J. ; RUBIN, M. A. ; CHINNAIYAN, A. M.** *Nature,* 2001, vol. 412 (6849), 822-826
- **LUO, J., DUGGAN, D. J. ; CHEN, Y. ; SAUVAGEOT, J. ; EWING, C. M. ; BITTNER, M. L. ; TRENT, J. M. ; ISAACS, W. B.** *Cancer Res,* 2001, vol. 61 (12), 4683-4688
- **MAGEE, J. A. ; ARAKI, T. ; PATIL, S. ; EHRIG, T. ; TRUE, L. ; HUMPHREY, P. A. ; CATALONA, W. J. ; WATSON, M. A. ; MILBRANDT, J.** *Cancer Res,* 2001, vol. 61 (15), 5692-5696
- **STAMEY, T. A. ; WARRINGTON, J. A. ; CALDWELL, M. C. ; CHEN, Z. ; FAN, Z. ; MAHADEVAPPA, M. ; MCNEAL, J. E. ; NOLLEY, R. ; ZHANG, Z.** *J Urol,* 2001, vol. 166 (6), 2171-2177
- **STEPHAN, C. ; YOUSEF, G. M. ; SCORILAS, A. ; JUNG, K. ; JUNG, M. ; KRISTIANSEN, G. ; HAUPTMANN, S. ; KISHI, T. ; NAKAMURA, T. ; LOENING, S. A.** *J Urol,* 2004, vol. 171 (1), 187-191
- **WELSH, J. B. ; SAPINOSO, L. M. ; SU, A. I. ; KERN, S. G. ; WANG-RODRIGUEZ, J. ; MOSKALUK, C. A. ; FRIERSON, H. F., JR. ; HAMPTON, G. M.** *Cancer Res,* 2001, vol. 61 (16), 5974-5978
- **XUAN, J. A. ; SCHNEIDER, D. ; TOY, P. ; LIN, R. ; NEWTON, A. ; ZHU, Y. ; FINSTER, S. ; VOGEL, D. ; MINTZER, B. ; DINTER, H.** *Cancer Res,* 2006, vol. 66 (7), 3611-3619
- **CHEN, Z. ; FAN, Z. ; MCNEAL, J. E. ; NOLLEY, R. ; CALDWELL, M. C. ; MAHADEVAPPA, M. ; ZHANG, Z. ; WARRINGTON, J. A. ; STAMEY, T. A.** *J Urol,* 2003, vol. 169 (4), 1316-1319
- **SRIKANTAN, V. ; VALLADARES, M. ; RHIM, J. S. ; MOUL, J. W. ; SRIVASTAVA, S.** *Cancer Res,* 2002, vol. 62 (23), 6812-6816
- **TORRES-ROSADO, A. ; O'SHEA, K. S. ; TSUJI, A. ; CHOU, S. H. ; KURACHI, K.** *Proc Natl Acad Sci U S A,* 1993, vol. 90 (15), 7181-7185
- **KLEZOVITCH, O. ; CHEVILLET, J. ; MIROSEVICH, J. ; ROBERTS, R. L. ; MATUSIK, R. J. ; VASIOUKHIN, V.** *Cancer Cell,* 2004, vol. 6 (2), 185-195
- **HERTER, S. ; PIPER, D. E. ; AARON, W. ; GABRIELE, T. ; CUTLER, G. ; CAO, P. ; BHATT, A. S. ; CHOC, Y. ; CRAIK, C. S. ; WALKER, N.** *Biochem J,* 2005, vol. 390, 125-136
- **KIRCHHOFER, D. ; PEEK, M. ; LIPARI, M. T. ; BILLECI, K. ; FAN, B. ; MORAN, P.** *FEBS Lett,* 2005, vol. 579 (9), 1945-1950
- **KAZAMA, Y. ; HAMAMOTO, T. ; FOSTER, D. C. ; KISIEL, W.** *J Biol Chem,* 1995, vol. 270 (1), 66-72
- **KIRCHHOFER, D. ; PEEK, M. ; LI, W. ; STAMOS, J. ; EIGENBROT, C. ; KADKHODAYAN, S. ; ELLIOTT, J. M. ; CORPUZ, R. T. ; LAZARUS, R. A. ; MORAN, P.** *J Biol Chem,* 2003, vol. 278 (38), 36341-36349
- **SHIA, S. ; STAMOS, J. ; KIRCHHOFER, D. ; FAN, B. ; WU, J. ; CORPUZ, R. T. ; SANTELL, L. ; LAZARUS, R. A. ; EIGENBROT, C.** *J Mol Biol,* 2005, vol. 346 (5), 1335-1349
- **HONGO, J. A. ; MORA-WORMS, M. ; LUCAS, C. ; FENDLY, B. M.** *Hybridoma,* 1995, vol. 14 (3), 253-260
- **CHENG, Y. ; PRUSOFF, W. H.** *Biochem Pharmacol,* 1973, vol. 22 (23), 3099-3108
- **MORRISON, J. F.** *Biochim Biophys Acta,* 1969, vol. 185 (2), 269-286
- **OLIVERO, A. G. ; EIGENBROT, C. ; GOLDSMITH, R. ; ROBARGE, K. ; ARTIS, D. R. ; FLYGARE, J. ; RAWSON, T. ; SUTHERLIN, D. P. ; KADKHODAYAN, S. ; BERESINI, M.** *J Biol Chem,* 2005, vol. 280 (10), 9160-9169
- **DELARIA, K. A. ; MULLER, D. K. ; MARLOR, C. W. ; BROWN, J. E. ; DAS, R. C. ; ROCZNIAK, S. O. ; TAMBURINI, P. P.** *J Biol Chem,* 1997, vol. 272 (18), 12209-12214

- KAWAGUCHI, T. ; QIN, L. ; SHIMOMURA, T. ; KONDO, J. ; MATSUMOTO, K. ; DENDA, K. ; KITAMURA, N. *J Biol Chem*, 1997, vol. 272 (44), 27558-27564
- LEE, S. L. ; DICKSON, R. B. ; LIN, C. Y. *J Biol Chem.*, 2000, vol. 275 (47), 36720-36725
- TAKEUCHI, T. ; HARRIS, J. L. ; HUANG, W. ; YAN, K. W. ; COUGHLIN, S. R. ; CRAIK, C. S. *J Biol Chem*, 2000, vol. 275 (34), 26333-26342
- MIYAZAWA, K. ; SHIMOMURA, T. ; KITAMURA, A. ; KONDO, J. ; MORIMOTO, Y. ; KITAMURA, N. *J Biol Chem*, 1993, vol. 268 (14), 10024-10028
- LIJNEN, H. R. ; VAN HOEF, B. ; COLLEN, D. *Eur J Biochem*, 1987, vol. 169 (2), 359-364
- COLLEN, D. ; ZAMARRON, C. ; LIJNEN, H. R. ; HOYLAERTS, M. *J Biol Chem*, 1986, vol. 261 (3), 1259-1266
- WOLF, B. B. ; VASUDEVAN, J. ; HENKIN, J. ; GONIAS, S. L. *J Biol Chem*, 1993, vol. 268 (22), 16327-16331
- STACK, M. S. ; JOHNSON, D. A. *J Biol Chem*, 1994, vol. 269 (13), 9416-9419
- YASUDA, S. ; MOROKAWA, N. ; WONG, G. W. ; ROSSI, A. ; MADHUSUDHAN, M. S. ; SALI, A. ; ASKEW, Y. S. ; ADACHI, R. ; SILVERMAN, G. A. ; KRILIS, S. A. *Blood*, 2005, vol. 105 (10), 3893-3901
- BRUNNER, G. ; VETTEL, U. ; JOBSTMANN, S. ; KRAMER, M. D. ; SCHIRRMACHER, V. *Blood*, 1992, vol. 79 (8), 2099-2106
- ICHINOSE, A. ; FUJIKAWA, K. ; SUYAMA, T. *J Biol Chem*, 1986, vol. 261 (8), 3486-3489
- LIST, K. ; JENSEN, O. N. ; BUGGE, T. H. ; LUND, L. R. ; PLOUG, M. ; DANO, K. ; BEHRENDT, N. *Biochemistry*, 2000, vol. 39 (3), 508-515
- GORETZKI, L. ; SCHMITT, M. ; MANN, K. ; CALVETE, J. ; CHUCHOLOWSKI, N. ; KRAMER, M. ; GUNZLER, W. A. ; JANICKE, F. ; GRAEFF, H. *FEBS Lett*, 1992, vol. 297 (1-2), 112-118
- KOBAYASHI, H. ; SCHMITT, M. ; GORETZKI, L. ; CHUCHOLOWSKI, N. ; CALVETE, J. ; KRAMER, M. ; GUNZLER, W. A. ; JANICKE, F. ; GRAEFF, H. *J Biol Chem*, 1991, vol. 266 (8), 5147-5152
- NAULAND, U. ; RIJKEN, D. C. *Eur J Biochem*, 1994, vol. 223 (2), 497-501
- ANDREASEN, P. A. ; KJOLLER, L. ; CHRISTENSEN, L. ; DUFFY, M. J. *Int J Cancer*, 1997, vol. 72 (1), 1-22
- DANO, K. ; BEHRENDT, N. ; HOYER-HANSEN, G. ; JOHNSEN, M. ; LUND, L. R. ; PLOUG, M. ; ROMER, J. *Thromb Haemost*, 2005, vol. 93 (4), 676-681
- HELENIUS, M. A. ; SARAMAKI, O. R. ; LINJA, M. J. ; TAMMELA, T. L. ; VISAKORPI, T. *Cancer Res*, 2001, vol. 61 (14), 5340-5344
- KNUDSEN, B. S. ; EDLUND, M. *Adv Cancer Res*, 2004, vol. 91, 31-67
- SHENG, S. *Cancer Metastasis Rev*, 2001, vol. 20 (3-4), 287-296
- VAN VELDHUIZEN, P. J. ; SADASIVAN, R. ; CHERIAN, R. ; WYATT, A. *Am J Med Sci*, 1996, vol. 312 (1), 8-11
- RIED, S. ; JAGER, C. ; JEFFERS, M. ; VANDE WOUDE, G. F. ; GRAEFF, H. ; SCHMITT, M. ; LENGYEL, E. *J Biol Chem*, 1999, vol. 274 (23), 16377-16386
- HALL, C. L. ; TSAN, R. ; MUGNAI, G. ; MAZAR, A. ; RADINSKY, R. ; PETTAWAY, C. A. *Prostate*, 2004, vol. 59 (2), 167-176
- JIN, J. S. ; HSIEH, D. S. ; LOH, S. H. ; CHEN, A. ; YAO, C. W. ; YEN, C. Y. *Mod Pathol*, 2006, vol. 19 (3), 447-452
- JOHNSON, M. D. ; OBERST, M. D. ; LIN, C. Y. ; DICKSON, R. B. *Expert Rev Mol Diagn*, 2003, vol. 3 (3), 331-338
- OBERST, M. D. ; JOHNSON, M. D. ; DICKSON, R. B. ; LIN, C. Y. ; SINGH, B. ; STEWART, M. ; WILLIAMS, A. ; AL-NAFUSSI, A. ; SMYTH, J. F. ; GABRA, H. *Clin Cancer Res*, 2002, vol. 8 (4), 1101-1107
- RIDDICK, A. C. ; SHUKLA, C. J. ; PENNINGTON, C. J. ; BASS, R. ; NUTTALL, R. K. ; HOGAN, A. ; SETHIA, K. K. ; ELLIS, V. ; COLLINS, A. T. ; MAITLAND, N. J. *Br J Cancer*, 2005, vol. 92 (12), 2171-2180
- SALEEM, M. ; ADHAMI, V. M. ; ZHONG, W. ; LONGLEY, B. J. ; LIN, C. Y., DICKSON, R. B. ; REAGAN-SHAW, S. ; JARRARD, D. F. ; MUKHTAR, H. *Cancer Epidemiol Biomarkers Prev*, 2006, vol. 15 (2), 217-227
- LIN, C. Y. ; ANDERS, J. ; JOHNSON, M. ; DICKSON, R. B. *J Biol Chem*, 1999, vol. 274 (26), 18237-18242
- LIST, K., SZABO, R. ; MOLINOLO, A. ; SRIURANPONG, V. ; REDEYE, V. ; MURDOCK, T. ; BURKE, B. ; NIELSEN, B. S. ; GUTKIND, J. S. ; BUGGE, T. H. *Genes Dev*, 2005, vol. 19 (16), 1934-1950
- LIST, K. ; HAUDENSCHILD, C. C. ; SZABO, R. ; CHEN, W. ; WAHL, S. M. ; SWAIM, W. ; ENGELHOLM, L. H. ; BEHRENDT, N. ; BUGGE, T. H. *Oncogene*, 2002, vol. 21 (23), 3765-3779
- HUAI, Q. ; MAZAR, A. P. ; KUO, A. ; PARRY, G. C. ; SHAW, D. E. ; CALLAHAN, J. ; LI, Y. ; YUAN, C. ; BIAN, C. ; CHEN, L. *Science*, 2006, vol. 311 (5761), 656-659
- PETERSEN, L. C. ; LUND, L. R. ; NIELSEN, L. S. ; DANO, K. ; SKRIVER, L. *J Biol Chem*, 1988, vol. 263 (23), 11189-11195